# EUROPEAN PATENT SPECIFICATION

(11) **EP 0 800 579 B1**
(45) Date of publication and mention of the grant of the patent: **24.07.2002**
(21) Application number: 95944420.9
(22) Date of filing: 22.12.1995
(51) Int. Cl.: C12N 15/19, A61K 48/00

(54) **COMBINATION GENE DELIVERY VEHICLES**
KOMBINATIONSVEKTOREN ZUM AUSTRAGEN VON GENMATERIAL
COMBINAISON DE VECTEURS D'ADMINISTRATION DE GENES

(30) Priority: 30.12.1994 US 368210
(43) Date of publication of application: 15.10.1997
(73) Proprietor: CHIRON CORPORATION, Emeryville, California 94608 (US)
(72) Inventor: JOLLY, Douglas, J., Leucadia, CA 92024 (US); MONTISANO, Dominic, San Diego, CA 92117 (US)
(74) Representative: Irvine, Jonquil Claire
(86) International application number: US9516964
(87) International publication number: WO9621015

(56) References cited:
- EP-A- 0 600 591
- WO-A-93/06867
- DAVIS H.L. ET AL: 'Plasmid DNA is superior to viral vectors for direct gene transfer into adult mouse skeletal muscle' HUMAN GENE THERAPY vol. 4, 1993, pages 733 - 740

### SEQUENCE LISTING

(1) GENERAL INFORMATION:
   (i) APPLICANT: CHIRON CORPORATION
   (ii) TITLE OF INVENTION: COMBINATION GENE DELIVERY VEHICLE
   (iii) NUMBER OF SEQUENCES: 25
   (iv) CORRESPONDENCE ADDRESS:
      (A) ADDRESSEE: Chiron Viagene Inc.
      (B) STREET: Intellectual Property - P.O Box 8097
      (C) CITY: Emeryville
      (D) STATE: California
      (E) COUNTRY: USA
      (F) ZIP: 94662-8097-
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: PCT - Unassigned
      (B) FILING DATE: Even Date Herewith
      (C) CLASSIFICATION:
   (viii) ATTORNEY/AGENT INFORMATION:
      (A) NAME:Kruse, Norman J.
      (B) REGISTRATION NUMBER: 35,235
      (C) REFERENCE/DOCKET NUMBER: 1133.500
   (ix) TELECOMMUNICATION INFORMATION:
      (A) TELEPHONE: (510) 601-3520
      (B) TELEFAX: (510) 655-3542
(2) INFORMATION FOR SEQ ID NO:1:
   (i) SEQUENCE CHARACTERISTICS:
      (A) LENGTH: 53 base pairs
      (B) TYPE: nucleic acid
      (C) STRANDEDNESS: single
      (D) TOPOLOGY: linear
   (xi) SEQUENCE DESCRIPTION: SEQ ID NO:1:
   CGAAGCTTAA GCTTGCCATG GGCCACACAC GGAGGCAGGG AACATCACCA TCC 53
(2) INFORMATION FOR SEQ ID NO:2:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:2:
   CCTCGAGCTC GAGCTGTTAT ACAGGGCGTA CACTTTCCCT TCTCAATCTC TC 52
(2) INFORMATION FOR SEQ ID NO:3:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:3:
   CCTCGAGCTC GAGGCCATGG GCCACACACG GAGGCAGGGA ACATCACCAT CC 52
(2) INFORMATION FOR SEQ ID NO:4:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:4:
   CGGGCCCGGG CCCCTGTTAT ACAGGGCGTA CACTTTCCCT TCTCAATCTC TC 52
(2) INFORMATION FOR SEQ ID NO:5:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:5:
   CTCGAGCTCG AGGCACCAGC ACCATGCAAC TTTTT 35
(2) INFORMATION FOR SEQ ID NO:6:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:6:
   CTACTAGATC CCTAGATGCT GGATCTTCC 29
(2) INFORMATION FOR SEQ ID NO:7:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:7:
   GGAAGATCCA GCATCTAGGG ATCTAGTAG 29
(2) INFORMATION FOR SEQ ID NO:8:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (xi) SEQUENCE DESCRIPTION: SEQ ID NO:8:
   GGGCGATATC AAGCTTATCG ATACCG 26
(2) INFORMATION FOR SEQ ID NO:9:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (xi) SEQUENCE DESCRIPTION: SEQ ID NO:9:
   AATACGACTC ACTATAGGG 19
(2) INFORMATION FOR SEQ ID NO:10:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (xi) SEQUENCE DESCRIPTION: SEQ ID NO:10:
   ATTAACCCTC ACTAAAG 17
(2) INFORMATION FOR SEQ ID NO:11:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:11:
   CCTCGAGCTC GAGCTTGGGT GGCTTTGGGG CATG 34
(2) INFORMATION FOR SEQ ID NO:12:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (xi) SEQUENCE DESCRIPTION: SEQ ID NO:12:
   ATTACCCCTC ACTAAAG 17
(2) INFORMATION FOR SEQ ID NO:13:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:13:
   TAATAAATAG ATTTAGATTT A 21
(2) INFORMATION FOR SEQ ID NO:14:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:14:
   GCCTCGAGAG CATGAAATAT ACAAGTTATA TCTTG 35
(2) INFORMATION FOR SEQ ID NO:15:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:15:
   GAATCGATCC ATTACTGGGA TGCTCTTCGA CCTGG 35
(2) INFORMATION FOR SEQ ID NO:16:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:16:
   GCCTCGAGCT CGAGGAGGAT GTGGCTGCAG AGCCTGCTG 39
(2) INFORMATION FOR SEQ ID NO:17:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:17:
   GCATCGATCG ATGTCTCACT CCTGGACTGG CTCCCAGCA 39
(2) INFORMATION FOR SEQ ID NO:18:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:18:
   ATAAATAGAA GGCCTGATAT G 21
(2) INFORMATION FOR SEQ ID NO:19:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:19:
   GCCTCGAGAC AATGTACAGG ATGCAACTCC TGTCT 35
(2) INFORMATION FOR SEQ ID NO:20:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:20:
   GAATCGATTT ATCAAGTCAG TGTTGAGATG ATGCT 35
(2) INFORMATION FOR SEQ ID NO:21:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:21:
   TATATCTCCA GATGAGGTAC ATGATTTTAG GCTTG 35
(2) INFORMATION FOR SEQ ID NO:22:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (xi) SEQUENCE DESCRIPTION: SEQ ID NO:22:
   TATATATCGA TTCAAGGCAT TTTCTTTTCA TCAATAAAAC 40
(2) INFORMATION FOR SEQ ID NO:23:
   (i) SEQUENCE CHARACTERISTICS:
      (A) LENGTH: 655 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:23:
   CACCAGCAAC ATGCAACTTT TTCACCTCTG CCTAATCATC TCTTGTACAT GTCCCACTGT       60
   TCAAGCCTCC AAGCTGTGCC TTGGGTGGCT TTGGGGCATG GACATTGACC CTTATAAAGA      120
   ATTTGGAGCT ACTGTGGAGT TACTCTCGTT TTTGCCTTCT GACTTCTTTC CTTCCGTCAG      180
   AGATCTCCTA GACACCGCCT CAGCTCTGTA TCGGGAAGCC TTAGAGTCTC CTGAGCATTG      240
   CTCACCTCAC CACACCGCAC TCAGGCAAGC CATTCTCTGC TGGGGGGAAT TGATGACTCT      300
   AGCTACCTGG GTGGGTAATA ATTTGGAAGA TCCAGCATCT AGGGATCTAG TAGTCAATTA      360
   TGTTAATACT AACATGGGTT TAAAAATTAG GCAACTATTG TGGTTTCATA TATCTTGCCT      420
   TACTTTTGGA AGAGAGACTG TACTTGAATA TTTGGTATCT TTCGGAGTGT GGATTCGCAC      480
   TCCTCCAGCC TATAGACCAC CAAATGCCCC TATCTTATCA ACACTTCCGG AAACTACTGT      540
   TGTTAGACGA CGGGACCGAG GCAGGTCCCC TAGAAGAAGA ACTCCCTCGC CTCGCAGACG      600
   CAGATCTCCA TCGCCGCGTC GCAGAAGATC TCAATCTCGG GAATCTCAAT GTTAG           655
(2) INFORMATION FOR SEQ ID NO:24:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:24:
   CCGCATCAAG GGATCTAGTA G
   21
(2) INFORMATION FOR SEQ ID NO:25:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:25:
   CCAGCATCTA GGGATCTAGT AG                                             22

## Description

### Field of the Invention

The field of the present invention is recombinant nucleic acid vectors and other vehicles suitable for the introduction of nucleic acid molecules having desirable properties into a plant or an animal, where the vehicle may, for example, express a desired substance or incorporate into a specified nucleic acid molecule, and methods of combining and administering the same.

### Background of the Invention

Recent advances in the field of biotechnology, including the engineering of desirable nucleic acid molecules, have given rise to significant advances in the treatment of diseases such as cancer, genetic diseases, arthritis and AIDS. Many of these advances involve the administration of desirable nucleic acid molecules to a subject, particularly animal subjects such as human subjects. The administration of more than one (*i*.*e*., multiple) such nucleic acid molecule at one time would provide significant advantages because multiple nucleic acid molecules can provide complementary substances or activities to a single organ or joint. Thus, for example, a nucleic acid molecule encoding a cytotoxin can be administered along with a a nucleic acid molecule that increases the susceptibility of target cells to the cytotoxin.

Administering multiple complementary substances and/or activities at one time and on more than one nucleic acid molecule provides significant advantages over administering such substances and/or activities on a single nucleic acid molecule. The difficulty, cost and time to engineer multiple nucleic acid molecules is much less than engineering a single molecule. For example, there are fewer difficulties with expressing a single substance or activity on one molecule, where each substance is under the control of its own expression system, than expressing multiple substances or activities from a single system. Further, with the use of multiple molecules, there is less chance that one substance or activity will sterically hinder or otherwise interfere with another substance or activity. The use of multiple molecules also permits the expression of different substances or activities from expression systems subject to differing activating events, thereby permitting better control of differential expression of the different substances or activities.

However, the administration of multiple nucleic acid molecules has been discouraged in the past because there has been a perceived concern that such administration would increase the likelihood of random, potentially harmful recombination events between the molecule and the host's genome.

In addition, it was perceived that administration of independent multiple nucleic acids that expressed agents having some degree of synergy was undesirable because that synergy would not occur if the two active nucleic acid sequences were not linked physically.

Thus, there has gone unmet a need for the administration of multiple nucleic acid molecules carrying multiple helpful substances or activities. The present invention provides such administration of desired nucleic acid molecules, compositions containing such desired nucleic acid molecules, and other, related advantages.

### Summary of the Invention

In one aspect, the present invention is directed towards a method of introducing nucleic acid molecules to an animal or patient comprising administering a composition comprising two or more gene delivery vehicles to the animal, each of the gene delivery vehicles containing a nucleic acid molecule not naturally contained within its corresponding gene delivery vehicle, in combination with a pharmaceutically acceptable carrier or diluent.

In another aspect, the present invention is directed towards a method of introducing nucleic acid molecules to an animal or patient comprising administering a composition comprising two or more gene delivery vehicles to an animal or patient, each of the gene delivery vehicles directing the expression of at least one substance in host cells containing the gene delivery vehicles, the substance not naturally expressed by its corresponding gene delivery vehicle, the gene delivery vehicles a) collectively directing the expression of at least two different substances, or b) directing the expression of at least one substance wherein the gene delivery vehicles differ in one or more biological functions, in combination with a pharmaceutically acceptable carrier or diluent.

In a further aspect, the present invention is directed towards a method of introducing nucleic acid molecules to an animal comprising administering a composition comprising two or more gene delivery vehicles to an animal, each of the gene delivery vehicles containing at least one biologically active nucleic acid molecule wherein such biological activity is not naturally present in its respective gene delivery vehicle, the gene delivery vehicles a) collectively containing at least two different biologically active nucleic acid sequences, or b) containing at least one biologically active nucleic acid sequence wherein the gene delivery vehicles differ in one or more biological functions, in combination with a pharmaceutically acceptable carrier or diluent.

In still a further aspect, the present invention is directed towards a method of introducing nucleic acid molecules to an animal comprising administering a composition comprising two or more gene delivery vehicles to an animal, at least one of the gene delivery vehicles directing the expression of at least one substance not naturally expressed by its corresponding gene delivery vehicle, and at least one of the gene delivery vehicles containing at least one biologically active nucleic acid sequence not naturally contained within its corresponding gene delivery vehicle, in combination with a pharmaceutically acceptable carrier or diluent.

In yet another aspect, the present invention is directed towards a method of introducing nucleic acid molecules to an animal comprising administering two or more gene delivery vehicles to an animal at the same time and same site via a single administration device, each of the gene delivery vehicles directing the expression of at least one substance in host cells containing the gene delivery vehicles, the substance not naturally expressed by its corresponding gene delivery vehicle, the gene delivery vehicles a) collectively directing the expression of at least two different substances, or b) directing the expression of at least one substance wherein the gene delivery vehicles differ in one or more biological functions, in combination with a pharmaceutically acceptable carrier or diluent.

In yet a further aspect, the present invention is directed towards a method of introducing nucleic acid molecules to an animal comprising administering a composition comprising two or more gene delivery vehicles to an animal, the method comprising the steps of a) separately preparing a first gene delivery vehicle and a second gene delivery vehicle, each of the gene delivery vehicles directing the expression of at least one substance in host cells containing the gene delivery vehicles, the substance not naturally expressed by its corresponding gene delivery vehicle, the gene delivery vehicles i) collectively directing the expression of at least two different substances, or ii) directing the expression of at least one substance wherein the gene delivery vehicles differ in one or more biological functions, b) combining the first gene delivery vehicle and the second gene delivery vehicle with a pharmaceutically acceptable carrier or diluent to provide the composition; and c) administering the composition to the animal.

In preferred embodiments, the present invention is directed towards methods as set forth above wherein the pharmaceutically acceptable carrier or diluent enhances the administration of the gene delivery vehicles.

In other preferred embodiments, the present invention is directed towards methods as set forth above wherein the substance or the biological activity is not exhibited in the animal prior to the administration. In yet other preferred embodiments, the present invention is directed towards such methods wherein the biological activity is not exhibited in the host cells prior to administration.

In other preferred embodiments, the present invention is directed towards methods as set forth above wherein the biological activity complements, activates, replaces or suppresses a biological activity present in the host cells prior to administration.

In still other preferred embodiments, in the methods of the invention, the composition comprises a first gene delivery vehicle directing the expression of an antigen stimulator and a second gene delivery vehicle directing the expression of a cytokine or an immune activating protein; wherein a first gene delivery vehicle directing the expression of an enzyme capable of activating a conditionally lethal gene product and a second gene delivery vehicle directing the expression of a cytokine; and wherein said gene delivery vehicles direct the expression of two or more antigens.

In alternative preferred embodiments, in the methods of the invention, at least one of said gene delivery vehicles directs the expression of a systemically distributed gene product or a locally distributed gene product; preferably, the gene product is a protein, and further preferably an immune suppressing protein.

These and other aspects of the present invention will become evident upon reference to the following detailed description. In addition, various references are set forth herein which describe in more detail certain procedures or compositions; such references are incorporated by reference in their entirety.

### Brief Description of the Drawings

Figure 1 is a schematic illustration which outlines the recovery of Hepatitis B e sequence from ATCC 45020.

Figure 2 is a diagrammatic representation of the nucleotide sequence of HBV (adw) precore/core (SEQ ID. NO. 23) and the region of the incorrect sequence from pAM6 (ATCC 95020) clone (SEQ ID. NO. 24).

Figure 3 is a schematic representation of the protocol utilized to correct the mutation in HB precore/core sequence from pAM6 (ATCC 45020).

Figure 4 is a DNA sequencing gel showing the corrected nucleotide sequences from SK⁺HBe-c.

Figure 5 is a table showing the level of expression of HBVe protein and HBV core protein from the following retrovirally transduced murine cell lines BC10ME, B1/6, L-M(TK⁻), EA2K^{b}, and retrovirally transduced human T-cell line JA2/K^{b} as determined by ELISA.

Figure 6 is a Western blot showing immunoprecipitation/expression of secreted p17 kD HBV e protein and p23 kD pre-core intermediate protein by retrovirally transduced BC10ME and B1/6 cells. This blot also shows expression of p21 HBV core protein in cell lysates from retrovirally transduced BC10ME cells.

Figure 7A is two graphs which show induction of antibody responses against HBVe antigen in Balb/C and C57B1/6 mice injected with syngeneic cells expressing the antigen, or, by direct injection with the retroviral vector encoding HBVe antigen.

Figure 7B is two graphs which show induction of antibody responses against HB/core antigen in Balb/C (BC) and C57B1/6 (B16) mice injected with synergenic cells expressing the HBV core antigen.

Figure 8 schematically illustrates the cloning of murine gamma-interferon into a replication defective retroviral vector.

Figure 9 is a Western blot which depicts MHC Class I protein expression in L33 and B16F10 cell lines.

Figure 10 illustrates the construction of the plasmids carrying the vectors TK1 (without SV-Neo) and TK3 (plus SV-Neo).

Figure 11 is a graph illustrating the effect of Ganciclovir on *in vivo* transduced CT26 cells by injection of TK-3 virus containing the HSVTK gene.

Figure 12 is a graph demonstrating retention of viral activity upon reconstitution of a representative recombinant retrovirus lyophilized in a formulation buffer containing mannitol.

Figure 13 is a graph demonstrating retention of viral activity upon reconstitution of a representative recombinant retrovirus lyophilized in a formulation buffer containing lactose.

Figure 14 is a graph demonstrating retention of viral activity upon reconstitution of a representative recombinant retrovirus lyophilized in a formulation buffer containing trehalose.

Figures 15A-15D are representative graphs comparing stability of liquid non-lyophilized recombinant retrovirus stored at -80°C versus lyophilized formulated recombinant retrovirus stored at -20°C, using various saccharides. For ease of comparison, the titers have been normalized.

Figure 16 is a graph showing induction of CTL responses against HBV core antigen and HBV e antigen in the C3H/He mice after i.m. administration of HBV core formulated HB Fcore/neo^{R} vector.

Figure 17 is a graph showing that CTL response against HBV core antigen in the C3H/He CR mice are MHC class I restricted.

Figure 18 (panels A & B) is a pair of graphs showing that CTL response against HBV core antigen in the C3H/He CR mice are CD4⁻CD8⁺ cells.

Figure 19 is a table which shows induction of antibody responses against HBV core antigen in C3H He CR mcie injected with formulated HB Fcore/neo^{R} vector.

Figure 20 is a table showing the isotypes of the antibody responses against HBV core antigen and HBV e antigen in C3H/He CR mice injected with formulated HB Fcore/neo^{R} vector.

Figure 21 is a graph showing induction of CTL responses against HBV core antigen and HBV e antigen in rhesus macaques after intramuscular injection of formulated HB Fcore/neo^{R} vector.

Figure 22 (panels A & B) is a pair of graphs showing that CTL responses against HBV core antigen in the rhesus macaques are CD4⁻CD8⁺ cells.

### Detailed Description of the Invention

The present invention is directed towards the combination of multiple, *i.e.,* two or more, gene delivery vehicles ("GDVs") with a pharmaceutically acceptable carrier or diluent to provide a pharmaceutically acceptable composition, and the administration of such a composition to an animal. The administration of multiple GDV in a single composition, or at the same time and site via a single administration device, provides numerous advantages over previous methods of treating diseases or other pathogenic agents that included the use of a GDV. The administration of multiple GDVs is desirable where the level of expression from genes carried by different GDVs is different from one GDV to the other, when it is preferred that the elicited response be predominantly against a gene product from one GDV, or if an immediate response is required from one GDV's gene product and a delayed or priming response is required from the other.

### I. Gene Delivery Vehicles

Gene Delivery Vehicles are recombinant vehicles, such as viral vectors, nucleic acid vectors (such as plasmids), naked nucleic acid molecules such as genes, nucleic acid molecules complexed to a polycationic molecule capable of neutralizing the negative charge on the nucleic acid molecule and condensing the nucleic acid molecule into a compact molecule, nucleic acids associated with liposomes bacteria, and certain eukaryotic cells such as producer cells, that are capable of delivering a nucleic acid molecule having one or more desirable properties to host cells in an organism. As discussed further below, the desirable properties include the ability to express a desired substance, such as a protein, enzyme or antibody, and/or the ability to provide a biological activity, which is where the nucleic acid molecule carried by the GDV is itself the active agent without requiring the expression of a desired substance. One example of such biological activity is gene therapy where the delivered nucleic acid molecule incorporates into a specified gene so as to inactivate the gene and "turn off" the product the gene was making.

Typically, the GDV is an assembly that carries a nucleic acid molecule (or sequence), such molecule often capable of expressing sequences or genes of interest. In the context of protein expression, the GDV must include promoter elements such as for RNA Polymerase II or an RNA replicase and may include a signal that directs polyadenylation. In addition, the GDV, when transcribed, is preferably operably linked to the molecules or genes of interest and acts as a translation initiation sequence. The GDV may include a selectable marker such as neomycin, thymidine kinase, hygromycin, phleomycin, histidinol, or dihydrofolate reductase (DHFR), as well as one or more restriction sites and a translation termination sequence. In addition, if the GDV is used to make a retroviral particle, the GDV must include a retroviral packaging signal and LTRs appropriate to the retrovirus used, provided these are not already present. The GDV can also be used in combination with other viral vectors or inserted physically into cells or tissues as described below. The GDV may include a sequence that encodes a protein or active portion of the protein, antisense or ribozyme. Such sequences may be designed to inhibit MHC antigen presentation in order to suppress the immune response of cytotoxic T-lymphocytes against a transplanted tissue.

Particularly preferred viral vectors for use as a GDV within the present invention include recombinant retroviral vectors and recombinant adenovirus vectors. The construction of recombinant retroviral vectors is described in greater detail in an application entitled "Recombinant Retroviruses" (U.S.S.N. 07/586,603, filed September 21, 1990) (this reference and all other references cited with this application are expressly incorporated herein in their entirety). These recombinant retroviral vectors may be used to generate transduction competent retroviral vector particles by introducing them into appropriate packaging cell lines *(see* U.S.S.N. 07/800,921 filed November 29, 1991). Similarly, adenovirus vectors may also be readily prepared and utilized given the disclosure provided herein (see also Berkner, *Biotechniques 6*:616-627, 1988, and Rosenfeld et al., *Science 252*:431-434, 1991, WO 93/07283, WO 93/06223, and WO 93/07282).

In another preferred embodiment, the one or both of the GDVs is a Sindbis RNA expression vector that includes, in order, a 5' sequence which is capable of initiating transcription of a Sindbis virus, a nucleotide sequence encoding Sindbis non-structural proteins, a viral junction region, a heterologous sequence, a Sindbis RNA polymerase recognition sequence, and a stretch of 25 consecutive polyadenylate residues. A wide variety of heterologous sequences may be included in the GDV. Within various embodiments of the invention, the GDV may contain (and express, within certain embodiments) two or more heterologous sequences.

Other viral vectors suitable for use in the present invention include, for example, poliovirus (Evans et al., *Nature 339*:385-388, 1989, and Sabin, *J*. *of Biol*. *Standardization 1*:115-118, 1973); rhinovirus (Arnold, *J*. *Cell*. *Biochem*. L401-405, 1990); pox viruses, such as canary pox virus or vaccinia virus (Fisher-Hoch et al., *PNAS 86*:317-321, 1989; Flexner et al., *Ann*. *N*.*Y*. *Acad*. *Sci. 569*:86-103, 1989; Flexner et al., *Vaccine 8*:17-21, 1990; U.S. Patent Nos. 4,603,112 and 4,769,330; WO 89/01973); SV40 (Mulligan et al., *Nature 277*:108-114, 1979); influenza virus (Luytjes et al., *Cell 59*:1107-1113, 1989; McMicheal et al., *The New England Journal of Medicine 309*:13-17, 1983; and Yap et al., *Nature 273*:238-239, 1978); pavoviruses such as adeno-associated virus (Samulski et al., *Journal of Virology 63*:3822-3828, 1989, and Mendelson et al., *Virology 166*:154-165, 1988); herpes (Kit, *Adv*. *Exp*. *Med. Biol. 215*:219-236, 1989); HIV; measles (EP 0 440,219); measles (EP 0 440,219); astrovirus (Munroe, S.S. et al., *J*. *Vir. 67*:3611-3614, 1993); Semliki Forest Virus, and coronavirus, as well as other viral systems *(e.g.,* EP 0,440,219; WO 92/06693; U.S. Patent No. 5,166,057). In addition, viral carriers may be homologous, non-pathogenic (defective), replication competent virus *(e.g.,* Overbaugh et al., *Science 239*:906-910, 1988).

In a preferred embodiment, where the GDV is a retroviral vector, the nucleic acid molecules carried by the retroviral vector should be of a size sufficient to allow production of viable virus. Within the context of the present invention, the production of any measurable titer of infectious virus on susceptible monolayers is considered to be "production of viable virus." Within preferred embodiments, a heterologous sequence within the retroviral vector GDV will comprise at least 100 bases, at least 2 kb, 3.5 kb, 5 kb, or 7 kb, or even a heterologous sequence of at least 8 kb.

Nucleic acid molecules without any covering, such as a viral capsid or bacterial cell membrane, are also suitable for use as a GDV within the present invention. Such "naked" nucleic acids include plasmids, viral vectors without coverings, and even naked genes without any control region. The GDV may be either DNA or RNA, or may be a combination of the two, comprising both DNA and RNA in a single molecule.

In another alternative embodiment, the GDV is a liposome. Liposomes are small, lipid vesicles comprised of an aqueous compartment enclosed by a lipid bilayer, typically spherical or slightly elongated structures and several hundred angstroms in diameter. Liposomes offer several readily exploited features. Under appropriate conditions, the liposome can fuse with the plasma membrane of a target cell or with the membrane of an endocytic vesicle within a cell which has internalized the liposome, thereby disgorging its contents into the cytoplasm. Prior to interaction with the surface of a target cell, however, the liposome membrane acts as a relatively impermeable barrier which sequesters and protects its contents, for example from degradative enzymes in the plasma. Liposomes have for this reason also been referred to as "micropills". Additionally, because a liposome is a synthetic structure, custom-formulated liposomes can be designed that incorporate desirable features. (Stryer, L., *Biochemistry*, pp236-240 1975 (W.H. Freeman, San Francisco); Szoka et al., *Biochim*. *Biophys*. *Acta 600*:1-18 (1980); Bayer et al., *Biochim*. *Biophys*. *Acta*. *550*:464 (1979); Rivnay et al., *Meth*. *Enzymol*. *149*:119 (1987); Wang et al., *PNAS 84*: 7851, 1987 and, Plant et al., *Anal*. *Biochem*. *176*:420 (1989).

Bacterial cells suitable for use as a GDV within the present invention include a bacteria that expresses a cytotoxic agent, such as an anti-tumor agent, on its cell surface or exported from the bacterium. Representative examples include BCG (Stover, *Nature 351:*456-458, 1991) and Salmonella (Newton et al., *Science 244*:70-72, 1989). Eukaryotic cells suitable for use in the present invention include producer cells.

The GDVs of the present invention may have the same the nucleic acid backbone (*e.g.,* be derived from the same virus, such as Sindbis or vaccinia), but then must have different promoters or other regulatory sequences, nucleic acid sequences, etc., that cause the viral vectors to differ in some biological function. Alternatively, the vectors may have different backbones (*e.g.,* be derived from different viruses, such as one vector from Sindbis and one vector from vaccinia), in which instance the vectors may have either the same or different regulatory sequences, desired nucleic acid sequences, etc. The backbone may be either DNA, RNA, or a combination of both DNA and RNA. Further, the vectors used as GDVs may be combined in any desired grouping of vectors, such as only viral or only bacterial vectors, or a grouping having one viral vector and one eukaryotic vector, or a grouping having one bacterial vector, one viral vector and one eukaryotic vector.

Within one embodiment of the present invention, the GDVs comprise a nucleic acid molecule under the transcriptional control of an event-specific promoter, such that upon activation of the event-specific promoter the nucleic acid molecule is expressed. Numerous event-specific promoters may be utilized within the context of the present invention, including for example, promoters which are activated by cellular proliferation (or are otherwise cell-cycle dependent) such as the thymidine kinase or thymidilate synthase promoters (Merrill, *Proc*. *Natl*. *Acad*. *Sci. USA 86*:4987-91, 1989; Deng et al., *Mol*. *Cell. Biol. 9*:4079-82, 1989); promoters such as the α- or β- interferon promoters which are activated when a cell is infected by a virus (Fan and Maniatis, *EMBO J*. *8*(1):101-110, 1989; Goodbourn et al. *Cell 45*:601-610, 1986); and promoters which are activated by the presence of hormones (*e*.*g*., estrogen response promoters; *see* Toohey et al., *Mol*. *Cell*. *Biol. 6*:4526-38, 1986).

Within a preferred embodiment, a recombinant viral vector (preferably, but not necessarily, a recombinant MLV retrovirus) carries a gene expressed from an event-specific promoter, such as a cell cycle-dependent promoter (*e.g.,* human cellular thymidine kinase or transferrin receptor promoters), which will be transcriptionally active primarily in proliferating cells, such as tumors. In this manner, replicating cells which contain factors capable of activating transcription from these promoters are preferentially affected *(e.g.,* destroyed) by the agent produced by the GDV.

Within another embodiment of the present invention, the GDVs comprise a nucleic acid molecule under the transcriptional control of a tissue-specific promoter, such that upon activation of the tissue-specific promoter the nucleic acid molecule is expressed. A wide variety of tissue-specific promoters may be utilized within the context of the present invention. Representative examples of such promoters include: liver-specific promoters such as Phospho-Enol-Pyruvate Carboxy-Kinase (Hatzogiou et al., *J. Biol. Chem. 263:* 17798-808, 1988; Benvenisty et al., *Proc. Natl. Acad*. *Sci. USA 86*:1118-22, 1989; Vaulont et al., *Mol. Cell. Biol. 9*:4409-15, 1989), the albumin promoter, the alphafetoprotein promoter (Feuerman et al., *Mol. Cell. Biol. 9*:4204-12, 1989; Camper and Tilghman, *Genes Develop. 3*:537-46, 1989), and the alcohol dehydrogenase promoter (Felder, *Proc. Natl. Acad. Sci. USA 86*:5903-07, 1989); B cell specific promoters such as the IgG promoter; breast carcinoma or hepatocellular carcinoma specific promoters such as Carcinoembryonic Antigen promoter (CEA) (Schrewe et al., *Mol*. *and Cell. Biol*. *10*:2738, 1990); pancreatic acinar cell specific promoters such as the elastase promoter (Swift et al., *Genes Develop. 3*:687-96, 1989); breast epithelial specific promoters such as the casein promoter (Doppler et al., *Proc. Natl. Acad*. *Sci. USA 86*:104-08, 1989); erythroid specific-transcription promoters which are active in erythroid cells, such as the porphobilinogen deaminase promoter (Mignotte et al., *Proc. Natl. Acad*. *Sci. USA 86*:6458-52, 1990); α- or β- globin specific promoters (van Assendelft et al., *Cell 56:*969-77, 1989, Forrester et al., *Proc. Natl. Acad*. *Sci*. *USA 86*:5439-43, 1989); promoters which regulate skeletal muscle such as the myo-D binding site (Burden, *Nature 341:*716, 1989; Weintraub et al., *Proc. Natl. Acad*. *Sci. USA 86*:5434-38, 1989); promoters which are specific for β cells of the pancreas, such as the insulin promoter (Ohlsson et al., *Proc. Natl. Acad*. *Sci. USA 85*:4228-31, 1988; Karlsson et al., *Mol. Cell. Biol. 9*:823-27, 1989); promoters that are specific for the pituitary gland, such as the growth hormone factor promoter (Ingraham et al., *Cell 55:*519-29, 1988; Bodner et al., *Cell 55*:505-18, 1988); promoters which are specific for melanocytes, such as the tyrosine hydroxylase promoter; breast carcinoma specific promoters such as the HER2/neu promoter (Tal et al., *Mol. and Cell. Biol. 7*:2597, 1987); T-cell specific promoters such as the T-cell receptor promoter (Anderson et al., *Proc. Natl. Acad*. *Sci. USA 85*:3551-54, 1988; Winoto and Baltimore, *EMBO J. 8*:729-33, 1989); osteoblasts or bone-specific promoters such as the osteocalcin promoter (Markose et al., *Proc. Natl. Acad*. *Sci. USA 87*:1701-1705, 1990; McDonnell et al., *Mol. Cell. Biol. 9*:3517-23, 1989; Kerner et al., *Proc. Natl. Acad*. *Sci. USA 86*:4455-59, 1989) the IL-2 promoter, IL-2 receptor promoter, the whey (WAP) promoter, and the MHC Class II promoter.

A variety of other elements which control gene expression may also be utilized within the context of the present invention, including for example locus-defining elements such as the β-globin gene and the T cell marker CD2. In addition, elements which control expression at the level of splicing and nuclear export are the β-globin intron sequences, the rev and rre elements in HIV-1, and the CTE element in the D-type masonpfizer monkey retrovirus.

Within preferred embodiments of the invention, the GDV is a retroviral vector and the gene produces an agent against a tumor, the gene being under control of a tissue-specific promoter having specificity for the tissue of tumor origin. Since the retroviral vector preferentially integrates into the genome of replicating cells (for example, normal liver cells are not replicating, while those of a hepatocarcinoma are), these two levels of specificity (viral integration/replication and tissue-specific transcriptional regulation) lead to preferential killing of tumor cells.

Within yet another related embodiment of the present invention, the GDV comprises a nucleic acid molecule under the transcriptional control of both an event-specific promoter and a tissue-specific promoter, such that the nucleic acid molecule is maximally expressed only upon activation of both the event-specific promoter and the tissue-specific promoter. In particular, by utilizing such vectors, the substance expressed from the nucleic acid molecule is expressed only in cell types satisfying both criteria (*e.g.,* in the example above, combined promoter elements are functional only in rapidly dividing liver cells). Within preferred embodiments of the invention, the number of transcriptional promoter elements may also be increased, in order to improve the stringency of cell-type specificity.

Transcriptional promoter/enhancer elements as discussed above need not necessarily be present as an internal promoter (lying between the viral LTRs for retroviruses, for example), but may be added to or replace the transcriptional control elements in the viral LTRs which are themselves transcriptional promoters, such that condition-specific (*i*.*e*., event or tissue specific) transcriptional expression will occur directly from the modified viral LTR. In this case, either the condition for maximal expression will need to be mimicked in retroviral packaging cell lines (*e*.*g*., by altering growth conditions, supplying necessary transregulators of expression or using the appropriate cell line as a parent for a packaging line), or the LTR modification is limited to the 3' LTR U3 region, to obtain maximal recombinant viral titers. In the latter case, after one round of infection/integration, the 3' LTR U3 is now also the 5' LTR U3, giving the desired tissue-specific expression. Similarly, for other viral vectors, the promoters may be exogenous, or hybrids with normal viral promoter elements.
The present invention also provides eukaryotic layered vector initiation systems, which are comprised of a 5' promoter, a construct which is capable of expressing one or more heterologous nucleotide sequences, and, of replication in a cell either autonomously or in response to one or more factors, a polyadenylation sequence, and a transcription termination sequence. Briefly, eukaryotic layered vector initiation systems provide a two stage or "layered" mechanism which controls expression of heterologous nucleotide sequences. The first layer initiates transcription of the second layer, and comprises a 5' promoter, polyadenylation site, and transcription termination site, as well as one or more splice sites if desired. Representative examples of promoters suitable for use in this regard include any viral or cellular promoters such as CMV, retroviral LTRs, SV40, β-actin, immunoglobulin promoters, and inducible promoters such as the metallothionein promoter and glucocorticoid promoter. The second layer comprises a construct which is capable of expressing one or more heterologous nucleotide sequences, and, of replication in a cell either autonomously or in response to one or more factors. Within one embodiment of the invention the construct may be a Sindbis GDV as described above.

The construct within the eukaryotic layered vector initiation systems of the present invention can be an alphavirus vector construct. Within other embodiments, the construct can be derived from a virus selected from the group consisting of poliovirus, rhinovirus, coxsackieviruses, rubella, yellow fever, HCV, TGEV, IBV, MHV, BCV, parainfluenza virus, mumps virus, measles virus, respiratory syncytial virus, influenza virus, RSV, MoMLV, HIV, HTLV, hepatitis delta virus and Astrovirus. Within yet other embodiments, the promoter which is capable of initiating the 5' synthesis of RNA from cDNA is selected from the group consisting of the MoMLV promoter, metallothionein promoter, glucocorticoid promoter, SV40 promoter, and the CMV promoter. Within further embodiments, the eukaryotic layered vector initiation systems further comprise a polyadenylation sequence.

In further embodiments of the invention, in any of the above aspects the alphavirus cDNA vector construct may be derived from an alphavirus selected from the group consisting of Aura, Fort Morgan, Venezuelan Equine Encephalitis, Ross River, Semliki Forest, Sindbis, and Mayaro.

Representative promoters suitable for use within the present invention include both eukaryotic (*e*.*g*., recognized by RNA polymerase I, II, or III) and prokaryotic promoters, and inducible or non-inducible (*i*.*e*., constitutive) promoters, such as, for example, murine leukemia virus promoters (*e*.*g*., MoMLV), metallothionein promoters, the glucocorticoid promoter, Drosophila actin 5C distal promoter, SV 40 promoter, heat shock protein 65 promoter, heat shock protein 70 promoter, immunoglobulin promoters, mouse polyoma virus promoter (Py), rous sarcoma virus (RSV), BK virus and JC virus promoters, MMTV promoter, alphavirus junction region, CMV MIE promoter, adenovirus VA1RNA, rRNA promoter, tRNA methionine promoter and the *lac* promoter. The second layer comprises a construct which is capable of expressing one or more heterologous nucleotide sequences, and of replication in a cell either autonomously or in response to one or more factors. Within one embodiment of the invention, the second layer construct may be an alphavirus vector construct as described above.

A wide variety of vector systems may be utilized as the first layer of the eukaryotic layered vector initiation system, including for example, viral vector constructs developed from DNA viruses such as those classified in the Poxviridae, including for example canary pox virus or vaccinia virus (*e*.*g*., Fisher-Hoch et al., *PNAS 86*:317-321, 1989; Flexner et al., *Ann*. *N*.*Y*. *Acad*. *Sci*. *569*:86-103, 1989; Flexner et al., *Vaccine 8*:17-21, 1990; U.S. Patent Nos. 4,603,112, 4,769,330 and 5,017,487; WO 89/01973); Papoviridae such as BKV, JCV or SV40 (*e*.*g*., Mulligan et al., *Nature 277*:108-114, 1979); Adenoviridae such as adenovirus (*e*.*g*., Berkner, *Biotechniques 6*:616-627, 1988; Rosenfeld et al., *Science 252*:431-434, 1991); Parvoviridae such as adeno-associated virus (*e.g.,* Samulski et al., *J*. *Vir. 63*:3822-3828, 1989; Mendelson et al., *Virol. 166*:154-165, 1988; PA 7/222,684); Herpesviridae such as Herpes Simplex Virus (*e.g.,* Kit, *Adv*. *Exp*. *Med*. *Biol. 215*:219-236, 1989); and Hepadnaviridae (*e.g.,* HBV), as well as certain RNA viruses which replicate through a DNA intermediate, such as the Retroviridae (*see, e.g.,* U.S. Patent No. 4,777,127, GB 2,200,651, EP 0,345,242 and WO91/02805; Retroviridae include leukemia in viruses such as MoMLV and immunodeficiency viruses such as HIV, *e.g.,* Poznansky, *J. Virol. 65*:532-536, 1991).

Similarly, a wide variety of vector systems may be utilized as second layer of the eukaryotic layered vector initiation system, including for example, vector systems derived from viruses from the families: Picornaviridae (*e.g.,* poliovirus, rhinovirus, coxsackieviruses), Caliciviridae, Togaviridae (*e.g.* alphavirus, rubella), Flaviviridae (*e.g.,* yellow fever), Coronaviridae (*e.g.,* HCV, TGEV, IBV, MHV, BCV), Rhabdoviridae, Filoviridae, Paramyxoviridae (*e.g.,* parainfluenza virus, mumps virus, measles virus, and respiratory syncytial virus), Orthomyxoviridae (*e.g.,* influenza virus), Bunyaviridae, Arenaviridae, Retroviridae (*e.g.,* RSV, MoMLV, HIV, HTLV), hepatitis delta virus and Astrovirus. In addition, non-mammalian RNA viruses (as well as components derived therefrom) may also be utilized, including for example, bacterial and bacteriophage replicases, as well as components derived from plant viruses such as Topamoviruses and Bromoviruses (*see* Strauss et al., *Micro*. *Rev*. *58*:491-562, 1994). Production of eukaryotic layered vector initiation systems is further described in PCT application No. WO 95/08404796, which is incorporated herein by reference.

In a preferred embodiment, the present invention provides multiple GDVs comprising retroviral vectors administered as a single composition. The retroviral vectors are preferably selected from the group comprising KT-1, KT-3, crossless backbone vectors, or a vector that employs a promoter that facilitates tissue- or event-specific expression. Further, the retroviral vector preferably includes one or both of a marker gene, such as neomycin resistance, and a "suicide gene," such as the herpes simplex virus thymidine kinase (HSVTK) gene.

These GDVs are then introduced into suitable packaging cell lines, which cell lines can be selected for particularly desirable characteristics, such as where the GDVs each display amphotropic, xenotropic or polytropic characteristics. Other suitable packaging cell lines include the 293 2-3 VSV-G system, and cell lines that exhibit vector structural protein modified to facilitate targeting of the transduction of the vector to a preferred location (*e*.*g*., a regional lymph node or a cell that presents a particular antigen). The cell lines can then be tested to confirm that they contain the desirable components.

Next, cell cultures are prepared, and supernatant fluids that contain the retroviral vectors are harvested. The fluids can be tested for GDV potency, typically measured in colony forming units (CFU) or plaque forming units (PFU), as appropriate. In a preferred approach, the GDVs are then concentrated and purified prior to administration to the host animal or plant.

### II. Nucleic Acid Molecules

A nucleic acid molecule administered to an animal in accordance with the present invention does not naturally occur in the GDV that carries it, and is neither inert nor generally harmful to the animal, but rather provides some desirable benefit, typically an ability to fight a disease or other pathogenic agent. As used herein, "pathogenic agent" refers to a cell that is responsible for a disease state. Representative examples of pathogenic agents include tumor cells, autoreactive immune cells, hormone secreting cells, cells which lack a function that they would normally have, cells that have an additional inappropriate gene expression which does not normally occur in that cell type, and cells infected with bacteria, viruses, or other intracellular parasites. In addition, as used herein "pathogenic agent" may also refer to a cell that over-expresses or inappropriately expresses a retroviral vector (*e*.*g*., in the wrong cell type), or that has become tumorigenic due to inappropriate insertion into a host cell's genome.

A wide variety of nucleic acid molecules may be carried by the GDV of the present invention. Examples of such nucleic acid molecules include genes and other nucleic acid molecules that encode a substance, as well as biologically active nucleic acid molecules such as inactivating sequences that incorporate into a specified intracellular nucleic acid molecule and inactivate that molecule. A nucleic acid molecule is biologically active when the molecule itself provides the desired benefit, without requiring the expression of a substance. For example, the biologically active nucleic acid molecule may be an inactivating sequence that incorporates into a specified intracellular nucleic acid molecule and inactivates that molecule, or the molecule may be a tRNA, rRNA or mRNA that has a configuration that provides a binding capability.

Substances include proteins (*e.g.,* antibodies including single chain molecules), immunostimulatory molecules (such as antigens) immunosuppressive molecules, blocking agents, and palliatives (such as toxins, antisense ribonucleic acids, ribozymes, enzymes, and other material capable of inhibiting a function of a pathogenic agent). Substances also include cytokines, and various polypeptides or peptide hormones, and their agonists or antagonists, where these hormones can be derived from tissues such as the pituitary, hypothalamus, kidney, endothelial cells, liver, pancreas, bone, hemopoetic marrow, and adrenal. Such substances can be used for induction of growth, regression of tissue, suppression of immune responses, apoptosis, gene expression, blocking receptor-ligand interaction, immune responses and can be used as treatment for certain anemias, diabetes, infections, high blood pressure, abnormal blood chemistry or chemistries (*e*.*g*., elevated blood cholesterol, deficiency of blood clotting factors, elevated LDL with lowered HDL). Such substances can also be used to control levels of Alzheimer associated amyloid protein, bone erosion/calcium deposition, and levels of various metabolites such as steroid hormones, purines, and pyrimidines.

Within the present invention, "capable of inhibiting a function" means that the palliative either directly inhibits the function or indirectly does so, for example, by converting an agent present in the cells from one which would not normally inhibit a function of the pathogenic agent to one which does. Examples of such functions for viral diseases include adsorption, replication, gene expression, assembly, and exit of the virus from infected cells. Examples of such functions for cancerous diseases include cell replication, susceptibility to external signals (*e.g.,* contact inhibition), and lack of production of anti-oncogene proteins. Two or more substances are different substances when they are selected from different groups of substances as described above, and two or more substances are different substances even if they are from the same group (or may even be the same underlying substance, such as the same enzyme or antibody), so long as the substances have a biologically different function, such as a difference in isoelectric point for a desired protein, or a difference in affinity between two versions of the same antibody where the difference causes one substance to be active under different conditions from a second substance. Such differentiation does not include the natural variation that is inherent in the reproduction and expression of virtually all nucleic acids, for example due to polymerase errors during cellular division, transcription or translation. Thus, GDVs that differ only as would be expected from natural variation, but which are otherwise identical, are not different GDVs within the context of the present invention.

Within certain embodiments, the biologically different function is such that the respective nucleic acid sequences carried by the two or more GDVs of the present invention are not simultaneously expressed or incorporated into a genome (or otherwise used for their desired purpose(s)) under all biological conditions; one is "on" (*i*.*e*., have a detectable therapeutic effect) while the other is "off" (*i*.*e*., have no detectable therapeutic effect) under some biological condition.

The GDVs and the substances are preferably selected from different groups as recited above, or otherwise, to provide a synergistic or cooperative effect. However, the GDV or substances in a composition may also exhibit little or no cooperation.

Within one embodiment of the present invention, a method is provided for administration of various GDVs, such as eukaryotic viral cDNA expression vectors, which direct the expression of a palliative as a DNA molecule. Within another embodiment of the present invention, a method is provided for administration of various GDVs which direct the expression of a palliative as an RNA molecule.

Representative examples of palliatives that act directly to inhibit the growth of cells include toxins such as ricin (Lamb et al., *Eur*. *J*. *Biochem. 148*:265-270, 1985), abrin (Wood et al., *Eur*. *J*. *Biochem. 198:*723-732, 1991; Evensen et al., *J*. *of Biol*. *Chem. 266*:6848-6852, 1991; Collins et al., *J. of Biol*. *Chem*. *265*:8665-8669, 1990; Chen et al., *Fed*. *of Eur*. *Biochem Soc. 309*:115-118, 1992), diphtheria toxin (Tweten et al., *J*. *Biol. Chem*. *260*:10392-10394, 1985), cholera toxin (Mekalanos et al., *Nature 306*:551-557, 1983; Sanchez & Holmgren, *PNAS 86*:481-485, 1989), gelonin (Stirpe et al., *J. Biol*. *Chem. 255*:6947-6953, 1980), pokeweed (Irvin, *Pharmac*. *Ther*. *21*:371-387, 1983), antiviral protein (Barbieri et al., *Biochem. J*. *203*:55-59, 1982; Irvin et al., *Arch*. *Biochem*. *& Biophys. 200*:418-425, 1980; Irvin, *Arch*. *Biochem*. *& Biophys. 169*:522-528, 1975), tritin, Shigella toxin (Calderwood et al., *PNAS 84*:4364-4368, 1987; Jackson et al., *Microb*. *Path*. *2*:147-153, 1987), and Pseudomonas exotoxin A (Carroll and Collier, *J*. *Biol. Chem. 262*:8707-8711, 1987).

Within other aspects of the invention, the GDVs carry a gene specifying a product which is not in itself toxic, but when processed or modified by a protein, such as a protease specific to a viral or other pathogen, is converted into a toxic form. For example, the recombinant retrovirus GDV could carry a gene encoding a proprotein chain, which becomes toxic upon processing by the HIV protease. More specifically, a synthetic inactive proprotein form of the toxic ricin or diphtheria A chains could be cleaved to the active form by arranging for the HIV virally encoded protease to recognize and cleave off an appropriate "pro" element.

Within yet another aspect of the invention, recombinant viral vectors are provided carrying a construct which directs the expression of a substance capable of activating an otherwise inactive precursor into an active inhibitor of a pathogenic agent, or a conditional toxic palliative, which are palliatives that are toxic for the cell expressing the pathogenic condition. As will be evident to one of skill in the art given the disclosure provided herein, a wide variety of inactive precursors may be converted into active inhibitors of a pathogenic agent. For example, antiviral nucleoside analogues such as AZT or ddC are metabolized by cellular mechanisms to the nucleotide triphosphate form in order to specifically inhibit retroviral reverse transcriptase, and thus viral replication (Furmam et al., *Proc. Natl. Acad*. *Sci. USA 83*:8333-8337, 1986). Recombinant viral vectors which direct the expression of a gene product (*e.g.,* a protein) such as Herpes Simplex Virus Thymidine Kinase (HSVTK) or Varicella Zoster Virus Thymidine Kinase (VZVTK) which assists in metabolizing antiviral nucleoside analogues to their active form are therefore useful in activating nucleoside analogue precursors (*e.g.,* AZT or ddC) into their active form. AZT or ddC therapy will thereby be more effective, allowing lower doses, less generalized toxicity, and higher potency against productive infection. Additional nucleoside analogues whose nucleotide triphosphate forms show selectivity for retroviral reverse transcriptase but, as a result of the substrate specificity of cellular nucleoside and nucleotide kinases are not phosphorylated, will be made more efficacious.

Within one embodiment of the invention, the HSVTK gene may be expressed under the control of a constitutive macrophage or T-cell-specific promoter, and introduced into macrophage or T-cells. Constitutive expression of HSVTK results in more effective metabolism of nucleotide analogues such as AZT or ddC to their biologically active nucleotide triphosphate form, and thereby provides greater efficacy, delivery of lower doses, less generalized toxicity, and higher potency against productive infection. Additional nucleoside analogues whose nucleotide triphosphate forms show selectivity for retroviral reverse transcriptase but, as a result of the substrate specificity of cellular nucleoside and nucleotide kinases are not phosphorylated, may also be utilized within the context of the present invention.

Within a related aspect of the present invention, a GDV directs the expression of a substance that activates another compound with little or no cytotoxicity into a toxic product in the presence of a pathogenic agent, thereby effecting localized therapy to the pathogenic agent. In this case, expression of the gene product from the GDV is limited to situations wherein an entity associated with the pathogenic agent, such as an intracellular signal identifying the pathogenic state, is present, thereby avoiding destruction of nonpathogenic cells. This cell-type specificity may also be conferred at the level of infection, by targeting GDV carrying the vector to cells having or being susceptible to the pathogenic condition.

Within a related aspect of the present invention, a GDV directs the expression of a gene product(s) that activates a compound with little or no cytotoxicity into a toxic product. Briefly, a wide variety of gene products which either directly or indirectly activate a compound with little or no cytotoxicity into a toxic product may be utilized within the context of the present invention. Representative examples of such gene products include HSVTK and VZVTK which selectively monophosphorylate certain purine arabinosides and substituted pyrimidine compounds, converting them to cytotoxic or cytostatic metabolites. More specifically, exposure of the drugs ganciclovir, acyclovir, or any of their analogues (*e*.*g*., FIAC, DHPG) to HSVTK, phosphorylates the drug into its corresponding active nucleotide triphosphate form.

For example, within one embodiment of the invention, the GDV directs the expression of the herpes simplex virus thymidine kinase ("HSVTK") gene downstream, and under the transcriptional control of an HIV promoter (which is known to be transcriptionally silent except when activated by HIV tat protein (*see* U.S.S.N. 07/586,603)). Briefly, expression of the tat gene product in human cells infected with HIV and carrying the GDV causes increased production of HSVTK. The cells (either *in vitro* or *in vivo*) are then exposed to a drug such as ganciclovir, acyclovir or its analogues (FIAC, DHPG). As noted above, these drugs are known to be phosphorylated by HSVTK (but not by cellular thymidine kinase) to their corresponding active nucleotide triphosphate forms. Acyclovir triphosphates inhibit cellular polymerases in general, leading to the specific destruction of cells expressing HSVTK in transgenic mice (see Borrelli et al., *Proc*. *Natl*. *Acad. Sci. USA 85*:7572, 1988). Those cells containing the recombinant vector and expressing HIV tat protein are selectively killed by the presence of a specific dose of these drugs.

Within one embodiment of the invention, expression of a conditionally lethal HSVTK gene may be made even more HIV-specific by including cis-acting elements in the transcript ("CRS/CAR"), which require an additional HIV gene product (*see* U.S.S.N. 07/586,603), rev, for optimal activity (Rosen et al., *Proc*. *Natl*. *Acad*. *Sci*. *USA 85*:2071, 1988). More generally, cis elements present in mRNAs have been shown in some cases to regulate mRNA stability or translatability. Sequences of this type (*i*.*e*., post-transcriptional regulation of gene expression) may be used for event- or tissue-specific regulation of vector gene expression. In addition, multimerization of these sequences (*i*.*e*., rev-responsive "CRS/CAR" or tat-responsive "TAR" elements for HIV) may be utilized in order to generate even greater specificity.

In a manner similar to the preceding embodiment, GDVs may be generated which carry a gene for phosphorylation, phosphoribosylation, ribosylation, or other metabolism of a purine- or pyrimidine-based drug. Such genes may have no equivalent in mammalian cells, and might come from organisms such as a virus, bacterium, fungus, or protozoan. Representative examples include: *E. coli* guanine phosphoribosyl transferase ("gpt") gene product, which converts thioxanthine into thioxanthine monophosphate (see Besnard et al., *Mol. Cell. Biol. 7*:4139-4141, 1987); alkaline phosphatase, which will convert inactive phosphorylated compounds such as mitomycin phosphate and doxorubicinphosphate to toxic dephosphorylated compounds; fungal (*e.g., Fusarium oxysporum*) or bacterial cytosine deaminase which will convert 5-fluorocytosine to the toxic compound 5-fluorouracil (Mullen, *PNAS 89*:33, 1992); carboxypeptidase G2 which will cleave the glutamic acid from para-N-bis (2-chloroethyl) aminobenzoyl glutamic acid, thereby creating a toxic benzoic acid mustard; and Penicillin-V amidase, which will convert phenoxyacetabide derivatives of doxorubicin and melphalan to toxic compounds. Conditionally lethal gene products of this type have application to many presently known purine- or pyrimidine-based anticancer drugs, which often require intracellular ribosylation or phosphorylation in order to become effective cytotoxic agents. The conditionally lethal gene product could also metabolize a nontoxic drug, which is not a purine or pyrimidine analogue, to a cytotoxic form (see Searle et al., *Brit. J*. *Cancer 53*:377-384, 1986).

These kinds of conditional activation of an inactive precursor into an active product in cells may be achieved using GDVs such as viral vectors including adeno-associated viral vectors, and those with a shorter term effect, *e*.*g*., adenovirus vectors and others mentioned below. Such vectors are capable of efficiently entering cells and expressing proteins encoded by the vector over a period of time from a couple of days to a month or so. This period of time should be sufficient to allow killing of pathogenic cells. In addition, physical methods of gene transfer may be utilized in a similar manner.

Additionally, in the instance where the target pathogen is a mammalian virus, the GDV may be constructed to take advantage of the fact that mammalian viruses in general tend to have "immediate early" genes, which are necessary for subsequent transcriptional activation of other viral promoter elements. Gene products of this nature are excellent candidates for intracellular signals (or "identifying agents") of viral infection. Thus, conditionally lethal genes transcribed from transcriptional promoter elements that are responsive to such viral "immediate early" gene products could specifically kill cells infected with any particular virus. Additionally, since the human α and β interferon promoter elements are transcriptionally activated in response to infection by a wide variety of nonrelated viruses, the introduction of vectors expressing a conditionally lethal gene product like HSVTK, for example, from these viral-responsive elements (VREs) could result in the destruction of cells infected with a variety of different viruses.

In another embodiment of the invention, methods are provided for producing substances such as inhibitor palliatives involving the delivery and expression of defective interfering viral structural proteins, which inhibit viral assembly. In this context, GDVs code for defective gag, pol, env or other viral particle proteins or peptides which inhibit in a dominant fashion the assembly of viral particles. Such inhibition occurs because the interaction of normal subunits of the viral particle is disturbed by interaction with the defective subunits.

One way of increasing the effectiveness of inhibitory palliatives is to express inhibitory genes, such as viral inhibitory genes, in conjunction with the expression of genes which increase the probability of infection of the resistant cell by the virus in question. The result is a nonproductive "dead-end" event which would compete for productive infection events. In the specific case of HIV, GDV may be administered that inhibit HIV replication (by expressing anti-sense tat, etc., as described above) and also overexpress proteins required for infection, such as CD4. In this way, a relatively small number of vector-infected HIV-resistant cells act as a "sink" or "magnet" for multiple nonproductive fusion events with free virus or virally infected cells.

In another embodiment of the invention, methods are provided for the expression of substances such as inhibiting peptides or proteins specific for viral protease. Viral protease cleaves the viral gag and gag/pol proteins into a number of smaller peptides. Failure of this cleavage in all cases leads to complete inhibition of production of infectious retroviral particles. The HIV protease is known to be an aspartyl protease, and these are known to be inhibited by peptides made from amino acids from protein or analogues. GDV that inhibit HIV will express one or multiple fused copies of such peptide inhibitors.

The approaches discussed above should be effective against many virally linked diseases, cancers, or other pathogenic agents.

Within still other embodiments of the invention, a GDV is provided that expresses a palliative, wherein the palliative has a membrane anchor and acts as an anti-tumor agent(s). Such a palliative may be constructed, for example, as an anti-tumor agent - membrane anchor fusion protein. Briefly, the membrane anchor aspect of the fusion protein may be selected from a variety of sequences, including, for example, the transmembrane domain of well known molecules. Generally, membrane anchor sequences are regions of a protein that bind the protein to a membrane. Customarily, there are two types of anchor sequences that attach a protein to the outer surface of a cell membrane: (1) transmembrane regions that span the lipid bilayer of the cell membrane, and interact with the hydrophobic center region (proteins containing such regions are referred to integral membrane proteins), and (2) domains which interact with an integral membrane protein or with the polar surface of the membrane (such proteins are referred to as peripheral, or extrinsic, proteins).

Membrane anchors for use within the present invention may contain transmembrane domains which span the membrane one or more times. For example, in glycophorin and guanylyl cyclase, the membrane binding region spans the membrane once, whereas the transmembrane domain of rhodopsin spans the membrane seven times, and that of the photosynthetic reaction center of Rhodopseudomonas viridis spans the membrane eleven times (*see* Ross et al., *J*. *Biol*. *Chem. 257*:4152, 1982; Garbers, *Pharmac*. *Ther. 50*:337-345, 1991; Engelman et al., *Proc*. *Natl*. *Acad*. *Sci*. *USA 77*:2023, 1980; Heijne and Manoil, *Prot*. *Eng. 4*:109-112, 1990). Regardless of the number of times the protein crosses the membrane, the membrane spanning regions typically have a similar structure. More specifically, the 20 to 25 amino-acid residue portion of the domain that is located inside the membrane generally consists almost entirely of hydrophobic residues (*see* Eisenberg et al., *Ann. Rev. Biochem. 53*:595-623, 1984). For example, 28 of the 34 residues in the membrane spanning region of glycophorin are hydrophobic (*see* Ross et al.; Tomita et al., *Biochemistry 17*:4756-4770, 1978). In addition, although structures such as beta sheets and barrels do occur, the membrane spanning regions typically have an alpha helical structure, as determined by X-ray diffraction, crystallography and cross-linking studies (*see* Eisenberg et al. at 20; Heijne and Manoil at 109). The location of these transmembrane helices within a given sequence can often be predicted based on hydrophobicity plots. Stryer et al., *Biochemistry*, 3rd. ed. 304, 1988. Particularly preferred membrane anchors for use within the present invention include naturally occurring cellular proteins (that are non-immunogenic) which have been demonstrated to function as membrane signal anchors (such as glycophorin).

Within a preferred embodiment of the present invention, a DNA sequence is provided which encodes a membrane anchor - gamma interferon fusion protein. Within one embodiment, this fusion protein may be constructed by genetically fusing the sequence which encodes the membrane anchor of the gamma-chain of the Fc receptor, to a sequence which encodes gamma-interferon.

In yet another aspect, the GDVs provide a therapeutic effect by encoding one or more ribozymes (RNA enzymes) (Haseloff and Gerlach, *Nature 334*:585, 1989) which will cleave, and hence inactivate, RNA molecules corresponding to a pathogenic function. Since ribozymes function by recognizing a specific sequence in the target RNA and this sequence is normally 12 to 17 bp, this allows specific recognition of a particular RNA sequence corresponding to a pathogenic state, such as HIV tat, and toxicity is specific to such pathogenic state. Additional specificity may be achieved in some cases by making this a conditional toxic palliative, as discussed above.

In still another aspect, the GDVs comprise a biologically active nucleic acid molecule that is an antisense sequence (an antisense sequence may also be encoded by a nucleic acid sequence and then produced within a host cell via transcription). In preferred embodiments, the antisense sequence is selected from the group consisting of sequences which encode influenza virus, HIV, HSV, HPV, CMV, and HBV. The antisense sequence may also be an antisense RNA complementary to RNA sequences necessary for pathogenicity. Alternatively, the biologically active nucleic acid molecule may be a sense RNA (or DNA) complementary to RNA sequences necessary for pathogenicity.

More particularly, the biologically active nucleic acid molecule may be an antisense sequence. Briefly, antisense sequences are designed to bind to RNA transcripts, and thereby prevent cellular synthesis of a particular protein, or prevent use of that RNA sequence by the cell. Representative examples of such sequences include antisense thymidine kinase, antisense dihydrofolate reductase (Maher and Dolnick, *Arch*. *Biochem*. *& Biophys. 253*:214-220, 1987; Bzik et al., *PNAS 84*:8360-8364, 1987), antisense HER2 (Coussens et al., *Science 230*:1132-1139, 1985), antisense ABL (Fainstein et al., *Oncogene 4*:1477-1481, 1989), antisense Myc (Stanton et al., *Nature 310*:423-425, 1984) and antisense *ras*, as well as antisense sequences which block any of the enzymes in the nucleotide biosynthetic pathway.

In addition, within a further embodiment of the invention antisense RNA may be utilized as an anti-tumor agent in order to induce a potent Class I restricted response. Briefly, in addition to binding RNA and thereby preventing translation of a specific mRNA, high levels of specific antisense sequences are believed to induce the increased expression of interferons (including gamma-interferon), due to the formation of large quantities of double-stranded RNA. The increased expression of gamma interferon, in turn, boosts the expression of MHC Class I antigens. Preferred antisense sequences for use in this regard include actin RNA, myosin RNA, and histone RNA. Antisense RNA which forms a mismatch with actin RNA is particularly preferred.

In another embodiment, the substances of the invention include a surface protein that is itself therapeutically beneficial. For example, in the particular case of HIV, expression of the human CD4 protein specifically in HIV-infected cells may be beneficial in two ways:
1. Binding of CD4 to HIV env intracellularly could inhibit the formation of viable viral particles much as soluble CD4 has been shown to do for free virus, but without the problem of systematic clearance and possible immunogenicity, since the protein will remain membrane bound and is structurally identical to endogenous CD4 (to which the patient should be immunologically tolerant).
2. Since the CD4/HIV env complex has been implicated as a cause of cell death, additional expression of CD4 (in the presence of excess HIV-env present in HIV-infected cells) leads to more rapid cell death and thus inhibits viral dissemination. This may be particularly applicable to monocytes and macrophages, which act as a reservoir for virus production as a result of their relative refractility to HIV-induced cytotoxicity (which, in turn, is apparently due to the relative lack of CD4 on their cell surfaces).

Still further aspects of the present invention relate to the administration of a GDV capable of immunostimulation. The ability to recognize and defend against foreign pathogens is essential to the function of the immune system. In particular, the immune system must be capable of distinguishing "self' from "nonself" (*i.e.,* foreign), so that the defensive mechanisms of the host are directed toward invading entities instead of against host tissues. Cytolytic T lymphocytes (CTLs) are typically induced, or stimulated, by the display of a cell surface recognition structure, such as a processed, pathogen-specific peptide, in conjunction with a MHC class I or class II cell surface protein.

Diseases suitable to treatment include viral infections such as HIV, HBV and HPV, cancers such as melanomas, renal carcinoma, breast cancer, ovarian cancer and other cancers, and heart disease.

In one embodiment, the invention provides methods for stimulating a specific immune response and inhibiting viral spread by using GDVs that direct the expression of an antigen or modified form thereof in susceptible target cells, wherein the antigen is capable of either (1) initiating an immune response to the viral antigen or (2) preventing the viral spread by occupying cellular receptors required for viral interactions. Expression of the protein may be transient or stable with time. Where an immune response is to be stimulated to a pathogenic antigen, the recombinant viral vector is preferably designed to express a modified form of the antigen which will stimulate an immune response and which has reduced pathogenicity relative to the native antigen. This immune response is achieved when cells present antigens in the correct manner, *i.e.,* in the context of the MHC class I and/or II molecules along with accessory molecules such as CD3, ICAM-1, ICAM-2, LFA-1, or analogs thereof (*e*.*g*., Altmann et al., *Nature 338*:512, 1989). In accordance with a preferred embodiment, cells infected with Sindbis viral vectors are expected to do this efficiently because they closely mimic genuine viral infection and (a) are able to infect non-replicating cells; (b) do not integrate into the host cell genome; and (c) are not associated with any life threatening diseases.

This embodiment of the invention has a further advantage over other systems that might be expected to function in a similar manner, in that the presenter cells are fully viable and healthy, and no other viral antigens (which may well be immunodominant) are expressed. This presents a distinct advantage since the antigenic epitopes expressed can be altered by selective cloning of sub-fragments of the gene for the antigen into the recombinant Sindbis virus, leading to responses against immunogenic epitopes which may otherwise be overshadowed by immunodominant epitopes. Such an approach may be extended to the expression of a peptide having multiple epitopes, one or more of the epitopes being derived from different proteins. Further, this aspect of the invention allows efficient stimulation of cytotoxic T lymphocytes (CTL) directed against antigenic epitopes, and peptide fragments of antigens encoded by sub-fragments of genes, through intracellular synthesis and association of these peptide fragments with MHC Class I molecules. This approach may be utilized to map major immunodominant epitopes for CTL induction.

An immune response can also be achieved by transferring to an appropriate immune cell (such as a T lymphocyte) (a) the gene for the specific T-cell receptor that recognizes the antigen of interest (in the context of an appropriate MHC molecule if necessary), (b) the gene for an immunoglobulin which recognizes the antigen of interest, or (c) the gene for a hybrid of the two which provides a CTL response in the absence of the MHC context. Thus the GDV may be used as an immunostimulant, immunomodulator, or vaccine, etc.

In the particular case of disease caused by HIV infection, where immunostimulation is desired, the antigen generated from the recombinant retroviral genome is of a form which will elicit either or both an HLA class I- or class II-restricted immune response. In the case of HIV envelope antigen, for example, the antigen is preferably selected from gp 160, gp 120, and gp 41, which have been modified to reduce their pathogenicity. In particular, the selected antigen is modified to reduce the possibility of syncytia, to avoid expression of epitopes leading to a disease enhancing immune response, to remove immunodominant, but strain-specific epitopes or to present several strain-specific epitopes, and allow a response capable of eliminating cells infected with most or all strains of HIV. The strain-specific epitopes can be further selected to promote the stimulation of an immune response within an animal which is cross-reactive against other strains of HIV. Antigens from other HIV genes or combinations of genes, such as gag, pol, rev, vif, nef, prot, gag/pol, gag prot, etc., may also provide protection in particular cases.

HIV is only one example. This approach should be effective against many virally linked diseases or cancers where a characteristic antigen (which does not need to be a membrane protein) is expressed, such as in HPV and cervical carcinoma, HTLV-I-induced leukemias, prostate-specific antigen (PSA) and prostate cancer, mutated p53 and colon carcinoma and melanoma, melanoma specific antigens (*e.g.,* MAGEs), and melanoma, mucin and breast cancer.

In accordance with the immunostimulation aspects of the invention, the substances of the present invention may also include "immunomodulatory factors," many of which are set forth above. Immunomodulatory factors refer to factors that, when manufactured by one or more of the cells involved in an immune response, or, which when added exogenously to the cells, causes the immune response to be different in quality or potency from that which would have occurred in the absence of the factor. The factor may also be expressed from a non-GDV derived gene, but the expression is driven or controlled by the GDV. The quality or potency of a response may be measured by a variety of assays known to one of skill in the art including, for example, *in vitro* assays which measure cellular proliferation (*e.g.,* ³H thymidine uptake), and *in vitro* cytotoxic assays (*e.g.,* which measure ⁵¹Cr release) (*see,* Warner et al., *AIDS Res. and Human Retroviruses 7*:645-655, 1991). Immunomodulatory factors may be active both *in vivo* and *ex vivo.*

Representative examples of such factors include cytokines, such as IL-1, IL-2 (Karupiah et al., *J*. *Immunology 144*:290-298, 1990; Weber et al., *J*. *Exp. Med. 166*:1716-1733, 1987; Gansbacher et al., *J*. *Exp*. *Med*. *172*:1217-1224, 1990; U.S. Patent No. 4,738,927), IL-3, IL-4 (Tepper et al., *Cell 57*:503-512, 1989; Golumbek et al., *Science 254*:713-716, 1991; U.S. Patent No. 5,017,691), IL-5, IL-6 (Brakenhof et al., *J*. *Immunol. 139*:4116-4121, 1987; WO 90/06370), IL-7 (U.S. Patent No. 4,965,195), IL-8, IL-9, IL-10, IL-11, IL-12, particularly IL-2, IL-4, IL-6, and IL-12, alpha interferon (Finter et al., *Drugs 42*(5):749-765, 1991; U.S. Patent No. 4,892,743; U.S. Patent No. 4,966,843; WO 85/02862; Nagata et al., *Nature* 284:316-320, 1980; Familletti et al., *Methods in Enz. 78*:387-394, 1981; Twu et al., *Proc. Natl. Acad. Sci. USA 86*:2046-2050, 1989; Faktor et al., *Oncogene 5*:867-872, 1990), beta interferon (Seif et al., *J*. *Virol. 65*:664-671, 1991), gamma interferons (Radford et al., *The American Society of Hepatology* 20082015, 1991; Watanabe et al., *PNAS 86*:9456-9460, 1989; Gansbacher et al., *Cancer Research 50*:7820-7825, 1990; Maio et al., *Can. Immunol. Immunother. 30*:34-42, 1989; U.S. Patent No. 4,762,791; U.S. Patent No. 4,727,138), G-CSF (U.S. Patent Nos. 4,999,291 and 4,810,643), GM-CSF (WO 85/04188), tumor necrosis factors (TNFs) (Jayaraman et al., *J*. *Immunology 144*:942-951, 1990), CD3 (Krissanen et al., *Immunogenetics 26:258-266,* 1987), ICAM-1 (Altman et al., *Nature 338*:512-514, 1989; Simmons et al., *Nature 331:624-627,* 1988), ICAM-2, LFA-1, LFA-3 (Wallner et al., *J*. *Exp*. *Med*. *166*(4):923-932, 1987), MHC class I molecules, MHC class II molecules, B7.1-.3, β₂-microglobulin (Parnes et al., *PNAS 78*:2253-2257, 1981), chaperones, MHC linked transporter proteins or analogs thereof (Powis et al., *Nature 354*:528-531, 1991). Within one preferred embodiment, the gene encodes gamma-interferon.

An example of an immunomodulatory factor cited above is a member of the B7 family of molecules (*e*.*g*., B7.1-.3 costimulatory factor). Briefly, activation of the full functional activity of T cells requires two signals. One signal is provided by interaction of the antigen-specific T cell receptor with peptides which are bound to major histocompatibility complex (MHC) molecules, and the second signal, referred to as costimulation, is delivered to the T cell by antigen presenting cells. The second signal is required for interleukin-2 (IL-2) production by T cells, and appears to involve interaction of the B7.1-.3 molecule on antigen-presenting cells with CD28 and CTLA-4 receptors on T lymphocytes (Linsley et al., *J*. *Exp*. *Med*., *173*:721-730, 1991a and *J*. *Exp*. *Med*., *174*:561-570, 1991). Within one embodiment of the invention, B7.1-.3 may be introduced into tumor cells in order to cause costimulation of CD8⁺ T cells, such that the CD8⁺T cells produce enough IL-2 to expand and become fully activated. These CD8⁺ T cells can kill tumor cells that are not expressing B7 because costimulation is no longer required for further CTL function. Vectors that express both the costimulatory B7.1-.3 factor, and, for example, an immunogenic HBV core protein, may be made utilizing methods which are described herein. Cells transduced with these vectors will become more effective antigen presenting cells. The HBV core-specific CTL response will be augmented from the fully activated CD8⁺ T cell via the costimulatory ligand B7.1-.3.

The choice of which immunomodulatory factor to include within a GDV may be based upon known therapeutic effects of the factor, or, experimentally determined. For example, a known therapeutic effector in chronic hepatitis B infections is alpha interferon. This has been found to be efficacious in compensating a patient's immunological deficit, and thereby assisting recovery from the disease. Alternatively, a suitable immunomodulatory factor may be experimentally determined. Briefly, blood samples are first taken from patients with a hepatic disease. Peripheral blood lymphocytes (PBLs) are restimulated *in vitro* with autologous or HLA matched cells (*e*.*g*., EBV transformed cells) that have been transduced with a GDV which directs the expression of an immunogenic portion of a hepatitis antigen and the immunomodulatory factor. These stimulated PBLs are then used as effectors in a CTL assay with the HLA matched transduced cells as targets. An increase in CTL response over that seen in the same assay performed using HLA matched stimulator and target cells transduced with a vector encoding the antigen alone, indicates a useful immunomodulatory factor. Within one embodiment of the invention, the immunomodulatory factor gamma interferon is particularly preferred.

The present invention also includes immunogenic portions of desired antigens. For example, various immunogenic portions of the HBV S antigens may be combined in order to present an immune response when administered by one of the GDVs described herein. In addition, due to the large immunological variability that is found in different geographic regions for the S open reading frame of HBV, particular combinations of antigens may be preferred for administration in particular geographic regions. Briefly, epitopes that are found in all human hepatitis B virus S samples are defined as determinant "*a*". Mutually exclusive subtype determinants however have also been identified by two-dimensional double immunodiffusion (Ouchterlony, *Progr*. *Allergy 5:*1, 1958). These determinants have been designated "*d*" or "*y*" and "*w*" or *"r"* (LeBouvier, *J*. *Infect*. *123*:671, 1971; Bancroft et al., *J*. *Immunol. 109*:842, 1972; Courouce et al., *Bibl*. *Haematol*. *42*:1-158, 1976). The immunological variability is due to single nucleotide substitutions in two areas of the hepatitis B virus S open reading frame resulting in the following amino acid changes: (1) exchange of lysine-122 to arginine in the hepatitis B virus S open reading frame causes a subtype shift from *d to y*, and (2) exchange of arginine-160 to lysine causes the shift from subtype *r* to *w*. In black Africa, subtype *ayw* is predominant, whereas in the U.S. and northern Europe the subtype *adw*₂ is more abundant (*Molecular Biology of the Hepatitis B Virus,* McLachlan (ed.), CRC Press, 1991). As will be evident to one of ordinary skill in the art, it is generally preferred to construct a vector for administration which is appropriate to the particular hepatitis B virus subtype which is prevalent in the geographical region of administration. Subtypes of a particular region may be determined by two-dimensional double immunodiffusion or, preferably, by sequencing the S open reading frame of HBV virus isolated from individuals within that region.

Also presented by HBV are pol ("HBV pol"), ORF 5, and ORF 6 antigens. Briefly, the polymerase open reading frame of HBV encodes reverse transcriptase activity found in virions and core-like particles in infected liver. The polymerase protein consists of at least two domains: the amino terminal domain encodes the protein that primes reverse transcription, and the carboxyl terminal domain which encodes reverse transcriptase and RNase H activity. Immunogenic portions of HBV pol may be determined utilizing methods utilizing GDVs administered in order to generate an immune response within an animal, preferably a warm-blooded animal. Similarly, other HBV antigens such as ORF 5 and ORF 6, (Miller et al., *Hepatology 9*:322-327, 1989), may be expressed utilizing GDVs as described herein.

As noted above, at least one immunogenic portion of a hepatitis B antigen can be incorporated into a GDV. The immunogenic portion(s) which are incorporated into the GDV may be of varying length, although it is generally preferred that the portions be at least 9 amino acids long, and may include the entire antigen. Immunogenicity of a particular sequence is often difficult to predict, although T cell epitopes may be predicted utilizing the HLA A2.1 motif described by Falk et al. *(Nature 351*:290, 1991). From this analysis, peptides may be synthesized and used as targets in an *in vitro* cytotoxic assay. Other assays, however, may also be utilized, including, for example, ELISA which detects the presence of antibodies against the newly introduced vector, as well as assays which test for T helper cells, such as gamma-interferon assays, IL-2 production assays, and proliferation assays. Within one embodiment of the present invention, at least one immunogenic portion of a hepatitis C antigen can be incorporated into a GDV. For example, immunogenic portion(s) of hepatitis C may be found in the C and NS3-NS4 regions since these regions are the most conserved among various types of hepatitis C virus (Houghton et al., *Hepatology 14*:381-388, 1991).

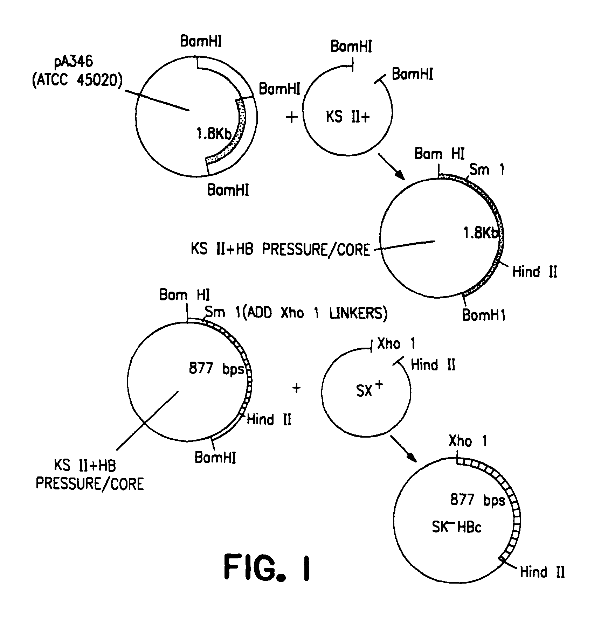

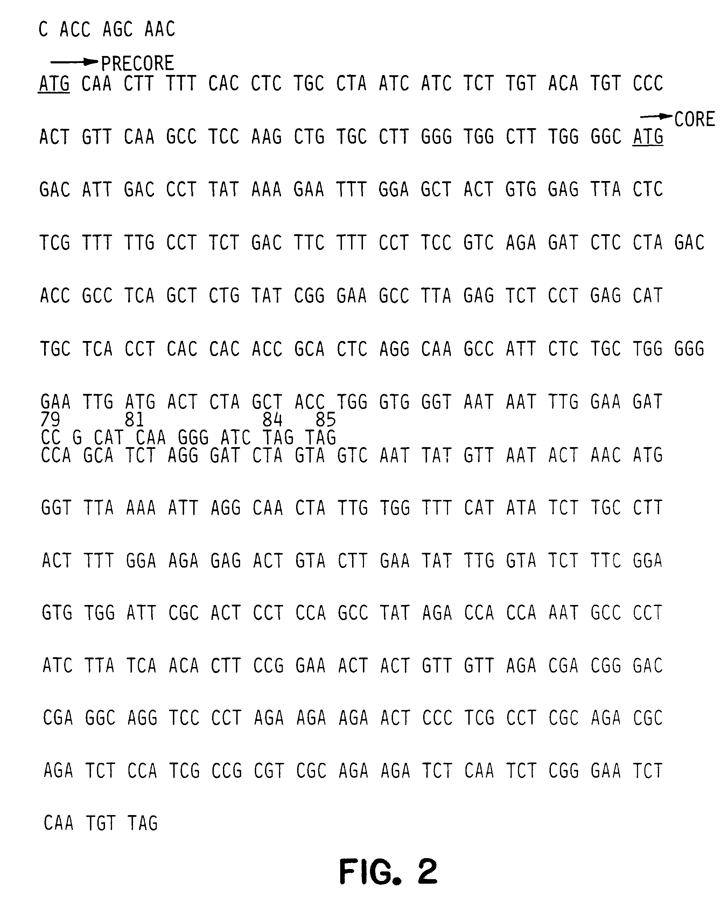

Truncated hepatitis C virus polypeptides designated E1 polypeptides and E2 polypeptides are particularly preferred. By an "E1 polypeptide" is meant a molecule derived from an HCV E1 region. Such a molecule can be physically derived from the region or produced recombinantly or synthetically, based on the known sequence. The mature E1 region of HCV1 begins at approximately amino acid 192 of the polyprotein and continues to approximately amino acid 383 (see Figures 1 and 2). Amino acids at around 173 through approximately 191 serve as a signal sequence for E1. Thus, by an "E1 polypeptide" is meant either a precursor E1 protein, including the signal sequence, or a mature E1 polypeptide which lacks this sequence, or even an E1 polypeptide with a heterologous signal sequence. Furthermore, as elucidated herein, the E1 polypeptide includes a C-terminal membrane anchor sequence which occurs at approximately amino acid positions 360-383. A hepatitis C E1 polypeptide lacking the C-terminus region beginning at about amino acid 360, numbered with reference to the HCV1 amino acid sequence is particularly preferred.

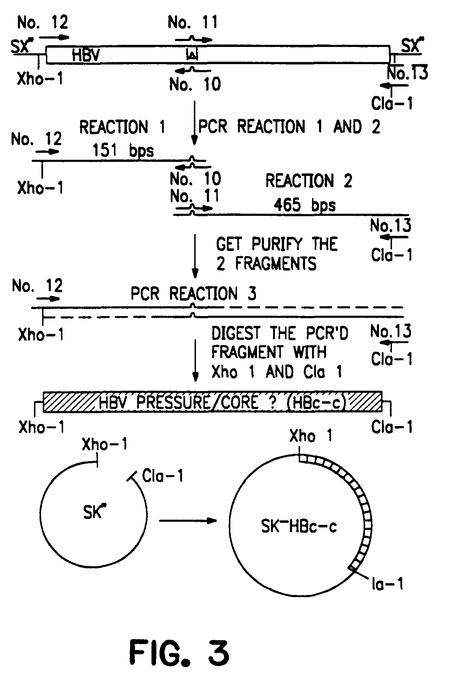

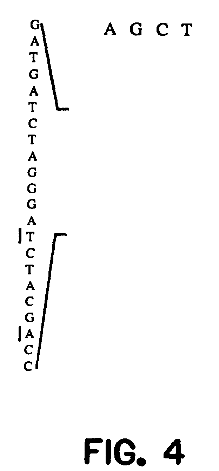

By an "E2 polypeptide" is meant a molecule derived from an HCV E2 region. Such a molecule can be physically derived from the region or produced recombinantly or synthetically, based on the known sequence. The mature E2 region of HCV1 is believed to begin at approximately amino acid 384-385 (see Figures 3 and 4A-4C). A signal peptide begins at approximately amino acid 364 of the polyprotein. Thus, by an "E2 polypeptide" is meant either a precursor E2 protein, including the signal sequence, or a mature E2 polypeptide which lacks this sequence, or even an E1 polypeptide with a heterologous signal sequence. Furthermore, as elucidated herein, the E2 polypeptide includes a C-terminal membrane anchor sequence which occurs at approximately amino acid positions 715-730 and may extend as far as approximately amino acid residue 746 (see, Lin et al., *J*. *Virol.* (1994) 68:5063-5073). A hepatitis C E2 polypeptide lacking its C-terminal sequence beginning at amino acid 715, numbered in reference to the HCV E2 amino acid sequence is particularly preferred.

Immunogenic portions of the hepatitis C antigen can be determined by a variety of methods known to those of skill in the art. For example, as noted above for the hepatitis B virus, identification of immunogenic portions of the polyprotein may be predicted based upon amino acid sequence. Briefly, various computer programs which are known to those of ordinary skill in the art may be utilized to predict CTL epitopes. For example, CTL epitopes for the HLAA2.1 haplotype may be predicted utilizing the HLA A2.1 motif described by Falk et al. (*Nature 351:290*, *1991).* From this analysis, peptides are synthesized and used as targets in an *in vitro* cytotoxic assay.

Within another aspect of the present invention, methods are provided for destroying hepatitis B carcinoma cells comprising the step of administering to a warm-blooded animal a GDV which directs the expression of an immunogenic portion of antigen X, such that an immune response is generated. Sequences which encode the HBxAg may readily be obtained by one of skill in the art given the disclosure provided herein. Briefly, within one embodiment of the present invention, a 642 bp Nco I-Taq I is recovered from ATCC 45020, and inserted into GDVs as described above for other hepatitis B antigens.

The X antigen, however, is a known transactivator which may function in a manner similar to other potential oncogenes (*e*.*g*., E1A). Thus, it is generally preferable to first alter the X antigen such that the gene product is non-tumorigenic before inserting it into a GDV. Various methods may be utilized to render the X antigen non-tumorigenic including, for example, by truncation, point mutation, addition of premature stop codons, or phosphorylation site alteration. Within one embodiment, the sequence or gene of interest which encodes the X antigen is truncated. Truncation may produce a variety of fragments, although it is generally preferable to retain greater than or equal to 50% of the encoding gene sequence. In addition, it is necessary that any truncation leave intact some of the immunogenic sequences of the gene product. Alternatively, within another embodiment of the invention, multiple translational termination codons may be introduced into the gene. Insertion of termination codons prematurely terminates protein expression, thus preventing expression of the transforming portion of the protein.

The X gene or modified versions thereof may be tested for tumorigenicity in a variety of ways. Representative assays include tumor formation in nude mice, colony formation in soft agar, and preparation of transgenic animals, such as transgenic mice.

Within another aspect of the present invention, methods are provided for destroying hepatitis C carcinoma cells comprising the step of administering to a warm-blooded animal a GDV which directs the expression of an immunogenic portion of a hepatitis C antigen. Preferred immunogenic portion(s) of a hepatitis C antigen may be found in the polyprotein which contains the Core antigen and the NS1-NS5 regions (Choo et al., *Proc. Natl. Acad*. *Sci. USA 88*:2451-2455, 1991). Particularly preferred immunogenic portions may be determined by a variety of methods. For example, as noted above preferred immunogenic portions may be predicted based upon amino acid sequence. Briefly, various computer programs which are known to those of ordinary skill in the art may be utilized to predict CTL epitopes. For example, CTL epitopes for the HLA A2.1 haplotype may be predicted utilizing the HLA A2.1 motif described by Falk et al. (*Nature 351*:290, 1991). Another method that may also be utilized to predict immunogenic portions is to determine which portion has the property of CTL induction in mice utilizing retroviral vectors (*see,* Warner et al., *AIDS Res. and Human Retroviruses 7*:645-655, 1991). As noted within Warner et al., CTL induction in mice may be utilized to predict cellular immunogenicity in humans. Preferred immunogenic portions may also be deduced by determining which fragments of the polyprotein antigen or peptides are capable of inducing lysis by autologous patient lymphocytes of target cells (*e*.*g*., autologous EBV-transformed lymphocytes) expressing the fragments after vector transduction of the corresponding genes.

Preferred immunogenic portions may also be selected in the following manner. Briefly, blood samples from a patient with a target disease, such as HCV, are analyzed with antibodies to individual HCV polyprotein regions (*e.g.,* HCV core, E1, E2/SNI and NS2-NS5 regions), in order to determine which antigenic fragments are present in the patient's serum. In patients treated with alpha interferon to give temporary remission, some antigenic determinants will disappear and be supplanted by endogenous antibodies to the antigen. Such antigens are useful as immunogenic portions within the context of the present invention (Hayata et al., *Hepatology 13*:1022-1028, 1991; Davis et al., *N. Eng. J. Med. 321*:1501-1506, 1989).

Additional immunogenic portions of a chosen antigen, such as those from the hepatitis B or C virus, may be obtained by truncating the coding sequence. For example, with HBV the following sites may be truncated: Bst UI, Ssp I, Ppu M1, and Msp I (Valenzuela et al., *Nature 280*:815-19, 1979; Valenzuela et al., *Animal Virus Genetics: ICN*/*UCLA Symp*. *Mol*. *Cell Biol*., 1980, B. N. Fields and R. Jaenisch (eds.), pp. 57-70, New York: Academic). Further methods for determining suitable immunogenic portions as well as methods are also described below in the context of hepatitis C.

With respect to the treatment of HBV, particularly preferred immunogenic portions for incorporation into GDVs include HBeAg, HBcAg, and HBsAgs. Further, more than one immunogenic portion (as well as immunomodulatory factors, if desired) may be incorporated into the GDV. For example, within one embodiment GDVs may be prepared which direct the co-expression of both an immunogenic portion of the hepatitis B antigen, as well as an immunogenic portion of the hepatitis C polyprotein. Such constructs may be administered in order to prevent or treat acute and chronic hepatitis infections of either type B or C. Similarly, within other embodiments, GDVs may be prepared which direct the co-expression of both an immunogenic portion of the hepatitis B X antigen, as well as an immunogenic portion of the hepatitis C polyprotein. Such constructs may similarly be administered in order to treat hepatocellular carcinoma of which is associated with either hepatitis B or C. In addition, because those individuals chronically infected with hepatitis B and C are at higher risk for developing hepatocellular carcinoma, such a vector may also be utilized as a prophylactic treatment for the disease.

Immunogenic portions may also be selected by other methods. For example, the HLA A2.1/K^{b} transgenic mouse has been shown to be useful as a model for human T-cell recognition of viral antigens. Briefly, in the influenza and hepatitis B viral systems, the murine T-cell receptor repertoire recognizes the same antigenic determinants recognized by human T-cells. In both systems, the CTL response generated in the HLA A2.1/K^{b} transgenic mouse is directed toward virtually the same epitope as those recognized by human CTLs of the HLA A2.1 haplotype (Vitiello et al., *J*. *Exp*. *Med*. *173*:1007-1015, 1991; Vitiello et al., *Abstract of Molecular Biology of Hepatitis B Virus Symposia*, 1992).

Immunogenic proteins of the present invention may also be manipulated by a variety of methods known in the art, in order to render them more immunogenic. Representative examples of such methods include: adding amino acid sequences that correspond to T helper epitopes; promoting cellular uptake by adding hydrophobic residues; by forming particulate structures; or any combination of these *(see generally*, Hart, op. cit., Milich et al., *Proc. Natl. Acad*. *Sci. USA 85*:1610-1614, 1988; Willis, *Nature 340*:323-324, 1989; Griffiths et al., *J. Virol. 65*:450-456, 1991).

Still other examples include GDVs which direct the expression of a non-tumorigenic, tumor associated antigen, such as the altered ras (ras*) gene *(see* U.S.S.N. 07/800,328). Briefly, the ras* gene is an attractive target because it is causally linked to the neoplastic phenotype, and indeed may be necessary for the induction and maintenance of tumorigenesis in a wide variety of distinct cancers, such as pancreatic carcinoma, colon carcinoma and lung adenocarcinoma. In addition, ras* genes are found in pre-neoplastic tumors, and therefore immune intervention therapy may be applied prior to detection of a malignant tumor.

Normal ras genes are non-tumorigenic and ubiquitous in all mammals. They are highly conserved in evolution and appear to play an important role in maintenance of the cell cycle and normal growth properties. The normal ras protein is a G-protein which binds GTP and has GTPase activity, and is involved in transmitting signals from the external milieu to the inside of the cell, thereby allowing a cell to respond to its environment. Ras* genes on the other hand alter the normal growth regulation of neoplastic cells by uncoupling cellular behavior from the environment, thus leading to the uncontrolled proliferation of neoplastic cells. Mutation of the ras gene is believed to be an early event in carcinogenesis (Kumar et al., "Activation of ras Oncogenes Preceding the Onset of Neoplasia," *Science 248*:1101-1104, 1990), which, if treated early, may prevent tumorigenesis.

Ras* genes occur in a wide variety of cancers, including for example, pancreatic, colon, and lung adenocarcinomas. However, the spectrum of mutations occurring in the ras* genes found in a variety of cancers is quite limited. These mutations alter the GTPase activity of the ras protein by converting the normal on/off switch to a constitutive ON position. Tumorigenic mutations in ras* occur primarily *(in vivo)* in only 3 codons: 12, 13 and 61. Codon 12 mutations are the most prevalent in both human and animal tumors.

Within another embodiment of the present invention, a GDV is provided which directs the expression of an altered p53 (p53*) gene. Briefly, p53 is a nuclear phosphoprotein which was originally discovered in extracts of transformed cells, and thus was initially classified as an oncogene (Linzer and Levine, *Cell 17*:43-52, 1979; Lane and Crawford, *Nature 278*:261-263, 1979). It was later discovered that the original p53 cDNA clones were mutant forms of p53 (Hinds et al., *J. Virol*. *63*:739-746, 1989). It now appears that p53 is a tumor suppressor gene, which negatively regulates the cell cycle, and that mutation of this gene may lead to tumor formation. Of colon carcinomas that have been studied, 75%-80% show a loss of both p53 alleles, one through deletion, and the other through point mutation. Similar mutations are found in lung cancer, and in brain and breast tumors.

The majority of p53 mutations (*e*.*g*., p53*¹, p53*², etc.) are clustered between amino-acid residues 130 to 290 *(see* Levine et al., *Nature 351*:453-456, 1991; *see also* the following references which describe specific mutations in more detail: Baker et al., *Science 244*:217-221, 1989; Nigro et al., *Nature 342*:705-708, 1989 (p53 mutations cluster at four "hot spots" which coincide with the four highly conserved regions of the genes and these mutations are observed in human brain, breast, lung and colon tumors); Vogelstein, *Nature 348*:681-682, 1990; Takahashi et al., *Science 246*:491-494, 1989; Iggo et al., *Lancet 335*:675-679, 1990; James et al., *Proc*. *Natl*. *Acad*. *Sci*. *USA 86*:2858-2862, 1989; Mackay et al., *Lancet 11*:1384-1385, 1988; Kelman et al., *Blood 74*:2318-2324, 1989; Malkin et al., *Science 250*:1233-1238, 1990; Baker et al., *Cancer Res. 50*:7717-7722, 1991; Chiba et al., *Oncogene 5*:1603-1610, 1990 (pathogenesis of early stage non-small cell lung cancer is associated with somatic mutations in the p53 gene between codons 132 to 283); Prosser et al., *Oncogene 5*:1573-1579, 1990 (mutations in the p53 gene coding for amino acids 126 through 224 were identified in primary breast cancer); Cheng and Hass, *Mol*. *Cell. Biol. 10*:5502-5509, 1990; Bartek et al., *Oncogene 5*:893-899, 1990; Rodrigues et al., *Proc*. *Natl*. *Acad*. *Sci*. *USA 87*:7555-7559, 1990; Menon et al., *Proc*. *Natl*. *Acad*. *Sci*. *USA 87*:5435-5439, 1990; Mulligan et al., *Proc*. *Natl*. *Acad*. *Sci*. *USA 87*:5863-5867, 1990; and Romano et al., *Oncogene 4*:1483-1488, 1990 (identification of a p53 mutation at codon 156 in human osteosarcoma derived cell line HOS-SL)).

Certain alterations of the p53 gene may be due to certain specific toxins. For example, Bressac et al. (*Nature 350*:429-431, 1991) describes specific G to T mutations in codon 249, in patients affected with hepatocellular carcinoma. One suggested causative agent of this mutation is aflatoxin B₁, a liver carcinogen which is known to be a food contaminant in Africa.

Four regions of the gene that are particularly affected occur at residues 132-145, 171-179, 239-248, and 272-286. Three "hot spots" of particular interest occur at residues 175, 248 and 273 (Levine et al., *Nature 351*:453-456, 1991). These alterations as well as others which are described above result in the production of protein(s) which contain novel coding sequence(s). The novel proteins encoded by these sequences may be used as a marker of tumorigenic cells, and an immune response directed against these novel coding regions may be utilized to destroy tumorigenic cells containing the altered sequence (p53*).

Within another embodiment of the present invention, a GDV is provided which directs the expression of an altered Rb (Rb*) gene. Briefly, retinoblastoma is a childhood eye cancer associated with the loss of a gene locus designated Rb, which is located in chromosome band 13q14. A gene from this region has been cloned which produces a nuclear phosphoprotein of about 110 kd (Friend et al., *Nature 323*:643, 1986; Lee et al., *Science 235*:1394, 1987; and Fung et al., *Science 236*:1657, 1987).

Rb is believed to be a negative regulator of cellular proliferation, and has a role in transcriptional control and cell-cycle regulation. Rb binds to at least seven proteins found in the nucleus, and in particular, appears to be involved with a cellular transcription factor which has been designated both E2F (Bagchi et al., *Cell 62*:659-669, 1990) and DRTF (Shivji and La Thangue, *Mol. Cell. Biol. 11*:1686-1695, 1991). Rb is believed to restrict cellular growth by sequestering a variety of nuclear proteins involved in cellular proliferation.

Deletions within the Rb gene have been detected which evidence that the Rb gene may be responsible for tumorigenicity. These deletions include, for example, a deletion in exon 21 in a prostate cancer and bladder cancer cell line (Bookstein et al., *Science 247*:712-715, 1990; Horowitz et al., *Science 243*:937, 1989), a deletion of exon 16 in a small-cell carcinoma of the lung (Shew et al., *Cell Growth and Diff*. *1*:17, 1990), and a deletion between exons 21 and 27 (Shew et al., *Proc. Natl*. *Acad*. *Sci. USA 87*:6, 1990). Deletion of these exons results in the production of a protein containing a novel coding sequence at the junction of the deleted exons. This novel protein coding sequence may be used as a marker of tumorigenic cells, and an immune response directed against this novel coding region may eliminate tumorigenic cells containing the Rb exon deletion.

Within another embodiment of the present invention, a GDV is provided which directs the expression of an altered gene which causes Wilms' tumor. Briefly, Wilms' tumor is typically found in children younger than 16 years of age. One child in 10,000 will develop this tumor, which comprises approximately 5% of childhood cancers. The tumor usually presents itself as a large abdominal mass which is surrounded by a fibrous pseudocapsule. Approximately 7% of the tumors are multifocal in one kidney, and 5.4% are involved with both kidneys. The Wilms' tumor gene has been localized to chromosome 11p13, and a cDNA clone (wt1) has been isolated that is characteristic of a tumor suppressor gene (Call et al., *Cell 60*:509, 1990; Gessler et al., *Nature 343*:744, 1990; Rose et al., *Cell 60*:495, 1990; and Haber et al., *Cell 61*:1257, 1990). The wt1 gene encodes a protein which contains four zinc fingers and a glutamine and proline rich amino terminus. Such structures are believed to be associated with transcriptional and regulatory functions.

Mutations of the Wilms' tumor gene include the insertion of lysine, threonine, and serine between the third and forth zinc fingers. A wt1 protein which contains such insertions does not bind to the EGR-1 site. A second alternative mutation results in the insertion of about 17 amino acids in the region immediately NH₂-terminal to the zinc finger domain (Madden et al., *Science 253*:1550-1553, 1991; Call et al., *Cell 60*:509, 1990; Gessler et al., *Nature 343*:744, 1990; Rose et al., *Cell 60*:495, 1990; Haber et al., *Cell 61*:1257, 1990; and Buckler et al., *Mol. Cell. Biol. 11*:1707, 1991).

Within another embodiment of the present invention, a GDV is provided which directs the expression of an altered mucin. Mucins are large molecular weight glycoproteins which contain approximately 50% carbohydrate. Polymorphic epithelial mucin (PEM) is a tumor-associated mucin (Girling et al., *Int*. *J*. *Cancer 43*:1072-1076, 1989) which is found in the serum of cancer patients. The full-length cDNA sequence has been identified (Gendler et al., *J*. *Biol*. *Chem. 265*(25):15286-15293, 1990; Lan et al., *J*. *Biol*. *Chem. 265*(25):15294-15299, 1990; and Ligtenberg et al., *J*. *Biol*. *Chem*. *265*:5573-5578, 1990). Breast tumors and pancreatic tumors both express a mucin with an identical core sequence, containing a 20 amino-acid tandem repeat (Jerome et al., *Cancer Res*. *51*:2908-2916, 1991). CTL lines which have been developed to breast tumors which cross-react with pancreatic tumor targets, and further appear to specifically recognize the specific 20 amino-acid tandem repeat (Jerome et al., *supra*). A sequence encoding one or more of the 20 amino-acid tandem repeats may be expressed by a GDV of the present invention, in order to develop an immune response against tumor cells which contain this sequence.

Within another embodiment of the present invention, a GDV is provided which directs the expression of an altered DCC (deleted in colorectal carcinomas) gene. Briefly, a very common region of allelic loss in colorectal tumors is chromosome 18q, which is lost in more than 70% of carcinomas, and in almost 50% of late adenomas. A presumptive tumor suppressor gene (DCC) from this region has been identified (Fearon et al., 1990), which encodes a protein with significant homology to cell-surface adhesion molecules, such as neural cell-adhesion molecule (NCAM) and contactin (reviewed by Edelman in *Biochem 27*:3533-3543, 1988). This protein is believed to play a role in the development of colorectal tumors, perhaps through alterations in normal cell-cell and/or cell-extracellular matrix interactions.

The DCC gene is expressed in normal colonic mucosa, but its expression is reduced or absent in the majority of colorectal carcinomas (Solomon, *Nature 343*:412-414, 1990). This loss of expression has been associated in some cases with somatic mutations of the DCC gene. A contiguous stretch of DNA comprising 370 kb has been cloned which encodes an approximately 750 amino acid protein (Fearon et al., "Identification of a Chromosome 18q Gene That Is Altered in Colorectal Cancers," *Science 247*:49-56, 1990).

Within another embodiment of the present invention, a GDV is provided which directs the expression of MCC or APC. Both MCC (mutated in colorectal cancer) and APC have been identified as tumor suppressor genes (Kinzler et al., *Science 251*:1366-1370, 1991) which undergo mutation in familial adenomatous polyposis (FAP). FAP is believed to be the most common autosomal dominant disease which leads to cancer, and it affects at least 1 in 5,000 individuals in the United States (Nishiho et al., *Science 253*:665-669, 1991). Affected individuals usually develop hundreds to thousands of adenomatous polyps of the colon and rectum, which may progress to carcinoma. Gardner's syndrome ("GS," a variant of FAP) presents desmoid tumors, osteomas, and other neoplasms together with multiple adenomas of the colon and rectum. This proliferation is believed to be induced by loss or inactivation of the familial adenomatous polyposis gene (and in particular, MCC and APC) which is found on chromosome 5q.

For example, in Nishiho et al. *(supra),* the following germ line mutations of the APC gene were found in FAP and GS patients: (1) Codon 280, a serine to stop mutation (in a patient with mandibular osteoma), (2) codon 302, an arginine to stop mutation in two separate patients, one with a desmoid tumor, (3) codon 414, an arginine to cysteine mutation in a patient with mandibular osteoma, and (5) codon 713, a serine to stop mutation in another patient with mandibular osteoma (Nishiho et al., *Science 253*:665-669, 1991). In addition, six point mutations were identified in MCC codon numbers 12, 145, 267, 490, 506, and 698, as well as an additional 4 somatic mutations in APC (codons number 289, 332, 438, and 1338).

Within other embodiments of the invention, a GDV is provided which directs the expression of an altered receptor which is functionally locked or stuck in an "ON" or "OFF" mode. Briefly, many cellular receptors are involved in cell growth by monitoring the external environment and signaling the cell to respond appropriately. If either the monitoring or signaling mechanisms fail, the cell will no longer respond to the external environment and may exhibit uncontrolled growth. Many different receptors or receptor-like structures may function as altered cellular components, including, for example, neu and mutated or altered forms of the thyroid hormone receptor, the PDGF receptor, the insulin receptor, the Interleukin receptors (*e*.*g*., IL-1, -2, -3, etc. receptors), or the CSF receptors, such as the G-CSF, GM-CSF, or M-CSF receptors.

For example, neu (also referred to as the Human Epidermal Growth Factor Receptor "HER" or the Epidermal Growth Factor "EGF" receptor) is an altered receptor which is found in at least 28% of women with breast cancer. A cDNA clone which encodes this protein has been isolated (Slamon et al., *Science 244*:707-712, 1989; Slamon et al., *Cancer Cells 7*:371-380, 1989; Shih et al., *Nature 290*:261, 1981). This clone encodes a protein that has extracellular, transmembrane, and intracellular domains (Schechter, *Nature 312*:513, 1984; Coussens et al., *Science 230*:1132, 1985) and thus is believed to encode the neu receptor.

Studies of the rat neu gene isolated from chemically induced neuroglioblastoma cells indicate that it contains a single mutation at position 664 from valine to glutamic acid (Bargmann et al., *EMBO J. 7*:2043, 1988). In other studies, baby rats which were treated with N-ethyl-N-nitrosourea developed malignant tumors of the nervous system. All 47 trigeminal schwannomas and 12 neurinomas which developed carried a T to A transversion at position 664 of the neu gene (Nikitin et al., *Proc. Natl*. *Acad*. *Sci USA 88*:9939-9943, 1991).

Other altered receptors may also be expressed by GDVs in order to destroy selected tumor cells. For example, a deletion in chromosome 3p21-p25 has been associated with small-cell lung carcinomas (Leduc et al., *Am*. *J*. *Hum. Genet. 44*:282-287, 1989). A deletion is believed to occur in the ERBAb gene which otherwise codes for a DNA-binding thyroid hormone receptor (THR).

Alterations in receptors as described above result in the production of protein(s) (or receptors) containing novel coding sequence(s). The novel protein(s) encoded by these sequence(s) may be used as a marker of tumorigenic cells, and an immune response directed against these novel coding region(s) may be utilized to destroy tumorigenic cells containing the altered sequence(s) or gene(s).

If the altered cellular component is associated with making the cell tumorigenic, then, it is necessary to make the altered cellular component non-tumorigenic. For example, within one embodiment, the sequence or gene of interest which encodes the altered cellular component is truncated in order to render the gene product non-tumorigenic. The gene encoding the altered cellular component may be truncated to a variety of sizes, although it is preferable to retain as much as possible of the altered cellular component. In addition, it is necessary that any truncation leave intact at least some of the immunogenic sequences of the altered cellular component. Alternatively, multiple translational termination codons may be introduced into the gene which encodes the altered cellular component, downstream of the immunogenic region. Insertion of termination codons will prematurely terminate protein expression, thus preventing expression of the transforming portion of the protein.

Within one embodiment, the ras* gene is truncated in order to render the ras* protein non-tumorigenic. Briefly, the carboxy-terminal amino acids of ras* functionally allow the protein to attach to the cell membrane. Truncation of these sequences renders the altered cellular component non-tumorigenic. Preferably, the ras* gene is truncated in the purine ring formation, for example around the sequence which encodes amino acid number 110. The ras* gene sequence may be truncated such that as little as about 20 amino acids (including the altered amino acid(s) are encoded by the GDV, although preferably, as many amino acids as possible should be expressed (while maintaining non-tumorigenicity).

Within another embodiment, the p53* protein is modified by truncation in order to render the cellular component non-tumorigenic. As noted above, not all mutations of the p53 protein are tumorigenic, and therefore, not all mutations would have to be truncated. Nevertheless, within a preferred embodiment, p53* is truncated to a sequence which encodes amino acids 100 to 300, thereby including all four major "hot spots."

Other altered cellular components which are oncogenic may also be truncated in order to render them non-tumorigenic. For example, both neu and bcr/abl may be truncated in order to render them non-tumorigenic. Non-tumorigenicity may be confirmed by assaying the truncated altered cellular component as described above.

It should be noted, however, that if the altered cellular component is only associated with non-tumorigenic cells in general, and is not required or essential for making the cell tumorigenic, then it is not necessary to render the cellular component non-tumorigenic. Representative examples of such altered cellular components which are not tumorigenic include Rb*, ubiquitin*, and mucin*.

As noted above, in order to generate an appropriate immune response, the altered cellular component must also be immunogenic. Immunogenicity of a particular sequence is often difficult to predict, although T cell epitopes often possess an immunogenic amphipathic alpha-helix component. In general, however, it is preferable to determine immunogenicity in an assay. Representative assays include an ELISA which detects the presence of antibodies against the newly introduced vector, as well as assays which test for T helper cells such as gamma-interferon assays, IL-2 production assays, and proliferation assays. A particularly preferred method for determining immunogenicity is the CTL assay.

Once a sequence encoding at least one anti-tumor agent has been obtained, it is preferable to ensure that the sequence encodes a non-tumorigenic protein. Various assays are known and may easily be accomplished which assess the tumorigenicity of a particular cellular component. Representative assays include tumor formation in nude mice or rats, colony formation in soft agar, and preparation of transgenic animals, such as transgenic mice.

For this and many other aspects of the invention, tumor formation in nude mice or rats is a particularly important and sensitive method for determining the tumorigenicity of an anti-tumor agent. Nude mice lack a functional cellular immune system (*i.e.,* do not possess CTLs), and therefore provide a useful *in vivo* model in which to test the tumorigenic potential of cells. Normal non-tumorigenic cells do not display uncontrolled growth properties if injected into nude mice. However, transformed cells will rapidly proliferate and generate tumors in nude mice. Briefly, in one embodiment the GDV is delivered to syngeneic murine cells, followed by injection into nude mice. The mice are visually examined for a period of 2 to 8 weeks after injection in order to determine tumor growth. The mice may also be sacrificed and autopsied in order to determine whether tumors are present. (Giovanella et al., *J*. *Natl*. *Cancer Inst*. *48*:1531-1533, 1972; Furesz et al., "Tumorigenicity testing of cell lines considered for production of biological drugs," *Abnormal Cells,* New Products and Risk, Hopps and Petricciani (eds.), Tissue Culture Association, 1985; and Levenbook et al., *J*. *Biol*. *Std*. *13*:135-141, 1985). Tumorigenicity may also be assessed by visualizing colony formation in soft agar (MacPherson and Montagnier, *Vir. 23*:291-294, 1964). Briefly, one property of normal non-tumorigenic cells is "contact inhibition" (*i*.*e*., cells will stop proliferating when they touch neighboring cells). If cells are plated in a semi-solid agar support medium, normal cells rapidly become contact inhibited and stop proliferating, whereas tumorigenic cells will continue to proliferate and form colonies in soft agar.

Transgenic animals, such as transgenic mice, may also be utilized to assess the tumorigenicity of an anti-tumor agent (*e.g.,* Stewart et al., *Cell 38*:627-637, 1984; Quaife et al., *Cell 48*:1023-1034, 1987; and Koike et al., *Proc. Natl. Acad. Sci. USA 86*:5615-5619, 1989). In transgenic animals, the gene of interest may be expressed in all tissues of the animal. This unregulated expression of the transgene may serve as a model for the tumorigenic potential of the newly introduced gene.

In addition to tumorigenicity studies, it is generally preferable to determine the toxicity of the toxic palliatives, such as anti-tumor agent(s), prior to administration. A variety of methods well known to those of skill in the art may be utilized to measure such toxicity, including for example, clinical chemistry assays which measure the systemic levels of various proteins and enzymes, as well as blood cell volume and number. Preferred doses for the assay will be 10⁵, 10⁶, 10⁷, 10⁸, 10⁹, 10¹⁰, and 10¹¹ colony forming units (when the GDV is assayable by drug selection markers) or the same range of plaque forming units for vectors that can be assayed by plaque formation (*e*.*g*., adenoviral vectors) or the same range from selection units for GDV that need to be assayed differently.

Cell mediated and humoral responses may also be induced against a pathogenic agent, particularly viral and bacterial diseases, by administration of immunogenic portion(s) as discussed above. Briefly, immunogenic portions carrying relevant epitopes can be produced in a number of known ways (Ellis and Gerety, *J*. *Med*. *Virol. 31*:54-58, 1990), including chemical synthesis (Bergot et al., *Applied Biosystems Peptide Synthesizer User Bulletin No*. *16*, *1986,* Applied Biosystems, Foster City, California) and DNA expression in recombinant systems, such as the insect-derived baculovirus system (Doerfler, *Current Topics in Immunology 131*:51-68, 1986), mammalian-derived systems (such as CHO cells) (Berman et al., *J*. *Virol*. *63*:3489-3498, 1989), yeast-derived systems (McAleer et al., *Nature 307*:178-180), and prokaryotic systems (Burrel et al., *Nature 279*:43-47, 1979).

The present invention also provides GDVs capable of immune down-regulation. Specific down-regulation of inappropriate or unwanted immune responses, such as in autoimmune or pseudo-autoimmune diseases such as chronic hepatitis, diabetes, rheumatoid arthritis, graft vs. host disease and Alzheimer's, or in transplants of heterologous tissue such as bone marrow, can be engineered using immune-suppressive viral gene products, or active portion thereof, which suppress surface expression of transplantation (MHC) antigen. Within the present invention, an "active portion" of a gene product is that fragment of the gene product which must be retained for biological activity. Such fragments or active domains can be readily identified by systematically removing nucleotide sequences from the protein sequence, transforming target cells with the resulting recombinant GDV, and determining MHC class I presentation on the surface of cells using FACS analysis or other immunological assays, such as a CTL assay. These fragments are particularly useful when the size of the sequence encoding the entire protein exceeds the capacity of the viral carrier. Alternatively, the active domain of the MHC antigen presentation inhibitor protein can be enzymatically digested and the active portion purified by biochemical methods. For example, a monoclonal antibody that blocks the active portion of the protein can be used to isolate and purify the active portion of the cleaved protein (Harlow et al., *Antibodies: A Laboratory Manual,* Cold Springs Harbor, 1988).

Within one embodiment, the suppression is effected by specifically inhibiting the activation of CTL by the display of processed peptides in the context of self MHC molecules along with accessory molecules such as CD8, intercellular adhesion molecule -1 (ICAM-1), ICAM-2, ICAM-3, leukocyte functional antigen-1 (LFA-1) (Altmann et al., *Nature 338*:521, 1989), the B7.1-.3 molecule (Freeman et al., *J*. *Immunol*. *143*:2714, 1989), LFA-3 (Singer, *Science 255*:1671, 1992; Rao, *Crit. Rev*. *Immunol*. *10*:495, 1991), or other cell adhesion molecules. Antigenic peptide presentation in association with MHC class I molecules leads to CTL activation. Transfer and stable integration of specific sequences capable of expressing products expected to inhibit MHC antigen presentation block activation of T-cells, such as CD8⁺ CTL, and therefore suppress graft rejection. A standard CTL assay is used to detect this response. Components of the antigen presentation pathway include the 45 Kd MHC class I heavy chain, β₂-microglobulin, processing enzymes such as proteases, accessory molecules, chaperones, and transporter proteins such as PSF1.

In an alternative example, the recombinant GDV directs the expression of a gene product or an active portion of a gene product capable of binding β₂-microglobulin. Transport of MHC class I molecules to the cell surface for antigen presentation requires association with β₂-microglobulin. Thus, proteins that bind β₂-microglobulin and inhibit its association with MHC class I indirectly inhibit MHC class I antigen presentation. Suitable proteins include the H301 gene product. Briefly, the H301 gene, obtained from the human cytomegalovirus (CMV) encodes a glycoprotein with sequence homology to the β₂-microglobulin binding site on the heavy chain of the MHC class I molecule (Browne et al., *Nature 347*:770, 1990). H301 binds β₂-microglobulin, thereby preventing the maturation of MHC class I molecules, and renders transformed cells unrecognizable by cytotoxic T-cells, thus evading MHC class I restricted immune surveillance.

Within another embodiment, the recombinant GDV directs the expression of a protein or active portion of a protein that binds to newly synthesized MHC class I molecules intracellularly. This binding prevents migration of the MHC class I molecule from the endoplasmic reticulum, resulting in the inhibition of terminal glycosylation. This blocks transport of these molecules to the cell surface and prevents cell recognition and lysis by CTL. For instance, one of the products of the E3 gene may be used to inhibit transport of MHC class I molecules to the surface of the transformed cell. More specifically, E3 encodes a 19 kD transmembrane glycoprotein, E3/19K, transcribed from the E3 region of the adenovirus 2 genome. Within the context of the present invention, tissue cells are transformed with a recombinant GDV containing the E3/19K sequence, which upon expression produces the E3/19K protein. The E3/19K protein inhibits the surface expression of MHC class I surface molecules, and cells transformed by the GDV evade an immune response. Consequently, donor cells can be transplanted with reduced risk of graft rejection and may require only a minimal immunosuppressive regimen for the transplant patient. This allows an acceptable donor-recipient chimeric state to exist with fewer complications. Similar treatments may be used to treat the range of so-called auto immune diseases, including lupus erythromatosis, multiple sclerosis, rheumatoid arthritis or chronic hepatitis B infection.

Another alternative method of immunosuppression involves the use of antisense message, ribozyme, or other gene expression inhibitor specific for T-cell clones which are autoreactive in nature. These block the expression of the T-cell receptor of particular unwanted clones responsible for an auto immune response. The anti-sense, ribozyme, or other gene may be introduced using a viral vector delivery system.

Other proteins, not discussed above, that function to inhibit, suppress or down-regulate MHC class I antigen presentation may also be identified and utilized within the context of the present invention. In order to identify such proteins, in particular those derived from mammalian pathogens (and, in turn, active portions thereof), a recombinant GDV that expresses a protein or an active portion thereof suspected of being capable of inhibiting MHC class I antigen presentation is transformed into a tester cell line, such as BC. The tester cell lines with and without the sequence encoding the candidate protein are compared to stimulators and/or targets in the CTL assay. A decrease in cell lysis corresponding to the transformed tester cell indicates that the candidate protein is capable of inhibiting MHC presentation.

Another alternative method to determine down-regulation of MHC class I surface expression is by FACS analysis. More specifically, cell lines are transformed with a recombinant GDV encoding the candidate protein. After drug selection and expansion, the cells are analyzed by FACS for MHC class I expression and compared to that of non-transformed cells. A decrease in cell surface expression of MHC class I indicates that the candidate protein is capable of inhibiting MHC presentation. This aspect of the present invention is further discussed in co-pending U.S.S.N. 08/116,827.

Many infectious diseases, cancers, auto immune diseases, and other diseases involve the interaction of viral particles with cells, cells with cells, or cells with factors. In viral infections, viruses commonly enter cells via receptors on the surface of susceptible cells. In cancers, cells may respond inappropriately or not at all to signals from other cells or factors. In auto immune disease, there is inappropriate recognition of "self' markers. Within the present invention, such interactions may be blocked by utilizing GDVs that produce, *in vivo*, an analogue to either of the partners in an interaction. Such an analogue is known as a blocking agent.

This blocking action may occur intracellularly, on the cell membrane, or extracellularly. The blocking action of a viral or, in particular, a retroviral GDV carrying a gene for a blocking agent, can be mediated either from inside a susceptible cell or by secreting a version of the blocking protein to locally block the pathogenic interaction.

For example, in the case of HIV, the two agents of interaction are the gp 120/gp 41 envelope protein and the CD4 receptor molecule. Thus, an appropriate blocker would be a GDV expressing either an HIV env analogue that blocks HIV entry without causing pathogenic effects, or a CD4 receptor analogue. The CD4 analogue would be secreted and would function to protect neighboring cells, while the gp 120/gp 41 is secreted or produced only intracellularly so as to protect only the vector-containing cell. It may be advantageous to add human immunoglobulin heavy chains or other components to CD4 in order to enhance stability or complement lysis. Delivery of a retroviral vector encoding such a hybrid-soluble CD4 to a host results in a continuous supply of a stable hybrid molecule.

Vector particles leading to expression of HIV env may also be constructed. It will be evident to one skilled in the art which portions are capable of blocking virus adsorption without overt pathogenic side effects (Willey et al., *J*. *Virol. 62*:139, 1988; Fisher et al., *Science 233*:655, 1986).

Another aspect of the invention involves the delivery of suppressor genes which, when deleted, mutated or not expressed in a cell type, lead to tumorigenesis in that cell type. Reintroduction of the deleted gene by means of a viral vector leads to regression of the tumor phenotype in these cells. Since malignancy can be considered to be an inhibition of cellular terminal differentiation compared with cell growth, the delivery and expression of gene products which lead to differentiation of a tumor should also, in general, lead to regression.

In another alternative embodiment, the GDVs are administered via the use of cationic liposomes *(see* Wang et al., *PNAS 84:* 7851, 1987). These are charged polymers, such as DOTMA and lipofectamine, that naturally complex with nucleic acids in aqueous solution to give complexes that are readily taken up by cells *in vivo* or *ex vivo.*

Sequences which encode the above-described altered cellular components may be obtained from a variety of sources. For example, plasmids which contain sequences that encode altered cellular products may be obtained from a depository such as the American Type Culture Collection (ATCC, Rockville, Maryland), or from commercial sources such as Advanced Biotechnologies (Columbia, Maryland). Representative examples of plasmids containing some of the above-described sequences include ATCC No. 41000 (containing a G to T mutation in the 12th codon of ras), and ATCC No. 41049 (containing a G to A mutation in the 12th codon).

Alternatively, plasmids which encode normal cellular components may also be obtained from depositories such as the ATCC (*see*, *for example*, ATCC No. 41001 which contains a sequence which encodes the normal ras protein, ATCC No. 57103 which encodes abl; and ATCC Nos. 59120 or 59121 which encode the bcr locus) and mutated to form the altered cellular component. Methods for mutagenizing particular sites may readily be accomplished using methods known in the art (*see* Sambrook et al., *supra.,* 15.3 *et seq*.). In particular, point mutations of normal cellular components such as ras may readily be accomplished by site-directed mutagenesis of the particular codon, for example, codons 12, 13 or 61.

Other nucleic acid molecules that encode the above-described substances, as well as other nucleic acid molecules that are advantageous for use within the present invention, may be readily obtained from a variety of sources, including for example depositories such as the American Type Culture Collection (ATCC, Rockville, Maryland), or from commercial sources such as British Bio-Technology Limited (Cowley, Oxford England). Representative examples include BBG 12 (containing the GM-CSF gene coding for the mature protein of 127 amino acids), BBG 6 (which contains sequences encoding gamma interferon), ATCC No. 39656 (which contains sequences encoding TNF), ATCC No. 20663 (which contains sequences encoding alpha interferon), ATCC Nos. 31902, 31902 and 39517 (which contains sequences encoding beta interferon), ATCC No 67024 (which contains a sequence which encodes Interleukin-1b), ATCC Nos. 39405, 39452, 39516, 39626 and 39673 (which contains sequences encoding Interleukin-2), ATCC Nos. 59399, 59398, and 67326 (which contain sequences encoding Interleukin-3), ATCC No. 57592 (which contains sequences encoding Interleukin-4), ATCC Nos. 59394 and 59395 (which contain sequences encoding Interleukin-5), and ATCC No. 67153 (which contains sequences encoding Interleukin-6).

Molecularly cloned genomes which encode the hepatitis B virus may be obtained from a variety of sources including, for example, the American Type Culture Collection (ATCC, Rockville, Maryland). For example, ATCC No. 45020 contains the total genomic DNA of hepatitis B (extracted from purified Dane particles) (*see* Figure 3 of Blum et al., *TIG 5*(5):154-158, 1989) in the Bam HI site of pBR322 (Moriarty et al., *Proc*. *Natl*. *Acad*. *Sci*. *USA 78*:2606-2610, 1981). (Note that correctable errors occur in the sequence of ATCC No. 45020.)

Alternatively, cDNA sequences for use with the present invention may be obtained from cells which express or contain the sequences. Briefly, within one embodiment mRNA from a cell which expresses the gene of interest is reverse transcribed with reverse transcriptase using oligo dT or random primers. The single stranded cDNA may then be amplified by PCR (*see* U.S. Patent Nos. 4,683,202, 4,683,195 and 4,800,159. See also *PCR Technology: Principles and Applications for DNA Amplification,* Erlich (ed.), Stockton Press, 1989) utilizing oligonucleotide primers complementary to sequences on either side of desired sequences. In particular, a double stranded DNA is denatured by heating in the presence of heat stable Taq polymerase, sequence specific DNA primers, ATP, CTP, GTP and TTP. Double-stranded DNA is produced when synthesis is complete. This cycle may be repeated many times, resulting in a factorial amplification of the desired DNA.

Nucleic acid molecules that are suitable for use with the present invention may also be synthesized, for example, on an Applied Biosystems Inc. DNA synthesizer (*e*.*g*., APB DNA synthesizer model 392 (Foster City, California).

### III. Gene Therapy

One further aspect of the present invention is the use of vectors and nucleic acid sequences such as those described above, in combinations to achieve a therapeutic effect or an enhanced therapeutic effect.

In preferred embodiments, the GDVs, preferably retroviral vectors, are selected to provide the following combinations of products and/or effects. Each GDV can encode one or more antigen-based products, such as when one GDV encodes HIV envelope antigen and another GDV encodes HBV core antigen. This combination can be produced, for example, by building one GDV containing the HIV IIIB strain env/rev genes and one GDV containing the HBV adr strain core gene. Alternatively, one GDV can be an antigen-based vector with a second GDV being an immune enhancing gene-based vector, such as where the HIV env gene is in one GDV and IL-2, IFN, or IL-12 is in another. Further, one GDV can be a pro-drug vector (*i*.*e*., provide a conditionally lethal gene product that is activated at a desired time or location) and the other is an immune enhancing gene-based vector, such as where the TK (Thymidine Kinase) gene is in one GDV and the IFN gene is in another. Alternatively, both GDV may deliver antisense genes that block translation of different viral proteins, such as where one GDV provides antisense to rev of HIV and the other GDV provides antisense to RT of HIV; this combination provides the further advantage that there is a lowered chance of a single target virus mutating to escape both antisense molecules.

As an example of the present invention, GDVs can be used to treat Gaucher disease. Gaucher disease is a genetic disorder that is characterized by the deficiency of the enzyme glucocerebrosidase. This type of therapy is an example of a single gene replacement therapy by providing a deficient cellular enzyme. This enzyme deficiency leads to the accumulation of glucocerebroside in the lysosomes of all cells in the body. However, the disease phenotype is manifested only in the macrophages except in the very rare neuronpathic forms of the disease. The disease usually leads to enlargement of the liver and spleen and lesions in the bones. (For a review see *Science 256*:794, 1992 and *The Metabolic Basis of Inherited Disease,* 6th ed., Scriver, et al., vol. 2, p. 1677). Various approaches for treatment have included supplying exogenous enzyme purified from human placenta using various macrophage targeting techniques including resealed erythrocytes coated with immunoglobulin and liposome technology but the clinical improvements in patients was minimal. Not until the human glucocerebrosidase was purified on an industrial scale and modified for macrophage targeting (commercially known as aglucerase™) has significant clinical improvement been observed. However, this treatment can run an average $765,000 per year per patient (see *Science 256*:794, 1992 for review).

In the context of the present invention, when patients are treated with a gene for a protein that they do not have the capacity to express, there may not only be an antibody response to the protein, but also a cellular immune response to the cells making the protein. This can be obviated or minimized by administering a GDV encoding the glucocerebrosidase in combination with a GDV encoding an immune suppressive gene such as adenoviral E3 protein.

### IV. Pharmaceutically Acceptable Carriers and Diluents

Pharmaceutically acceptable carriers or diluents are nontoxic to recipients at the dosages and concentrations employed. Representative examples of carriers or diluents for injectable solutions include water, isotonic saline solutions which are preferably buffered at a physiological pH (such as phosphate-buffered saline or Tris-buffered saline), mannitol, dextrose, glycerol, and ethanol, as well as polypeptides or proteins such as human serum albumin. A preferred composition comprises a vector or recombinant virus in 10 mg/ml mannitol, 1 mg/ml HSA, 20 mM Tris, pH 7.2, and 150 mM NaCl. In this case, since the recombinant vector represents approximately 1 µg of material, it may be less than 1% of high molecular weight material, and less than 1/100,000 of the total material (including water). This composition is stable at -70°C for at least six months.

The pharmaceutically acceptable carrier or diluent may be combined with the GDVs to provide a composition either as a liquid solution, or as a solid form (*e*.*g*., lyophilized) which can be resuspended in a solution prior to administration. The two or more GDVs are typically administered via traditional direct routes, such as buccal/sublingual, rectal, oral, nasal, topical, (such as transdermal and ophthalmic), vaginal, pulmonary, intraarterial, intramuscular, intraperitoneal, subcutaneous, intraocular, intranasal or intravenous, or indirectly. Non-parenteral routes are discussed further in co-pending U.S. patent application No. , attorney's docket number 930049.429, filed contemporaneously herewith.

Within one embodiment, the GDVs include a polycationic molecule to provide polycation-assisted administration. Such a method of gene transfer facilitates delivery of a gene via mediation by a physical particle comprised of multiple components that augment the efficiency and specificity of the gene transfer. In particular, polycationic molecules, such as polylysine and histone, have been shown to neutralize the negative charges on a nucleic acid molecule and to condense the molecule into a compact form. This form of molecule is transferred with high efficiency in cells, apparently through the endocytic pathway. The uptake in expression of the nucleic acid molecule in the host cell results after a series of steps, as follows: (1) attachment to cell surface; (2) cell entry via endocytosis or other mechanisms; (3) cytoplasmic compartment entry following endosome release; (4) nuclear transport; and (5) expression of the nucleic acid molecule carried by the GDVs. In a further preferred embodiment, multi-layer technologies are applied to the polycation-nucleic acid molecule complex to facilitate completion of one or more of these steps. For example, a ligand such as asialoglycoprotein, transferrin, and immunoglobulin may be added to the complex to facilitate binding of the cell complex to the cell surface, an endosomal disruption component (*e*.*g*., a viral protein, a fusogenic peptide such as the n-terminus of the influenza virus hemaglutinin or an inactivated virus) is added to facilitate the release of DNA from the endosome, or a nuclear protein (or a peptide containing a nuclear localization signal) is added to facilitate the transport of the DNA into the nucleus. In a further preferred embodiment, the composition comprising the complex includes inactivated adenovirus particles (Harris, C.E., 1993; Curiel, D.T., 1993; Christiano, R.J., 1993a; Christiano, R.J., 1993b; Cotten, M., 1993; Michael, S.I., 1993; Curiel, D.T., 1992). The assorted components comprising the multi-layer complex may be varied as desired, so that the specificity of the complex for a given tissue, or the gene expressed from the GDV, may be varied to better suit a particular disease or condition.

A feature of this embodiment of the invention is that the complex tends to range in size from 80-100 nm (Wagner, E., 1991), which permits easy access into the endosome vesicle, whose average size is 100-200 nm, and the condensed particle size is not dependent on the molecular weight of the nucleic acid molecule, with a 48 kb DNA molecule transferred into target cells with the same efficiency as a smaller size DNA construct (Cotten, M., 1992).

In an alternative embodiment, the GDVs are administered via the use of DNA gun technology, which comprises the propulsion of microprojectile beads coated with GDV comprising a naked nucleic acid molecule via a detonation device through the cutaneous layer of the subject. Typically, this technique comprises initially precipitating the nucleic acid molecule onto beads (typically on inert material, such as gold or tungsten) using CaPO₄, spermidine, or ethanol, and then accelerating the coated beads directly into cells. This method of administration is particularly preferred for immunoregulation embodiments of the present invention, as the method provides a strong immuno-therapeutic effect, and the relatively inert and non-immunogenic beads facilitate repeated administration of the multiple GDVs.

In another alternative embodiment, the GDVs are administered via the use of liposomes. Liposomes are small, lipid vesicles comprised of an aqueous compartment enclosed by a lipid bilayer, typically spherical or slightly elongated structures and several hundred angstroms in diameter. Many of the properties of liposomes depend upon the lipid composition used for their formation, as well as solvent conditions such as ionic strength, polarity (which may be influenced by the presence of surfactants), pH, and temperature.

For delivery of GDVs, a liposome offers several readily exploited features. Under appropriate conditions, the liposome can fuse with the plasma membrane of a target cell or with the membrane of an endocytic vesicle within a cell which has internalized the liposome, thereby disgorging its contents into the cytoplasm. Prior to interaction with the surface of a target cell, however, the liposome membrane acts as a relatively impermeable barrier which sequesters and protects its contents, for example from degradative enzymes in the plasma. Liposomes have for this reason also been referred to as "micropills". Additionally, because a liposome is a synthetic structure, custom-formulated liposomes can be designed that incorporate desirable features. For example, liposomes have been formed with surface immunoglobulin incorporated into their membrane bilayers for the purpose of targeting. The liposome then binds preferentially to cells bearing an exteriorly disposed antigen for which the vesicle-borne immunoglobulin (*e*.*g*., a monoclonal antibody) has specificity. Other examples include the addition of membrane proteins that facilitate fusion of the liposome membrane with the target cell membrane, or the alteration of the liposome lipid composition (*e*.*g*., the addition of cholesterol) to influence the liposome's phase transition temperature, an index of membrane fluidity/rigidity.

Preparation of liposomes typically involve admixing solutions of one or more purified phospholipids and cholesterol in organic solvents and evaporating the solvent to dryness. An aqueous buffer containing the GDVs is then added to the lipid film and the mixture is sonicated to create a fairly uniform dispersion of liposomes. In certain embodiments, dialysis, gel filtration, or ultracentrifugation is then be used to separate unincorporated components from the intact liposomes. (Stryer, L., *Biochemistry,* pp236-240 1975 (W.H. Freeman, San Francisco); Szoka et al., *Biochim. Biophys. Acta 600*:1-18 (1980); Bayer et al., *Biochim. Biophys. Acta. 550*:464 (1979); Rivnay et al., *Meth. Enzymol. 149*:119 (1987); Wang et al., *PNAS 84:* 7851, 1987 and, Plant et al., *Anal. Biochem. 176*:420 (1989).

Within another embodiment of the present invention, GDV are provided which encode part or all of an unaltered antigen that is, however, tumor associated. Examples include MAGE1, MAGE3, tyrosinase hydroxylase genes (Brichard, V., *J*. *Exp*. *Med*., *178*: 489, 1993), MART1 (Kawakami, Y., *PNAS 91*:3515, 1994), and for melanoma and other cancers. Any such antigen identified as being associated with an anti-tumor response to any specific tumor type can be used.

Alternatively, the GDVs may also be administered via the use of *ex vivo* procedures. Such *ex vivo* procedures include physical and chemical methods of uptake of GDVs into host cells via methods such as calcium phosphate precipitation (Dubensky et al., *PNAS 81*:7529-7533, 1984), direct microinjection of DNA into intact target cells (Acsadi et al., *Nature 352*:815-818, 1991), and electroporation whereby cells suspended in a conducting solution are subjected to an intense electric field in order to transiently polarize the membrane, allowing entry of macromolecules. These cells then become the GDV of the invention. Other procedures include the use of DNA bound to ligand, DNA linked to an inactive adenovirus (Cotton et al., *PNAS 89*:6094, 1990), lipofection (Felgner et al., *Proc. Natl*. *Acad*. *Sci*. *USA 84*:7413-7417, 1989), microprojectile bombardment (Williams et al., *PNAS 88*:2726-2730, 1991), polycation compounds such as polylysine, receptor specific ligands, liposomes entrapping recombinant GDV, spheroplast fusion whereby E. coli containing GDV constructs are stripped of their outer cell walls and fused to animal cells using polyethylene glycol and viral transduction, (Cline et al., *Pharmac. Ther. 29*:69, 1985; and Friedmann et al., *Science 244*:1275, 1989), and DNA ligand (Wu et al, *J*. *of Biol*. *Chem. 264*:16985-16987, 1989), as well as psoralen inactivated viruses such as Sendai or Adenovirus.

In an *ex vivo* context, the transformed cells are transplanted into the animal, and monitored for gene expression. Protocols vary depending on the tissue cells chosen. Briefly, a recombinant GDV carrying a sequence, the expression of which inhibits MHC class I presentation, is transformed into tissue cells. Preferable 10⁵ to 10⁹ tissue cells are transformed. The cells are cultured, and transformed cells may be selected by antibiotic resistance. Cells are assayed for gene expression by Western blot and FACS analysis, or other means. For example, as described in more detail below, bone marrow cells that have been transformed are transplanted in an animal by intravenous administration of 2 to 3 x 10⁷ cells (see WO 93/00051).

Cells that can be transformed include, but are not limited to, fibroblast cells, bone marrow cells, endothelial cells, keratinocytes, hepatocytes, and thyroid follicular cells. Transformed cells may be administered to patients directly by intramuscular, intradermal, subdermal, intravenous, or direct catheter infusion into cavities of the body, or otherwise as discussed herein. *In vivo* gene expression of transduced bone marrow cells is detected by monitoring hematopoesis as a function of hematocrit and lymphocyte production.

As noted above, the GDV may be by a virus such as vaccinia, Sindbis or corona virus. Further methods for administering a GDV comprising a retroviral vector are described in more detail in an application entitled "Recombinant Retroviruses" (*see* U.S.S.N. 07/586,603).

The GDVs are typically purified to a level ranging from 0.25% to 25%, and preferably about 5% to 20% before formulation. Subsequently, after preparation of the composition, where the GDV is a recombinant virus, the recombinant virus will constitute about 10 ng to 1 µg of material per dose, with about 10 times this amount of material present as copurified contaminants. Preferably, the composition is prepared in 0.1-1.0 ml of aqueous solution formulated as described below. The composition is then administered to the host via the appropriate route.

Preferably, the composition, or a representative sample of the composition, is first administered to an animal via the desired route, then the animal is tested for biological response. Such testing may include immunological screening assays (*e*.*g*., CTL assays, antibody assays) for evidence of immune response to the HIV gene products and the HBV gene product. Based upon such testing, the titers of the GDVs may be adjusted to further enhance the desired effect(s). Next, the composition is administered to a human being via the appropriate route, followed by screening assays and other testing to determine the effectiveness of the composition.

In a further embodiment of the present invention, GDV are provided that express proteins that are exported from a transduced cell and provide local or systemic effects. Such proteins include cytokines such as those described above, factor VIII, factor VIII with a deleted B domain, various cytokine or cytokine receptor antagonists (*e.g.*, the naturally occurring IL-1 receptor antagonist), factor IX, erythropoietin, growth hormone, various brain and pituitary derived peptide hormones, angiotensin converting enzyme (ACE) inhibitors, and other vasodilatory or vasoconstricting agents, agents that reduce levels of free radicals (*e.g.*, superoxide dismutase (SOD)), and agents that reduce perceived pain levels.

In the embodiment where one or more, and preferably all, of the GDVs are retroviral vectors, an aqueous suspension containing the GDVs in crude or purified form can be dried by lyophilization or evaporation at ambient temperature. Specifically, lyophilization involves the steps of cooling the aqueous suspension below the glass transition temperature or below the eutectic point temperature of the aqueous suspension, and removing water from the cooled suspension by sublimation to form a lyophilized virus. Briefly, aliquots of the formulated recombinant virus are placed into an Edwards Refrigerated Chamber (3 shelf RC3S unit) attached to a freeze dryer (Supermodulyo 12K). A multistep freeze drying procedure as described by Phillips et al. (*Cryobiology 18:414,* 1981) is used to lyophilize the formulated recombinant virus, preferably from a temperature of -40°C to -45°C. The resulting composition contains less than 10% water by weight of the lyophilized virus. Once lyophilized, the recombinant virus is stable and may be stored at -20°C to -25°C.

With the evaporative method, water is removed from the aqueous suspension at ambient temperature by evaporation. Within one embodiment, water is removed through spray drying (EP 520,748). Within the spray drying process, the aqueous suspension is delivered into a flow of preheated gas, usually air, whereupon water rapidly evaporates from droplets of the suspension. Spray drying apparatus are available from a number of manufacturers (*e*.*g*., Drytec, Ltd., Tonbridge, England; Lab-Plant, Ltd., Huddersield, England). Once dehydrated, the recombinant virus is stable and may be stored at -20°C to -25°C. Within the methods described herein, the resulting moisture content of the dried or lyophilized virus may be determined through use of a Karl-Fischer apparatus (EM Science Aquastar™ V1B volumetric titrator, Cherry Hill, NJ), or through a gravimetric method.

As one example, where the GDV is a retroviral vector, the injection of the vector results in a highly localized action wherein a small number of cells (estimated at about 1 x 10⁵ per injection site from PCR data) are transduced by the retroviral vector. Thus, where a single composition comprising two or more retroviral vectors are injected at the same site, the composition can be formulated such that essentially all transducible cells are transduced with both GDVs, or that the cells are transduced by one GDV and that only a subset are transduced with the second GDV.

The pharmaceutically acceptable compositions of the present invention may also additionally include factors which stimulate cell division, and hence, uptake and incorporation of a GDV such as a recombinant retroviral vector. Representative examples include Melanocyte Stimulating Hormone (MSH), for melanomas, epidermal growth factor (EGF) for breast or other epithelial carcinomas, and the anesthetic bipuvocaine (or related compounds) for intramuscular injection. Particularly preferred methods and compositions for preserving recombinant viruses are described in U.S. applications entitled "Methods for Preserving Recombinant Viruses" (U.S. Serial No. 08/135,938, filed October 12, 1993, and U.S. Serial No. 08/122,791, filed November 15, 1993).

As noted above, the GDV may direct expression of an immunomodulatory cofactor in addition to at least one immunogenic portion of a hepatitis antigen. If the GDV, however, does not express an immunomodulatory cofactor which is a cytokine, this cytokine may be included in the above-described compositions, or may be administered separately (concurrently or subsequently) with the above-described compositions. Briefly, within such an embodiment, the immunomodulatory cofactor is preferably administered according to standard protocols and dosages as prescribed in *The Physician's Desk Reference.* For example, alpha interferon may be administered at a dosage of 1-5 million units/day for 2-4 months, and IL-2 at a dosage of 10,000-100,000 units/kg of body weight, 1-3 times/day, for 2-12 weeks. Gamma interferon may be administered at dosages of 150,000-1,500,000 units 2-3 times/week for 2-12 weeks.

The multiple GDVs may be administered to animals or plants. In preferred embodiments, the animal is a warm-blooded animal, further preferably selected from the group consisting of mice, chickens, cattle, pigs, pets such as cats and dogs, horses, and humans. Alternatively, the animal may be a cold-blooded animal, preferably selected from the group consisting of fish, aquatic vertebrates, and shellfish. Where the host organism is a plant, the GDVs are typically administered by a solution that is taken up by the roots, or via a solution that is sprayed on the leaves. For example, the FLAVR-SAVR® gene (Calgene) may be provided in one GDV while an insect resistance gene is provided the second GDV.

Within a preferred embodiment of the invention, a patient suffering from a non-metastatic, but otherwise untreatable tumor such as glioblastoma, astrocytoma, or other brain tumor, may be treated by injecting purified, concentrated HSVTK vector directly into the tumor. The vector is preferentially integrated and expressed in tumor cells since only growing cells are transducible with retroviral vectors. The vector may express HSVTK in an unregulated fashion or, to promote greater tumor specificity, may express HSVTK from a tissue or event specific promoter which is preferentially expressed in the tumor. For instance, a vector which expresses HSVTK from the CEA promoter may be utilized to treat breast or liver carcinomas. Multiple injections (>10) of vector (approximately 1 ml with a titer of 1x10⁷-1x10¹¹ cfu) can be delivered over an extended period of time (>3 months) since the purified vector contains non-immunogenic quantities of protein (<1 µg protein per 1x10⁷ cfu). Thus, injections may continue until a sizable fraction (>1%) of the tumor cells have become transduced. Vector may be delivered stereotactically before or after debulking surgery or chemotherapy. After *in vivo* transduction has occurred, the transduced tumor cells may be eliminated by treating the patient with pro-drugs that are activated by HSVTK, such as acyclovir or ganciclovir.

Within another embodiment of the present invention, wherein the GDV comprise Vector Producing Cells (also termed "VCLs" or "producer cells"), methods are provided for destroying pathogenic agents in an animal, comprising administering to the animal in order to destroy the pathogenic agent. Within a preferred embodiment, the VCLs may be injected directly into a tumor, thereby allowing for the continual production of retroviral vector *in vivo* and an increase in the efficiency of transduction.

One difficulty with the direct injection of VCLs however, is that in certain instances a very potent immune response may result, thus making such therapy feasible for only a very short term (<2 weeks). Therefore, within preferred embodiments of the invention the immune response against VCLs may be minimized by selecting packaging cell lines made from autologous or HLA-matched human cells. In addition, in order to further limit the immune response against viral structural proteins expressed by the VCLs, the cells may be enclosed in a structure, such as a bead or a bag, which has a semi-permeable membrane, allowing vector particles to diffuse into the tumor, but preventing host immune cells from passing through the membrane and thereby generating an immune response. Methods which decrease the immune response allow additional time for *in vivo* transduction to occur, and thus improves the therapy. In particular embodiments, the VCL encoding a conditionally activated GDV is preferably destroyed by treatment with the conditionally activated GDV (*e.g.,* acyclovir or ganciclovir) after it has accomplished its role in the *in vivo* transduction of cells.

Within another embodiment of the invention, metastatic, but highly localized cancers such as ovarian, neuroblastoma and cervical carcinomas (which are metastatic, but typically remain localized to the peritoneal cavity) may be treated according to the methods of the present invention. Within this embodiment, vector or VCLs may be injected directly into the peritoneal cavity. Within a particularly preferred approach, rapidly growing tumors are preferentially transduced *in vivo* by a HSVTK GDV, and a cytokine encoding (*e*.*g*., γ-interferon or GMCSF) GDV and may be subsequently destroyed by administering acyclovir or ganciclovir to the patient. The cells destroyed by the drug will elicit greatly enhanced immune responses if there is a local production of cytokine.

Within yet another embodiment of the invention, viral vectors or VCLs may be injected into the pleural cavity for the treatment of pleural carcinomatosis arising from lung, breast or colon carcinomas, or by intrathecal injection for the treatment of meningeal carcinomatosis.

Within another embodiment of the invention, patients with metastatic, disseminated cancer may also be treated according to the methods of the present invention. For instance, primary pancreatic carcinomas or colorectal carcinomas that have metastasized to, for example, the liver, may be injected directly with viral vector or VCL of the present invention by inserting a syringe, possibly targeted by stereotaxis, through the body wall. Tumors in the lung or colon may similarly be accessed by bronchoscopy or sigmoidoscopy, respectively. Tumor cells which have been transduced *in vivo* by, for example, a vector which expresses HSVTK, may then be destroyed by administration of acyclovir or ganciclovir to the patient, giving rise to an augmented anti-tumor response in the presence of cytokines which may be present due to the second GDV in the combination.

Within preferred embodiments of the invention, in addition to administration of a cytotoxic gene or gene products (*e*.*g*., HSVTK) as described above, a variety of additional therapeutic compositions may be co-administered or sequentially administered to a warm-blooded animal, in order to inhibit or destroy a pathogenic agent. Such therapeutic compositions may be administered directly, or, within other embodiments, expressed from independent GDVs. Alternatively, a GDV that directs the expression of both a cytotoxic gene or gene product, and a gene which encodes the therapeutic composition (*e*.*g*., a non-vector derived gene as discussed above) may be administered to the warm-blooded animal, in order to inhibit or destroy a pathogenic agent. Within a particularly preferred embodiment, vectors or VCLs which deliver and express both the HSVTK gene and a gene coding for an immune accessory molecule, such as human γ-IFN, may be administered to the patient followed or with another therapeutic vector (*e.g.*, encoding a second cytokine, such as IL-2). In such a construct, one gene may be expressed from the vector LTR and the other may utilize an additional transcriptional promoter found between the LTRs, or may be expressed as a polycistronic mRNA, possibly utilizing an internal ribosome binding site. After *in vivo* gene transfer, the patient's immune system is activated due to the expression of α-IFN and/or IL-2. After this has occurred, the overall tumor burden itself may be reduced by treating the patient with acyclovir or ganciclovir, allowing more effective immune attack of the tumor. Infiltration of the dying tumor with inflammatory cells, in turn, increases immune presentation and further improves the patient's immune response against the tumor.

Thus, in some embodiments, the GDVs can be administered in such a fashion such that the GDVs can either (a) transduce a normal, healthy cell and transform the cell into a producer of a therapeutic protein or other substance which is secreted systemically or (b) transform an abnormal or defective cell, transforming the cell into a normal functioning phenotype. Further, the multiple GDVs are typically administered in the same composition, but may be simultaneously administered at the same time and same site, such as via the use of a double barreled syringe or by joint formulation. A composition containing one or more GDVs may also be administered at different sites, as disclosed in co-pending U.S. patent application No , attorney's docket number 930049.427, filed contemporaneously herewith. The composition also may contain a high titer of virus, where the GDV is a virus, as disclosed in co-pending U.S. patent application No , attorney's docket number 930049.441, filed contemporaneously herewith.

The multiple GDVs may be administered to plants using traditional methods, such as via solutions suitable for uptake by the roots, or via solutions that are sprayed on the leaves.

### EXAMPLES

### Example 1

### Preparation of Retroviral Vector Backbones

### A. Preparation of Retroviral Backbones KT-1 and KT-3B

The Moloney murine leukemia virus (MoMLV) 5' long terminal repeat (LTR) EcoR I-EcoR I fragment, including *gag* sequences, from the N2 vector (Armentano *et al*., *J. Vir. 61*:1647-1650, 1987; Eglitas *et al*., *Science 230*:1395-1398, 1985) is ligated into the plasmid SK⁺ (Stratagene, La Jolla, CA). The resulting construct is designated N2R5. The N2R5 construct is mutated by site-directed *in vitro* mutagenesis to change the ATG start codon to ATT preventing gag expression. This mutagenized fragment is 200 base pairs (bp) in length and flanked by Pst I restriction sites. The Pst I-Pst I mutated fragment is purified from the SK⁺ plasmid and inserted into the Pst I site of N2 MoMLV 5' LTR in plasmid pUC31 to replace the non-mutated 200 bp fragment. The plasmid pUC31 is derived from pUC19 (Stratagene, La Jolla, CA) in which additional restriction sites Xho I, Bgl II, BssH II and Nco I are inserted between the EcoR I and Sac I sites of the polylinker. This construct is designated pUC31/N2R5gM.

A 1.0 kilobase (Kb) MoMLV 3' LTR EcoR I-EcoR I fragment from N2 is cloned into plasmid SK⁺ resulting in a construct designated N2R3⁻. A 1.0 Kb Cla I-Hind III fragment is purified from this construct.

The Cla I-Cla I dominant selectable marker gene fragment from pAFVXM retroviral vector (Kriegler *et al*., *Cell 38*:483, 1984; St. Louis *et al*., *PNAS 85*:3150-3154,1988), comprising a SV40 early promoter driving expression of the neomycin (neo) phosphotransferase gene, is cloned into the SK⁺ plasmid. This construct is designated SK⁺ SV₂-*neo* A 1.3 Kb Cla I-BstB I gene fragment is purified from the SK⁺ SV₂-*neo* plasmid.

KT-3B or KT-1 vectors are constructed by a three part ligation in which the Xho I-Cla I fragment containing the gene of interest and the 1.0 Kb MoMLV 3' LTR Cla I-Hind III fragment are inserted into the Xho I-Hind III site of pUC31/N2R5gM plasmid. This gives a vector designated as having the KT-1 backbone. The 1.3 Kb Cla I-BstB I *neo* gene fragment from the pAFVXM retroviral vector is then inserted into the Cla I site of this plasmid in the sense orientation to yield a vector designated as having the KT-3B backbone.

### B. Preparation of Retroviral Backbone KT-3BC

An alternative selectable marker, phleomycin resistance (Mulsant *et al*., *Som. Cell and Mol*. *Gen. 14*:243, 1988, available from Cayla, Cédex, FR) may be used to make the retroviral backbone KT-3BC, for use in transforming genes to cells that are already neomycin resistant. The plasmid pUT507 (Mulsant *et al*., *Som*. *Cell and Mol*. *Gen. 14*:243, 1988) is digested with Nde I and the ends blunted with Klenow polymerase I. The sample is then digested with Hpa I and Cla I linkers are ligated to the mix of fragments. The sample is then further digested with Cla I. The excess Cla I linkers are removed by digestion with Cla I and the 1.2 Kb Cla I fragment carrying the RSV LTR and the phleomycin resistance gene isolated by agarose gel electrophoresis followed by purification using Geneclean II™ (Bio101, San Diego, CA). This fragment is used in place of the 1.3 Kb Cla I-BstB I neomycin resistance fragment to give the backbone KT-3BC

### Example 2

### Administration of B7 Immuno-Enhancer Expression Vector and HBV Antigen Expression Vector

### A. B7 Immuno-Enhancer Expression Vector

### i. Amplification of the Gene Sequence of Immunomodulatory Cofactor B7-1

Raji cells (ATCC# CCL 86 ), which contains the B7-1 cofactor gene, are suspended at 1.0 x 10⁶ cells/ml to a total volume of 158 ml in five T75 flasks and incubated overnight at 37°C, 5% CO₂. On the following day, cells are harvested in three 50 ml centrifuge tubes. Cell pellets are combined in 50 ml PBS, centrifuged at 2,000 rpm for 10 minutes and supernatant decanted. This procedure is repeated. Poly A⁺ mRNA is isolated using the Micro-Fast Track mRNA Isolation Kit™, version 1.2 (Invitrogen, San Diego, CA). The isolated intact mRNA is used as the template to generate full-length first strand cDNA using the cDNA CYCLE Kit™ (Invitrogen, San Diego, CA), followed by two separate polymerase chain reaction (PCR) amplification reactions. The nucleotide numbering system is obtained from Freeman *et al*. (*J*. *Immunol*. *143*:2714-2722, 1989).

The first PCR amplification is performed with two primers. The sense primer corresponds to the nucleotide sequence 315 to 353 of B7-1. This primer contains the 5' region of the B7-1 open reading frame including the ATG start codon and has two Hind III restriction sites at the 5' end.

The second primer corresponds to the anti-sense nucleotide sequence 1,187 to 1,149 of B7-1. This primer is complementary to the 3' region of the B7-1 open reading frame ending at the TAA stop codon and contains two Xho I restriction sites at the 5' end.

The 868 bp PCR product from the first PCR reaction is ligated into the pCR II plasmid (Invitrogen, San Diego, CA) verified by DNA sequencing and transformed into frozen competent *E. coli* cells. This vector construct is designated pCR II H-Xh-B7-1 and verified by DNA sequencing.

The second PCR amplification is performed with two primers. The sense primer corresponds to the nucleotide sequence 315 to 353 of B7-1. This primer contains the 5' region of the B7-1 open reading frame including the ATG start codon and has two Xho I sites at its 5' end.

The second primer corresponds to the anti-sense nucleotide sequence 1,187 to 1,149 of B7-1. This primer is complementary to the 3' region of the B7-1 open reading frame ending at the TAA stop codon and contains two Apa I restriction sites at the 5' end.

The 868 bp PCR product from the second PCR reaction is ligated into the pCR II plasmid, verified by DNA sequencing and transformed into frozen competent *E*. *coli* cells. This vector construct is designated pCR II Xh-A-B7-1 and verified by DNA sequencing.

### ii. Construction of the B7-1 Retroviral Vector Containing IRBS

pGEM 5Z⁺BIP 5' (Peter Sarnow, University of Colorado, Health Sciences Center, Denver, CO., human immunoglobulin heavy chain binding protein) is digested with Sac I and Sph I. The 250 bp BIP fragment is isolate by 1.5% agarose gel electrophoresis and subcloned into the respective sites of pSP72. The vector construct is designated pSP72 BIP.

The Hind III-Xho I B7-1 sequence is excised from pCR II H-Xh B7-1 of Example 2Ai, and subcloned into the Hind III-Xho I sites of pSP72 BIP. This construct is designated pSP72 H-Xh BIP-B7-1.

The construct pSP72 H-Xh BIP-B7-1 is cleaved at the Xho I site, followed by cleavage with Cla I. This construct is then inserted into the KT-3B at the Xho I-Cla I site as previously described and is designated KT-B7-1 retroviral vector construct.

These constructs are used to make infectious vector particles as described in Examples 2Bva-2Bvc.

### iii. Assay of Utility of B7-1 Vector Expression by Transient Packaging and Transduction of Murine Cells

Cell lines, L33, (Dennert, USC Comprehensive Cancer Center, Los Angeles, CA, Patek, *et*. *al*., *Int*. *J*. *of Cancer 24*:624-628, 1979), BC10ME (Patek, et al., Cell Immuno 72:113, 1982, ATCC# TIB85), L33env, and BCenv (L33*env* and BC*env* express HIV-1 IIIB*env*, Warner et al, AIDS Res. and Human Retrovirus 7:645, 1991), transduced with the KT-B7-1 vector, carrying the amphotropic or VSVG envelope protein are examined for cell surface expression by Flow cytometry analysis. Non-transduced cells are also analyzed for surface expression and compared with B7-1 transduced cells to determine the effect of transduction on cell surface expression.

Murine cell lines, L33-B7-1, L33*env*-B7-1, L33*env*, L33, BC10ME, BC10ME-B7-1, BC*env*, and BC*env*-B7-1, are tested for expression of the B7-1 molecule on the cell surface. Cells grown to subconfluent density are removed from culture dishes by treatment with Versene (Irvine Scientific, Irvine, CA) and washed two times with cold (4°C) phosphate buffered saline (PBS) plus 1% bovine serum albumin (BSA), and 0.02% Na-azide (wash buffer) by centrifugation at 200 xg. Approximately 2.0 x 10⁶ cells are placed in microfuge tubes and pelleted by centrifugation at 200 xg Following removal of the supernatant, cell pellets are resuspended with the monoclonal antibody (Mab) BB1 (Becton Dickinson, Los Angeles, CA) ( 1µg/10⁶ cells) and incubated for 30 minutes at 4°C with occasional mixing. Antibody labeled cells are washed two times with 1 ml of wash buffer (4 °C), centrifuged, and the supernatant removed. Cells are resuspended with a goat anti-mouse IgG FITC conjugated antibody (Fisher Scientific, Tustin, CA) (50µg/10⁶ cells) and incubated for 30 minutes at 4°C. The cells are washed, resuspended in 1 ml of wash buffer, and held on ice prior to analysis on a FACSort Analyzer™ (Becton Dickinson, Los Angeles, CA). The mean fluorescence intensity of transduced cells is compared with that of non-transduced cells to determine the effect B7-1 protein has on surface expression. Additionally, the percent positive versus the percent negative stained cells can also be compared.

### iv. Assay of Utility of B7-1 Vector Expression by Transient Packaging and Transduction of Human Cells

Cell lines transduced with KT-B7-1 are examined for surface expression by flow cytometry analysis. Non-transduced cells are analyzed to compare with KT-B7-1 transduced cells and determine the effect that transduction has on surface expression.

Two human cell lines, JY-B7-1 and JY are tested for expression of the B7-1 molecule on the cell surface. Suspension cells grown to 10⁶ cells/ml are removed from culture flasks by pipet and washed two times with cold (4°C) PBS plus 1% BSA and 0.02% Na-azide (wash buffer) by centrifugation at 200 xg. Approximately 2 x 10⁶ cells are placed in microfuge tubes, pelleted at 200 xg, and the supernatant is removed. Cell pellets are resuspended with the Mab BB1 (Becton Dickinson, Los Angeles, CA) (1µg/10⁶ cells) and incubated for 30 minutes at 4°C with occasional mixing. Antibody labeled cells are washed two times with 1 ml of wash buffer (4°C). Prior to removing the supernatant, the cells are resuspended with a goat anti-mouse IgG FITC conjugated antibody (50µg/10⁶ cells) and incubated for 30 minutes at 4°C. The cells are washed, resuspended in 1 ml of wash buffer, and held on ice prior to analysis on a FACSort Analyzer™. The mean fluorescence intensity of transduced cells is compared with that of non-transduced cells to determine the effect B7-1 protein has on surface expression. Additionally, the percent positive versus the percent negative stained cells can also be compared.

### B. HBV Antigen Expression Vector

### i. Isolation of HBV e/core Sequence

A 1.8 Kb BamH I fragment containing the entire precore/core coding region of hepatitis B virus is obtained from plasmid pAM6 (ATCC No. 45020) and ligated into the BamH I site of KS II⁺ (Stratagene, La Jolla, CA). This plasmid is designated KS II⁺ HBpc/c. Xho I linkers are added to the Stu I site of precore/core in KS II⁺ HBpc/c (at nucleotide sequence 1,704), followed by cleavage with Hinc II (at nucleotide sequence 2,592). The resulting 877 bp Xho I-Hinc II precore/core fragment is cloned into the Xho I/Hinc II site of SK II⁺. This plasmid is designated SK⁺HBe, Figure 1.

### ii. Site-Directed Mutagenesis of HBV e/core Sequence Utilizing PCR

The precore/core gene in plasmid KS II⁺ HB pc/c is sequenced to determine if the precore/core coding region is correct. This sequence was found to have a single base-pair deletion which causes a frame shift at codon 79 that results in two consecutive in-frame TAG stop codons at codons 84 and 85, Figure 2. This deletion is corrected by PCR overlap extension (Ho *et al*., *Gene 77*:51-59, 1989) of the precore/core coding region in plasmid SK⁺ HBe. Four oligonucleotide primers are used for the 3 PCR reactions performed to correct the deletion.

The first reaction utilizes two primers. The sense primer sequence corresponds to the nucleotide sequence 1,855 to 1,827 of the adw strain and contains two Xho I restriction sites at the 5' end. The nucleotide sequence numbering is obtained from Genbank (Intelligenics, Inc., Mountain View, CA).

The second primer sequence corresponds to the anti-sense nucleotide sequence 2,158 to 2,130 of the *adw* strain of hepatitis B virus, and includes codons 79, 84 and 85.

The second reaction also utilizes two primers. The sense primer corresponds to nucleotide sequence 2,130 to 2,158 of the *adw* strain, and includes codons 79, 84 and 85.

The second primer corresponds to the anti-sense nucleotide sequence from SK⁺ plasmid polylinker and contains a Cla I site 135 bp downstream of the stop codon of the HBV precore/core coding region.

The third reaction also utilizes two primers. The sense primer corresponds to nucleotide sequence 5 to 27 of the *adw* strain, and contains two Xho I restriction sites at the 5' end.

The second primer sequence corresponds to the anti-sense nucleotide sequence from the SK⁺ plasmid polylinker and contains a Cla I site 135 bp downstream of the stop codon of the HBV precore/core coding region.

The first PCR reaction corrects the deletion in the anti-sense strand and the second reaction corrects the deletion in the sense strands. PCR reactions one and two correct the mutation from CC to CCA which occurs in codon 79 and a base pair substitution from TCA to TCT in codon 81 (Figure 2). Primer 1 contains two consecutive Xho I sites 10 bp upstream of the ATG codon of HBV e coding region and primer 4 contains a Cla I site 135 bp downstream of the stop codon of HBV precore/core coding region. The products of the first and second PCR reactions are extended in a third PCR reaction to generate one complete HBV precore/core coding region with the correct sequence (Figure 3).

The PCR reactions are performed using the following cycling conditions: The sample is initially heated to 94°C for 2 minutes. This step, called the melting step, separates the double-stranded DNA into single strands for synthesis. The sample is then heated at 56°C for 30 seconds. This step, called the annealing step, permits the primers to anneal to the single stranded DNA produced in the first step. The sample is then heated at 72°C for 30 seconds. This step, called the extension step, synthesizes the complementary strand of the single stranded DNA produced in the first step. A second melting step is performed at 94°C for 30 seconds, followed by an annealing step at 56°C for 30 seconds which is followed by an extension step at 72°C for 30 seconds. This procedure is then repeated for 35 cycles resulting in the amplification of the desired DNA product.

The PCR reaction product is purified by gel electrophoresis and transferred onto NA 45 paper (Schleicher and Schuell, Keene, NH). The desired 787 bp DNA fragment is eluted from the NA 45 paper by incubating for 30 minutes at 65°C in 400 µl high salt buffer (1.5 M NaCl, 20 mM Tris, pH 8.0, and 0.1 mM EDTA). Following elution, 500 µl of phenol:chloroform:isoamyl alcohol (25:24:1) is added to the solution. The mixture is vortexed and then centrifuged 14,000 rpm for 5 minutes in a Brinkmann Eppendorf centrifuge (5415L). The aqueous phase, containing the desired DNA fragment, is transferred to a fresh 1.5 ml microfuge tube and 1.0 ml of 100% EtOH is added. This solution is incubated on dry ice for 5 minutes, and then centrifuged for 20 minutes at 10,000 rpm. The supernatant is decanted, and the pellet is rinsed with 500 µl of 70% EtOH. The pellet is dried by centrifugation at 10,000 rpm under vacuum, in a Savant Speed-Vac™ concentrator, and then resuspended in 10 µl deionized H₂O. One microliter of the PCR product is analyzed by 1.5% agarose gel electrophoresis. The 787 Xho I-Cla I precore/core PCR amplified fragment is cloned into the Xho I-Cla I site of the SK⁺ plasmid. This plasmid is designated SK⁺HBe-c. *E*. *coli* (DH5 alpha, Bethesda Research Labs, Gaithersburg, MD) is transformed with the SK⁺HBe-c plasmid and propagated to generate plasmid DNA. The plasmid is then isolated and purified, essentially as described by Birnboim *et al*. (*Nuc*. *Acid Res. 7*:1513, 1979) and Sambrook *et al*. (Molecular Cloning: A Laboratory Manual, Cold Spring Harbor Press, 1989). The SK⁺HBe-c plasmid is analyzed to confirm the sequence of the precore/core gene (Figure 4).

### iii. Isolation of HBV core Sequence

The single base pair deletion in plasmid SK⁺ HBe is corrected by PCR overlap extension as described in Example 2Bii. The following oligonucleotide primers are used in four PCR reactions performed to correct the mutation.

The first reaction utilizes two primers. The sense primer corresponds to the nucleotide sequence for the T-7 promoter of SK⁺ HBe plasmid.

The second primer corresponds to the anti-sense sequence 2,158 to 2,130 of the *adw* strain, and includes codons 79, 84 and 85.

The second reaction utilizes two primers. The anti-sense primer corresponds to the nucleotide sequence for the T-3 promoter present in SK⁺HBe plasmid.

The second primer corresponds to the sense nucleotide sequence 2,130 to 2,158 of the *adw* strain, and includes codons 79, 84 and 85.

The third reaction utilizes two primers. The anti-sense primer corresponds to the nucleotide sequence for the T-3 promoter present in SK⁺HBe plasmid.

The second primer corresponds to the sense sequence of the T-7 promoter present in the SK⁺HBe plasmid.

The PCR product from the third reaction yields the correct sequence for HBV precore/core coding region.

To isolate HBV core coding region, a primer is designed to introduce the Xho I restriction site upstream of the ATG start codon of the core coding region, and eliminate the 29 amino acid leader sequence of the HBV precore coding region. In a fourth reaction, the HBV core coding region is produced using the PCR product from the third reaction and the following two primers.

The sense primer corresponds to the nucleotide sequence 1,885 to 1,905 of the *adw* strain and contains two Xho I sites at the 5' end.

The second primer corresponds to the anti-sense nucleotide sequence for the T-3 promoter present in the SK⁺ HBe plasmid. The approximately 600 bp PCR product from the fourth PCR reaction contains the HBV core coding region and novel Xho I restriction sites at the 5' end and Cla I restriction sites at the 3' end that was present in the multicloning site of SK⁺ HBe plasmid.

Following the fourth PCR reaction, the solution is transferred into a fresh 1.5 ml microfuge tube. Fifty microliters of 3 M sodium acetate is added to this solution followed by 500 µl of chloroform:isoamyl alcohol (24:1). The mixture is vortexed and then centrifuged at 14,000 rpm for 5 minutes. The aqueous phase is transferred to a fresh microfuge tube and 1.0 ml 100% EtOH is added. This solution is incubated at -20°C for 4.5 hours, and then centrifuged at 10,000 rpm for 20 minutes. The supernatant is decanted, and the pellet rinsed with 500 µl of 70% EtOH. The pellet is dried by centrifugation at 10,000 rpm under vacuum and then resuspended in 10 µl deionized H₂O. One microliter of the PCR product is analyzed by 1.5% agarose gel electrophoresis.

### iv. Construction of HBV core Retroviral Vector

The PCR product from Example 2Biii, approximately 600 bp in length, is digested with Xho I and Cla I restriction endonucleases, electrophoresed through a 1.5% agarose gel, and the DNA is purified from the gel slice by Geneclean II™. This Xho I-Cla I HBV core PCR product is inserted into the Xho I and Cla I sites of the KT-3B retroviral vector. The construct is designated KT-HBc.

The HBV core fragment (Xho I-Cla I) from KT-HBc is inserted into the respective sites of pBluescript KS⁺ II. This construct is designated KS⁺ II HBc, and is verified by DNA sequencing.

### v. Transient Transfection and Transduction of Packaging Cell Lines HX and DA with the Vector Constructs KT-B7-1 and KT-HBc

### a. Plasmid DNA Transfection

The packaging cell line, HX (WO92/05266), are seeded at 5.0 x 10⁵ cells on a 10 cm tissue culture dish on day 1 with Dulbecco's Modified Eagle Medium (DMEM) and 10% fetal bovine serum (FBS). On day 2, the media is replaced with 5.0 ml fresh media 4 hours prior to transfection. A standard calcium phosphate-DNA co-precipitation is performed by mixing 40.0 µl 2.5 M CaCl₂, 10 µg plasmid DNA, and deionized H₂O to a total volume of 400 µl. Four hundred microliters of the DNA-CaCl₂ solution is added dropwise with constant agitation to 400 µl precipitation buffer (50 mM HEPES-NaOH, pH 7.1; 0.25 M NaCl and 1.5 mM Na₂HPO₄-NaH₂PO₄). This mixture is incubated at room temperature for 10 minutes. The resultant fine precipitate is added to a culture dish of cells. The cells are incubated with the DNA precipitate overnight at 37°C. On day 3, the media is aspirated and fresh media is added. The supernatant is removed on day 4, passed through a 0.45 µl filter, and stored at -80°C.

Alternatively, 293-2-3 cells (WO 92/05266) ( these are 293 cells expressing gag and pol) are transfected with the vector DNA and the plasmid pMLP-VSVG ( or other VSVG encoding plasmids) to yield VSVG pseudotyped vector particles that are harvested and stored as described above.

### b. Packaging Cell Line Transduction

DA (an amphotropic cell line derived from D 17 cells ATCC No. 183, WO 92/05266) cells are seeded at 5.0 x 10⁵ cells/10 cm tissue culture dish in 10 ml DMEM and 10% FBS, 4 µg/ml polybrene (Sigma, St. Louis, MO) on day 1. On day 2, 3.0 ml, 1.0 ml and 0.2 ml of the freshly collected virus-containing HX media is added to the cells. The cells are incubated with the virus overnight at 37°C. On day 3, the media is removed and 1.0 ml DMEM, 10% FBS with 800 µg/ml G418 is added to the plate. Only cells that have been transduced with the vector and contain the neomycin selectable marker will survive. A G418 resistant pool is generated over a period of a week. The pool of cells is dilution cloned by removing the cells from the plate and counting the cell suspension, diluting the cells suspension down to 10 cells/ml and adding 0.1 ml to each well (1 cell/well) of a 96 well plate (Corning, Corning, NY). Cells are incubated for 14 days at 37°C, 10% CO₂. Twenty-four clones are selected and expanded up to 24 well plates, 6 well plates then 10 cm plates at which time the clones are assayed for expression and the supernatants are collected and assayed for viral titer.

The titer of the individual clones is determined by infection of HT1080 cells, (ATCC No. CCL 121). On day 1, 5.0 x 10⁵ HT1080 cells are plated on each well of a 6 well microtiter plate in 3.0 ml DMEM, 10% FBS and 4 µg/ml polybrene. On day 2, the supernatant from each clone is serially diluted 10 fold and used to infect the HT1080 cells in 1.0 ml aliquots. The media is replaced with fresh DMEM, 10% FBS media, and the cells incubated with the vector overnight at 37°C, 10% CO₂. On day 3, selection of transduced cells is performed by replacing the media with fresh DMEM, 10% FBS media containing 800 µg/ml G418. Cells are incubated at 37°C, 10% CO₂ for 14 days at which time G418 resistant colonies are scored at each dilution to determine the viral titer of each clone as colony forming units(cfu)/ml.

Using these procedures, cell lines are derived that produce greater than or equal to 10⁶ cfu/ml in culture.

The packaging cell line HX is transduced with vector generated from the DA vector producing cell line in the same manner as described for transduction of the DA cells from HX supernatant.

Transduction of the DA or HX cells with vectors lacking a *neo* selectable marker (Example 1) was performed as described above. However, instead of adding G418 to the cells on day 3, the cells are cloned by limiting dilution. Titer is analyzed as described above.

### c. Generation of Producer Cell Line via One Packaging Cell Line

In some situations it may be desirable to avoid using more than one cell line in the process of generating producer lines. In this case, DA cells are seeded at 5.0 x 10⁵ cells on a 10 cm tissue culture dish on day 1 with DMEM and 10% irradiated (2.5 megarads minimum) FBS. On day 2, the media is replaced with 5.0 ml fresh media 4 hours prior to transfection. A standard calcium phosphate-DNA coprecipitation is performed by mixing 60 µl 2.0 M CaCl₂, 10 µg MLP-G plasmid, 10 µg KT-HBe-c or KT-HBc retroviral vector plasmid, and deionized water to a volume of 400 µl. Four hundred microliters of the DNA-CaCl₂ solution is added dropwise with constant agitation to 400 µl 2X precipitation buffer (50 mM HEPES-NaOH, pH 7.1, 0.25 M NaCI and 1.5 mM Na₂HPO₄-NaH₂PO₄). This mixture is incubated at room temperature for 10 minutes. The resultant fine precipitate is added to a culture dish of DA cells plated the previous day. The cells are incubated with the DNA precipitate overnight at 37°C. On day 3, the medium is removed and fresh medium is added. The supernatant containing G-pseudotyped virus is removed on day 4, passed through a 0.45 µl filter and used to infect the DA packaging cell.

DA cells are seeded at 5.0 x 10⁵ cells on a 10 cm tissue culture dish in 10 ml DMEM and 10% FBS, 4 mg/ml polybrene (Sigma, St. Louis, MO) on day 1. On day 2, 2.0 ml, 1.0 ml or 0.5 ml of the freshly collected and filtered G-pseudotyped virus containing supernatant is added to the cells. The cells are incubated with the virus overnight at 37°C. On day 3 the medium is removed and 10 ml DMEM, 10% irradiated FBS with 800 µg/ml G418 is added to the plate. Only cells that have been transduced with the vector and contain the *neo* selectable marker will survive. A G418 resistant pool is generated over the period of 1-2 weeks. The pool is tested for expression and then dilution cloned by removing the cells from the plate, counting the cell suspension, diluting the cell suspension down to 10 cells/ml and adding 0.1 ml to each well (1 cell/well) of a 96-well plate. Cells are incubated for 2 weeks at 37°C, 10% CO₂ Twenty-four clones are selected and expanded up to 24-well plates, then 6-well plates, and finally 10 cm plates, at which time the clones are assayed for expression and the supernatants are collected and assayed for viral titer as described above.

### C. Detection of Replication Competent Retroviruses (RCR)

### i. The Extended S⁺L⁻ Assay

The extended S⁺L⁻ assay determines whether replication competent, infectious virus is present in the supernatant of the cell line of interest. The assay is based on the empirical observation that infectious retroviruses generate foci on the indicator cell line MiCl₁ (ATCC No. CCL 64.1). The MiCl₁ cell line is derived from the Mv1Lu mink cell line (ATCC No. CCL 64) by transduction with Murine Sarcoma Virus (MSV). It is a non-producer, non-transformed, revertant clone containing a replication defective murine sarcoma provirus, S⁺, but not a replication competent murine leukemia provirus, L⁻. Infection of MiCl₁ cells with replication competent retrovirus "activates" the MSV genome to trigger "transformation" which results in foci formation.

Supernatant is removed from the cell line to be tested for presence of replication competent retrovirus and passed through a 0.45 µ filter to remove any cells. On day 1, Mv1Lu cells are seeded at 1.0 x 10⁵ cells per well (one well per sample to be tested) of a 6 well plate in 2 ml DMEM, 10% FBS and 8 µg/ml polybrene. Mv1Lu cells are plated in the same manner for positive and negative controls on separate 6 well plates. The cells are incubated overnight at 37°C, 10% CO₂. On day 2, 1.0 ml of test supernatant is added to the Mv1Lu cells. The negative control plates are incubated with 1.0 ml of media. The positive control consists of three dilutions (200 focus forming units (ffu), 20 ffu and 2 ffu each in 1.0 ml media) of MA virus (referred to as pAm in Miller *et al., Molec. and Cell Biol. 5*:431, 1985) which is added to the cells in the positive control wells. The cells are incubated overnight. On day 3, the media is aspirated and 3.0 ml of fresh DMEM and 10% FBS is added to the cells. The cells are allowed to grow to confluency and are split 1:10 on day 6 and day 10, amplifying any replication competent retrovirus. On day 13, the media on the Mv1Lu cells is aspirated and 2.0 ml DMEM and 10% FBS is added to the cells. In addition, the MiCl₁ cells are seeded at 1.0 x 10⁵ cells per well in 2.0 ml DMEM, 10% FBS and 8 µg/ml polybrene. On day 14, the supernatant from the Mv1Lu cells is transferred to the corresponding well of the MiCl₁ cells and incubated overnight at 37°C, 10% CO₂. On day 15, the media is aspirated and 3.0 ml of fresh DMEM and 10% FBS is added to the cells. On day 21, the cells are examined for focus formation (appearing as clustered, refractile cells that overgrow the monolayer and remain attached) on the monolayer of cells. The test article is determined to be contaminated with replication competent retrovirus if foci appear on the MiCl₁ cells. Using these procedures, it can be shown that the HBV core producer cell lines are not contaminated with replication competent retroviruses.

### ii. Cocultivation of Producer Lines and MdH Marker Rescue Assay

As an alternate method to test for the presence of RCR in a vector-producing cell line, producer cells are cocultivated with an equivalent number of *Mus dunni* (NIH NIAID Bethesda, MD) cells. Small scale cocultivations are performed by mixing of 5.0 x 10⁵ *Mus dunni* cells with 5.0 x 10⁵ producer cells and seeding the mixture into 10 cm plates (10 ml standard culture media/plate, 4 µg/ml polybrene) at day 0. Every 3-4 days the cultures are split at a 1:10 ratio and 5.0 x 10⁵ *Mus dunni* cells are added to each culture plate to effectively dilute out the producer cell line and provide maximum amplification of RCR. On day 14, culture supernatants are harvested, passed through a 0.45 µ cellulose-acetate filter, and tested in the MdH marker rescue assay. Large scale cocultivations are performed by seeding a mixture of 1.0 x 10⁸ *Mus dunni* cells and 1.0 x 10⁸ producer cells into a total of twenty T-150 flasks (30 ml standard culture media/flask, 4 µg/ml polybrene). Cultures are split at a ratio of 1:10 on days 3, 6, and 13 and at a ratio of 1:20 on day 9. On day 15, the final supernatants are harvested, filtered and a portion of each is tested in the MdH marker rescue assay.

The MdH marker rescue cell line is cloned from a pool of *Mus dunni* cells transduced with LHL, a retroviral vector encoding the hygromycin B resistance gene (Palmer *et al., PNAS 84*: 1055-1059, 1987). The retroviral vector can be rescued from MdH cells upon infection of the cells with RCR. One ml of test sample is added to a well of a 6-well plate containing 10⁵ MdH cells in 2 ml standard culture medium (DMEM with 10% FBS, 1% 200 mM L-glutamine, 1% non-essential amino acids) containing 4 µg/ml polybrene. Media is replaced after 24 hours with standard culture medium without polybrene. Two days later, the entire volume of MdH culture supernatant is passed through a 0.45 µ cellulose-acetate filter and transferred to a well of a 6-well plate containing 5.0 x 10⁴ *Mus dunni* target cells in 2 ml standard culture medium containing polybrene. After 24 hours, supernatants are replaced with standard culture media containing 250 µg/ml of hygromycin B and subsequently replaced on days 2 and 5 with media containing 200 µg/ml of hygromycin B. Colonies resistant to hygromycin B appear and are visualized on day 9 post-selection, by staining with 0.2% Coomassie blue.

### D. Transduction of Murine Cells with Vector Construct Particles

The murine fibroblast cell lines BC10ME, B16 and L⁻M(TK⁻) (ATCC No. CCL 1.3) are grown in DMEM containing 4,500 mg/L glucose, 584 mg/L L-glutamine (Irvine Scientific, Santa Ana, CA) and 10% FBS (Gemini, Calabasas, CA).

The BC10ME, B16, and L⁻M(TK⁻) fibroblast cell lines are plated at 1.0 x 10⁵ cells each in a 10 cm dish in DMEM, 10% FBS complete and 4 µg/ml polybrene. Each is transduced with 1.0 ml of the retroviral vector having a vector titer of approximately 10⁵ cfu/ml. Clones are selected in DMEM, 10% FBS and 800 µg/ml G418 as described in Example 2Bvb.

The EL4 (ATCC No. TIB 39) cells and EL4/A2/K^{b} cells (Sherman, L. Scripps Institute, San Diego, CA) are transduced by co-culture with the DA producer cells. Specifically, 1.0 x 10⁶ EL4 cells or 1.0 x 10⁶ EL4/A2/K^{b} are added to 1.0 x 10⁶ irradiated (10,000 rads) DA (vector titer of approximately 10⁵-10⁶) producer cells in RPMI 1640 (Irvine Scientific, Santa Ana, CA), 10% FBS, and 4 µg/ml polybrene on day 1. On day 2, 1.0 x 10⁶ irradiated (10,000 rad) DA producer cells are added to the co-culture. On day 5, selection of the transduced EL4 or EL4/A2/KB cells is initiated with 800 µg/ml G418. The pool is dilution cloned as described in Example 2Bvb.

BC10ME, B16, L⁻M(TK⁻), and EL-4 cells, transduced by vectors that do not carry a selectable marker, are not selected in G418 but are cloned by limiting dilution and assayed for expression as described above.

### E. Transduction of Human Cells with KT- HBc HBV core Antigen or KT-B7-1 Vector Construct Particles

Lymphoblastoid cell lines (LCL) are established for each patient by infecting (transforming) their B-cells with fresh Epstein-Barr virus (EBV) taken from the supernatant of a 3-week-old culture of B95-8, EBV transformed marmoset leukocytes (ATCC No. CRL 1612). Three weeks after EBV-transformation, the LCL are transduced with retroviral vector expressing KT-HBc HBV core antigen or KT-B7-1. Transduction of LCL is accomplished by co-culturing 1.0 x 10⁶ LCL cells with 1.0 x 10⁶ irradiated (10,000 rads) HX producer cells in a 6 cm plate containing 4.0 ml of medium and 4.0 µg/ml polybrene. The culture medium consists of RPMI 1640, 20% heat inactivated FBS (Hyclone, Logan, UT), 5.0 mM sodium pyruvate and 5.0 mM non-essential amino acids. After overnight co-culture at 37°C and 5% CO₂, the LCL suspension cells are removed and 1.0 x 10⁶ cells are again co-cultured for another 6-18 hours in a fresh plate containing 1.0 x 10⁶ irradiated (10,000 rads) HX producer cells. Transduced LCL cells are selected by adding 800 µg/ml G418 and cloned to obtain high expression clones. The Jurkat A2/K^{b} cells (Sherman, L. Scripps Institute, San Diego, CA) are transduced essentially as described for the transduction of LCL cells. LCLs transduced by vectors are not selected in G418; they are cloned by limiting dilution as in Example 2Bvb and assayed for expression as in Example 2F. These cells can be used as targets or *in vitro* stimulators in CTL assays.

### F. Expression of Transduced Genes

### i. ELISA

Cell lysates from cells transduced by KT-HBc are made by washing 1.0 x 10⁷ cultured cells with PBS, resuspending the cells to a total volume of 600 µl on PBS, and sonicating for two 5-second periods at a setting of 30 in a Branson sonicator, Model 350, (Fisher, Pittsburgh, PA) or by freeze thawing three times. Lysates are clarified by centrifugation at 10,000 rpm for 5 minutes.

Core antigen and precore antigen in cell lysates and secreted e antigen in culture supernatant are assayed using the Abbott HBe, rDNA EIA kit™. Another sensitive EIA assay for precore antigen in cell lysates and secreted e antigen in culture supernatant is performed using the Incstar ETI-EB kit™ (Incstar Corporation, Stillwater, MN). A standard curve is generated from dilutions of recombinant hepatitis B core and e antigen obtained from Biogen (Cambridge, MA).

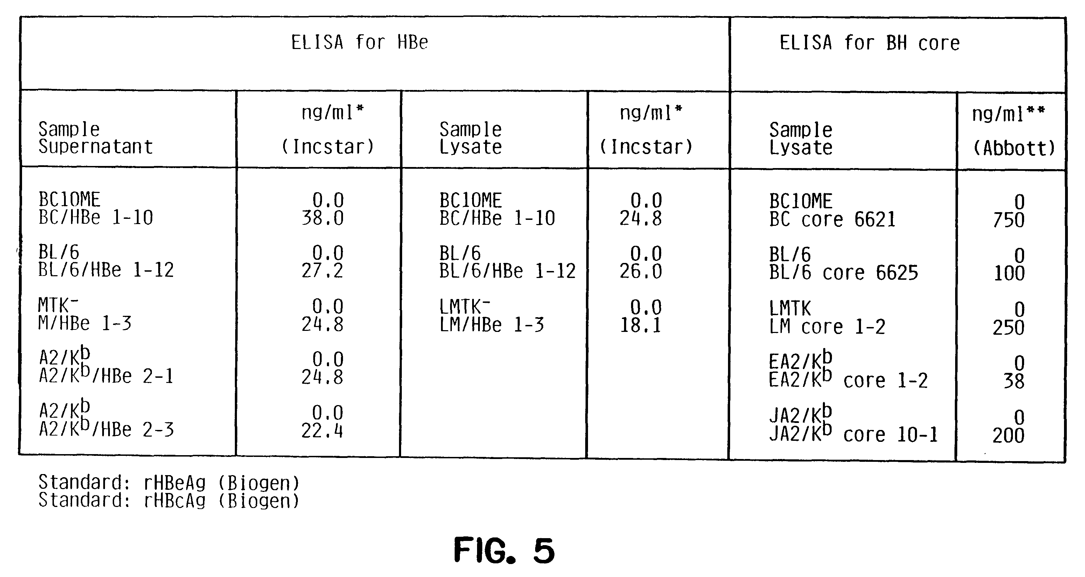

Using these procedures approximately 10 ng/ml HBV e antigen is expressed in transduced cell lines (Figure 5).

### ii. SDS PAGE/Western Blot Analysis

Proteins are separated according to their molecular weight (MW) by means of sodium dodecylsulfate (SDS) polyacrylamide gel electrophoresis. Proteins are then transferred from the gel to a IPVH Immobilon-P™ membrane (Millipore Corp., Bedford, MA). The Hoefer HSI TTE transfer apparatus (Hoefer Scientific Instruments, CA) is used to transfer proteins from the gel to the membrane. The membrane is then probed with polyclonal antibodies from patient serum that reacts specifically with the expressed protein. The bound antibody is detected using ¹²⁵I-labeled protein A, which allows visualization of the transduced protein by autoradiography.

### iii. Immunoprecipitation/Western Blot Analysis

Characterization of the core antigens expressed by transduced cells is performed by immunoprecipitation followed by Western blot analysis. Specifically, 0.5-1.0 ml of cell lysate in PBS or culture supernatant is mixed with polyclonal rabbit anti-hepatitis B core antigen (DAKO Corporation, Carpinteria, CA) bound to G-Sepharose (Pharmacia LKB, Upsala, Sweden) and incubated overnight at 4°C. Samples are washed twice in 20 mM Tris-HCl, pH 8.0, 100 mM NaCl, 10 mM EDTA and boiled in sample loading buffer with 0.5% β-2-mercaptoethanol. Proteins are first resolved by SDS polyacrylamide gel electrophoresis and then transferred to Immobilon (Millipore Corp., Bedford, ME) and probed with the DAKO polyclonal rabbit anti-hepatitis core antigen followed by ¹²⁵I-protein A.

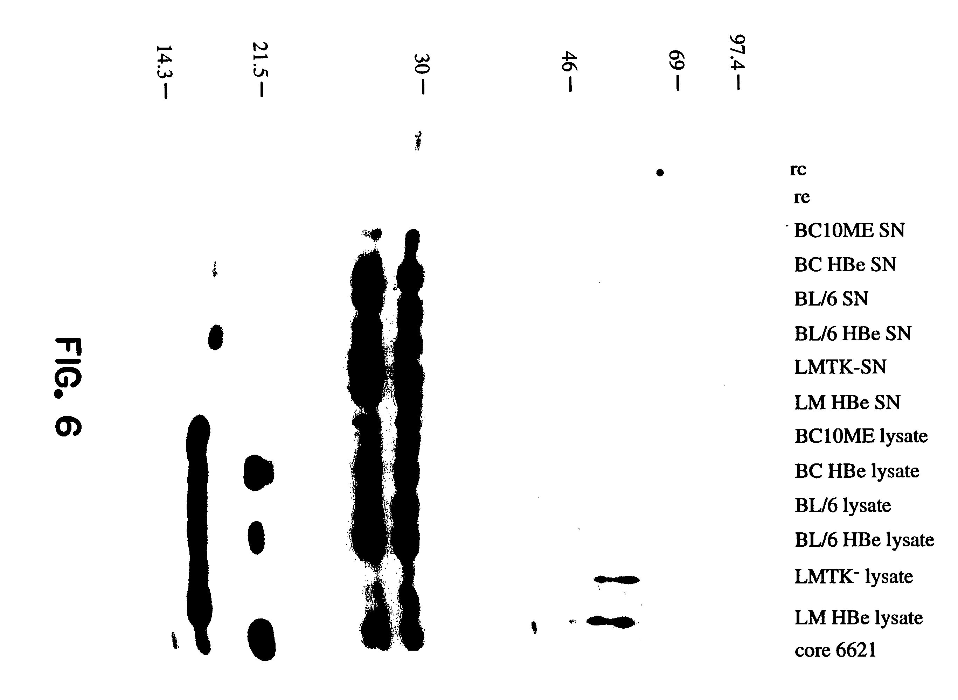

Using these procedures, it can be shown that the HBV core antigen is expressed in transduced mouse cells Figure 6.

### G. Administration of Vector Constructs KT- B7-1 and KT-HBc

### i. Mouse Administration Protocol

Six- to eight-week-old female BALB/c, C57BL/6, C3H/He (Harlan Sprague-Dawley, Indianapolis, IN), HLA A2.1 (Engelhard, V., Charlottesville, VA) HLA A2.1/K^{b} (Sherman, L., Scripps Institute, San Diego, CA) mice or HLA A2.1, human CD8⁺ mice are injected intraperitoneally (I.P.) with 1 ml of mixed media from the B7-1 and HBVc producer cell lines, or intramuscularly (I.M.), intradermally (I.D.), or subcutaneous (S.C.) with 0.1 ml of mixed formulated KT- B7-1 and KT-HBc retroviral vector. These vectors are mixed together prior to injection in a preferred ratio of 1:1. Up to six injections are given at one week intervals. After each injection, sera is removed by retro-orbital bleeds for detection of antibody induction as described in Example 2I. Seven days after the last injection, the animals are sacrificed. ⁵¹Chromium release CTL assays are then performed essentially as described in Example 2H.

### ii. Non-Human Primates and Human Administration Protocol

The data generated in the mouse system from Example 2H is used to determine the protocol of administration of vector in macaques or chimpanzees chronically infected with hepatitis B virus. Based on the induction of HBV-specific CTL in mice, the subjects in monkeys or chimpanzee trials receive three doses of vector encoding core antigen and B7-1 at 28 day intervals given in two successively escalating dosage groups. Control subjects will receive a placebo comprised of HBV-IT (V) formulation media (consisting of lactose (40 mg/ml), human serum albumin (1 mg/ml) and Tris (1 mM) pH 7.2 - 7.5) The combined dosage is either 10⁵, 10⁶, 10⁷, 10⁸, or 10⁹ HBV-IT (V) plus B7-1 (V) cfu given in four 0.5 ml injections I.M. on each injection day. Alternatively, injections may be S.C., I.D., I.P., or any other conventional vaccination route. Blood samples will be drawn on days 4, 12, 24, 36, 52, 70 and 84 and months 6, 12, 18, 24, 30, and 36 in order to measure serum alanine aminotransferase (ALT) levels, the presence of HBV e antigen, the presence of antibodies directed against the HBV e antigen, and to assess safety and tolerability of the treatment. The HBV e antigen and antibodies to HBV e antigen are detected by Abbott HB e rDNA EIA kit™ as described in Example 2F. Efficacy of the induction of CTL against HBV core antigen can be determined as in Example 2Hiv. Based on the safety and efficacy results from the monkey and chimpanzee studies, the dosage and inoculation schedule will be determined for administration of the vector to subjects in human trials. These will be the same or 10 to 100 fold higher than for the non-human primate dosing. These subjects are monitored for serum ALT levels, presence of HBV e antigen, and the presence of antibodies directed against the HBV e antigen, essentially as described above. Induction of human CTL against HBV core antigen is determined as in Example 2Hiv. Primate CTL responses are measured in an analogous fashion, described in Example 2Hiii.

### H. Cytotoxicity Assays

### i. Inbred Mice

Six- to eight-week-old female Balb/C, C57B1/6 and C3H mice are injected as in 2Gi. Animals are sacrificed after administration of vector or transduced syngeneic cells. Splenocytes (3 x 10⁶/ml) are harvested and cultured *in vitro* with their respective irradiated transduced cells (6 x 10⁴/ml) in T-25 flasks. Culture medium consists of RPMI 1640, 5% heat-inactivated fetal bovine serum, 1 mM sodium pyruvate, 50 µg/ml gentamycin and 10⁻⁵ M β-2-mercaptoethanol. Effector cells are harvested 4-7 days later and tested using various effector:target cell ratios in 96 well microtiter plates in a standard chromium release assay. Targets are the HBV core transduced L⁻M(TK⁻) cells whereas the non-transduced L⁻M(TK⁻) cell lines are used as a control for background lysis. Specifically, Na₂⁵¹CrO₄-labeled (Amersham, Arlington Heights, IL)(100 µCi, 1 hr at 37°C) target cells (1 x 10⁴ cells/well) are mixed with effector cells at various effector to target cell ratios in a final volume of 200 µl. Following incubation, 100 µl of culture medium is removed and analyzed in a Beckman gamma spectrometer (Beckman, Dallas, TX). Spontaneous release (SR) is determined as CPM from targets plus medium and maximum release (MR) is determined as CPM from targets plus 1M HCl. Percent target cell lysis is calculated as: [(Effector cell + target CPM) - (SR)/(MR) - (SR)] x 100. Spontaneous release values of targets are typically 10%-20% of the MR.

For certain CTL assays, the effectors may be *in vitro* stimulated multiple times, such as, for example, on day 8-12 after the primary *in vitro* stimulation. More specifically, 10⁷ effector cells are mixed with 6.0 x 10⁵ irradiated (10,000 rads) stimulator cells, and 2.0 x 10⁷ irradiated (3,000 rads) "filler" cells (prepared as described below) in 10 ml of "complete" RPMI medium. (RPMI containing: 5% heat inactivated FBS. 2 mM L-glutamine, 1 mM sodium pyruvate, 1X nonessential amino acids, and 5.0 x 10⁵ M β-2-mercaptoethanol). "Filler" cells are prepared from naive syngeneic mouse spleen cells resuspended in RPMI, irradiated with 3,000 rads at room temperature. Splenocytes are washed with RPMI, centrifuged at 3,000 rpm for 5 minutes at room temperature, and the pellet is resuspended in RPMI. The resuspended cells are treated with 1.0 ml Tris-ammonium chloride (100 ml of 0.17 M Tris base, pH 7.65, plus 900 ml of 0.155 M NH₄Cl; final solution is adjusted to a pH of 7.2) at 37°C for 3-5 minutes. The secondary *in vitro* restimulation is then cultured for 5-7 days before testing in a CTL assay. Any subsequent restimulations are cultured as described above with the addition of 2-10 U of recombinant human IL-2 (200 U/ml, catalog #799068, Boehringer Mannheim, W. Germany).

Using these procedures, it can be shown that CTL to HBV core antigen or B7-1 administered individually, or HBV and B7-1 administered together are induced. When compared, the vectors administered together yield a greater response than when injected individually or induce equivalent responses using lower doses of the combination vectors than the HBV core antigen vector alone. In certain cases, it may be necessary to add unlabeled non-transduced or β-*gal*/*neo*-transduced targets to labeled targets at a predetermined ratio. This reduces the background lysis of negative control cells.

The β-*gal*/*neo*-transduced targets are generated as follows. The plasmid pSP65 (Promega, Madison, WI) containing the bacterial β-*gal* gene is obtained, and the 3.1 Kb β-*gal* gene isolated as a Xba I-Sma I fragment and inserted into pC15CAT (Anya *et al*., *Science 229*:69-73, 1985) digested with Xba I-Sma I. The *β-gal* gene is excised as a 3.1 Kb Sal I-Sma I fragment and inserted into the KT-1 retroviral vector backbone at the Xho I and the blunted Cla I-site. The Cla I - Cla I fragment containing the SV₂*neo* cassette from PAFVXM is inserted with the same transcription orientation as the vector into the Cla I site by the resultant plasmid. This plasmid is designated CB-β-*gal*.

Infectious retroviral particles are produced through the generation of a stable producer cell line by transfection of CB-β-*gal* plasmid as described in Example 2Bvb The stable producer cell line utilized in these studies is derived from the DA cell line, and is designated DA-β-*gal*. DA-β-*gal* is then used to generate retroviral vector expressing β-*gal*/*neo*. The C3H (H-2k) cell line L-M(TK⁻)is transduced with *β-gal*/*neo* vector as described in Example 2D Clones are screened for *β-gal* expression and the highest expressing clone is chosen for use as a negative *"neo"* control in CTL assays.

### ii. HLA A2.1, HLA A2.1/K^{b} Transgenic Mice, HLA A2.1/Human CD8⁺, or HLA A2.1/k^{b}/Human CD8⁺ Doubly Transgenic Mice

Individual vectors or a combination are injected as in Example 2Gi. Animals are sacrificed and the splenocytes (3 x 10⁶/ml) cultured *in vitro* with irradiated (10,000 rads) transduced Jurkat A2/K^{b} cells or with peptide coated (Example 2K) Jurkat A2/K^{b} cells (6 x 10⁴/ml) in flasks (T-25). The remainder of the chromium release assay is performed as described in Example 2H, where the targets are transduced and non-transduced EL4 A2/K^{b} and Jurkat A2/K^{b} cells. Non-transduced cell lines are utilized as negative controls. The targets may also be peptide coated EL4 A2/K^{b} cells as described in Example 2K.

### iii. Macaque

Blood samples are collected in heparinized tubes 14 days after each injection. The peripheral blood mononuclear cells (PBMC) are then separated from blood using a Ficoll-hypaque (Sigma, St. Louis, MO) gradient at 2,000 rpm for 30 minutes at room temperature. The PBMC are stimulated *in vitro* at a stimulator:effector ratio of 10:1 for 7-10 days with autologous *H. papiovirus* LCL (ATCC# No. CRL 1855) transformed recombinant retroviral transduced cells. Culture medium consists of RPMI 1640 with 5% heat-inactivated FBS, 1 mM sodium pyruvate, 10 mM HEPES, 2 mM L-glutamine, and 50 µg/ml gentamycin. The resulting stimulated CTL effectors are tested for CTL activity against transduced and non-transduced autologous LCL in the standard chromium release assay.

### iv. Chimpanzee and Human

Human or chimpanzee PBMC are separated by Ficoll-hypaque gradient centrifugation. Specifically, cells are centrifuged at 3,000 rpm at room temperature for 5 minutes. The PBMC are restimulated *in vitro* with their autologous transduced LCL, at a stimulator:effector ratio of 10:1 for 10 days. Culture medium consists of RPMI 1640 with prescreened lots of 5% heat-inactivated FBS, 1 mM sodium pyruvate and 50 µg/ml gentamycin. The resulting stimulated CTL effectors are tested for CTL activity using transduced autologous LCL or HLA matched cells as targets in the standard chromium release assay, as described above. Since most patients have immunity to EBV, the non-transduced EBV-transformed B-cells (LCL) used as negative controls, will also be recognized as targets by EBV-specific CTL along with the transduced LCL. In order to reduce the high background due to killing of labeled target cells by EBV-specific CTL, it is necessary to add unlabeled non-transduced LCL to labeled target cells at a ratio of 50:1. Using these procedures, it is shown that the combination of KT-HBc vector and B7.1 vector gives better responses then the KT-HBc vector alone at equivalent doses, or that equivalent responses are seen using lower doses in the combination compared to the KT-HBc vector alone.

### I. Detection of Humoral Immune Response

Humoral immune responses in mice specific for HBV core antigens are detected by ELISA. The ELISA protocol utilizes 100 µg/well of recombinant HBV core and antigen (Biogen, Geneva, Switzerland) to coat 96-well plates. Sera from mice immunized with cells, or direct vector expressing HBV core or antigen separately or in combination with KT-B7-1 vector are then serially diluted in the antigen-coated wells and incubated for 1 to 2 hours at room temperature. After incubation, a mixture of rabbit anti-mouse IgG1, IgG2a, IgG2b, and IgG3 with equivalent titers is added to the wells. Horseradish peroxidase ("HRP")-conjugated goat anti-rabbit anti-serum (Boehringer Mannheim, Indianapolis, IN) is added to each well and the samples are incubated for 1 to 2 hours at room temperature. After incubation, reactivity is visualized by adding the appropriate substrate. Color will develop in wells that contain IgG antibodies specific for HBV core antigen.

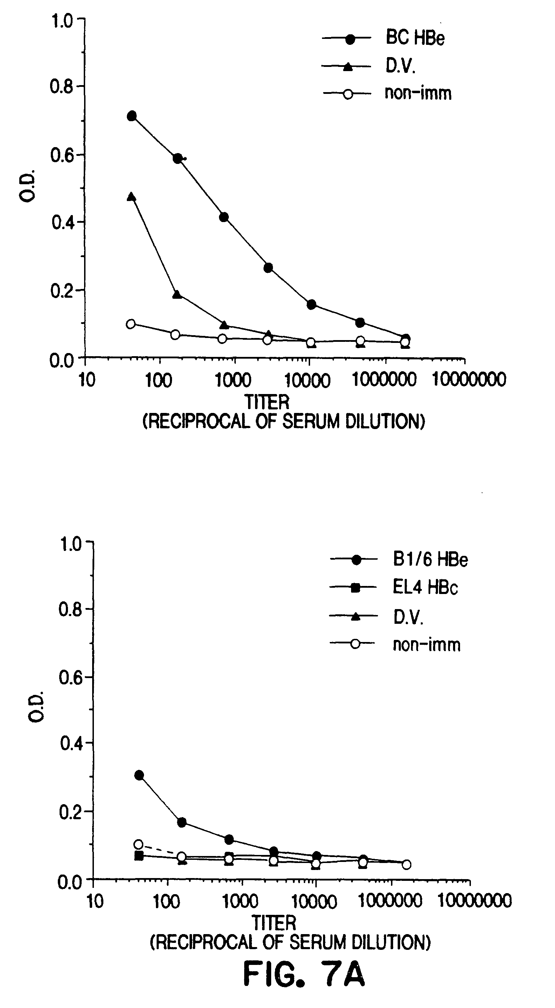

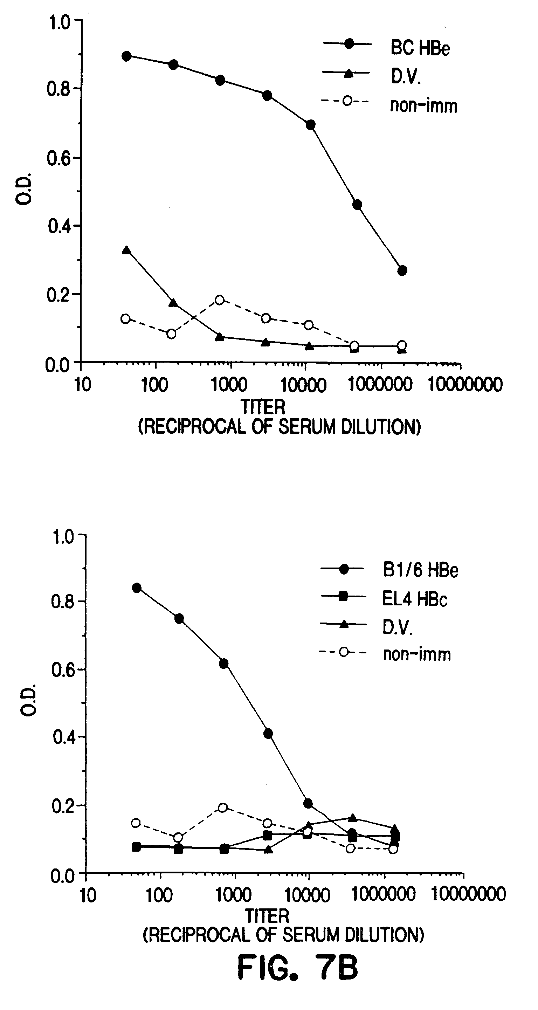

Using these procedures, it can be shown that IgG antibody to HBV core antigens can be induced in mice, Figures 7A and 7B.

### J. T-Cell Proliferation

Antigen induced T-helper activity resulting from two or three injections of direct vector preparations expressing HBV core antigen, is measured *in vitro.* Specifically, splenocytes from immunized mice are restimulated *in vitro* at a predetermined ratio with cells expressing HBV core or e antigen or with cells not expressing HBV core or e antigen as a negative control. After five days at 37°C and 5% CO₂ in RPMI 1640 culture medium containing 5% FBS, 1.0 mM sodium pyruvate and 10⁻⁵ β-2-mercaptoethanol, the supernatant is tested for IL-2 activity. IL-2 is secreted specifically by T-helper cells stimulated by HBV core or e antigen, and its activity is measured using the CTL clone, CTLL-2 (ATCC No. TIB 214). Briefly, the CTLL-2 clone is dependent on IL-2 for growth and will not proliferate in the absence of IL-2. CTLL-2 cells are added to serial dilutions of supernatant test samples in a 96-well plate and incubated at 37°C and 5%, CO₂ for 3 days. Subsequently, 0.5 µCi ³H-thymidine is added to the CTLL-2 ³H-thymidine is incorporated only if the CTLL-2 cells proliferate. After an overnight incubation, cells are harvested using a PHD cell harvester (Cambridge Technology Inc., Watertown, MA) and counted in a Beckman beta counter. The amount of IL-2 in a sample is determined from a standard curve generated from a recombinant IL-2 standard obtained from Boehringer Mannheim (Indianapolis, IN).

### K. Identification of Immunogenic Domains of HBV Precore/core

Cytotoxic T lymphocyte epitopes may be predicted utilizing the HLA A2.1 motif described by Falk *et al*., (*Nature 351*:290, 1991). From this analysis, peptides are synthesized and used to identify CTL epitopes. These peptides are tested on individuals with acute hepatitis B infection or on HLA A2.1 or HLA A2.1/K^{b} transgenic mice. Effector cells from individuals with acute hepatitis B infection are stimulated in vitro with transduced autologous (Example 2E) LCL and tested on autologous LCL coated with the peptide. The chromium release assay is performed as described in Example 2Hiv, except that peptide is added at a final concentration of 1-100 µg/ml to non-transduced Na₂⁵¹CrO₄-labeled LCL along with effector cells. The reaction is incubated 4-6 hours and a standard chromium release assay performed as described above.

Effector cells from HLA A2.1 or HLA A2.1/K^{b} transgenic mice are harvested and CTL assays performed as described above. The peptide is added at a final concentration of 1-10 µg/ml to non-transduced Na₂⁵¹CrO₄-labeled ELA A2/K^{b} cells. These peptide coated cells are utilized as targets in a CTL assay.

### Example 3

### Administration of HIV Antigen Expression Vector and HBV Antigen Sindbis Expression Vector

### A. Construction of HTV env Expression Vector

A 2.7 Kb Kpn I -Xho I DNA fragment was isolated from the HIV proviral clone BH10-R3 (for sequence, see Ratner *et al*., *Nature 313*:277, 1985) and a 400 bp Sal I/Kpn I DNA fragment from IIIexE7delta*env* (a Bal31 deletion to nucleotide 5,496) was ligated into the Sal I site in the plasmid SK⁺. From this clone, a 3.1 Kb *env* DNA fragment (Xho I-Cla I) which also encodes *rev,* essential for *env* expression, was purified and ligated into a retroviral vector called pAFVXM. This vector was modified in that the Bgl II site was changed by linker insertion to a Xho I site to facilitate cloning of the HIV *env* coding DNA fragment.

The expression vector is constructed by a three part ligation in which the Xho I-Cla I fragment containing the gene of interest and the 1.0 Kb MoMLV 3' LTR Cla I-Hind III fragment are inserted into the Xho I-Hind III site of pUC31/N2R5gM plasmid. The Cla I-Cla I *neo* gene fragment from the pAFVXM retroviral vector is then inserted into the Cla I site of this plasmid in the sense orientation.

This dominant selectable marker gene comprises a SV40 early promoter driving expression of neomycin phosphotransferase gene (the Cla I fragment from the plasmid pAFVXM). This gives the retroviral vector plasmid KT-1 (as opposed to the KT-1 backbone).

### B. Construction of Sindbis Vectors Expressing HBVe-c and HBV core

The plasmid SK⁺HBe-c is constructed as described above in Example 2Bii. Construction of a Sindbis vector expressing the HBVe-c sequence is accomplished by digesting the plasmid SK⁺HB e-c with Xho I and Cla I restriction enzyme sites to release the cDNA fragment encoding HBe-c sequences. The fragment is then agarose gel purified by electrophoresis, Geneclean II™ , and inserted onto the desired Sindbis vector backbone, prepared by digestion with Xho I and Cla I, and treated with calf intestine alkaline phophatase (CIAP). Several possible Sindbis vectors and whose detailed construction is described in (USSN 08/122,791) are suitable for the insertion of the HBV antigen sequences. Such Sindbis vectors include pSKSINBV, pSKSINd1JRsjrc, pSKSINd1JRsjrPC, pSKSINd1JRsjrNP(7582-7601) and pSKSINd1JRsexjr or related derivatives.

Construction of a Sindbis vector expressing the HBV core sequence is accomplished by Geneclean II™ treatment of the PCR product described above detailing the PCR amplification steps. The amplified product is then digested with Xho I and Cla I restriction enzyme sites, agarose gel purified, Geneclean II™ treated and then ligated into the same Sindbis vectors described above pre-treated with Xho I and Cla I enzymes.

### C. Generation of a Producer Cell Line which Expresses HBV Specific Antigens

To produce a vector producing cell line that expresses the HBV core antigen derived from the vector described above, transfected *in vitro* transcribed RNA transcripts in the cytoplasm are encapsidated by Sindbis viral structural proteins supplied in *trans* in a Sindbis packaging cell line (Example 3D). Specifically the Sindbis RNA vector molecules are initially produced by using a T7 *in vitro* transcribed RNA polymerase system used to transcribe from a cDNA Sindbis vector clone encoding the HIV specific sequences. The vector, *in vitro* generated RNA products, are then transfected into a Sindbis packaging or hopping cell line leading to the production of transient infectious vector particles within 24 hours. These vector particles are then collected in the supernatants of the cell line cultures and then filtered through a 0.45 µ filter to avoid cellular contamination. The filtered supernatants are then used to transduce a fresh monolayer of Sindbis packaging cells. With in 24 hours of transduction, Sindbis vector particles are being produced containing positive stranded Sindbis recombinant RNA encoding Sindbis non-structural proteins and HIV specific sequences.

An alternative configuration of a Sindbis HBV core antigen vector can be developed using a promoter driven cDNA Sindbis vector containing a selectable marker (Example 3D). In this configuration the above described Xho I to Cla I fragment containing the specific HBV core antigen sequence is placed in a similar cDNA Sindbis vector driven by a constitutive promoter in place of a bacteriophage polymerase recognition sequence. Using this configuration, the expression vector plasmids are transfected into the packaging cell line and selected for the drug resistance gene 24 to 48 hours post transfection. Resistant pools are then pooled 14 days later (dependent on the selection marker used) and then dilutioned cloned. From the dilution clone, several clones are then propagated, and assayed for highest vector titer. The highest titer clones are then expanded and frozen and tested for HIV specific protein production and immune response induction.

### D. Alternative Viral Vector Packaging Techniques

Sindbis packaging cell lines are constructed as described U.S.S.N. 08/198,450. Various alternative systems can be used to produce recombinant Sindbis virus carrying the vector construct. Each of these systems takes advantage of the fact that the baculovirus, and the mammalian viruses, vaccinia and adenovirus, have been adapted recently to make large amounts of any given protein for which the gene has been cloned. For example, Smith *et al*. (*Mol*. *Cell. Biol. 3*:12, 1983); Piccini *et al*. (*Meth*. *Enzymology 153*:545, 1987); and Mansour *et al*. (*Proc*. *Natl*. *Acad*. *Sci*. *USA 82*:1359, 1985).

These viral vectors can be used to produce proteins in tissue culture cells by insertion of appropriate genes into the viral vector and, hence, could be adapted to make Sindbis vector particles.

Adenovirus vectors are derived from nuclear replicating viruses and can be defective. Genes can be inserted into vectors and used to express proteins in mammalian cells either by *in vitro* construction (Ballay *et al., EMBO J*. *4*:3861, 1985) or by recombination in cells (Thummel *et al., J. Mol. Appl. Genetics 1*:435, 1982).

One preferred method is to construct plasmids using the adenovirus major late promoter (MLP) driving: (1) Sindbis non-structural proteins, and (2) a modified Sindbis vector construct. A modified Sindbis vector in this configuration would still contain a modified junction region, which would enable the RNA vector transcribed, to be self replicating as it would in a natural setting.

These plasmids can then be used to make adenovirus genomes in vitro (Ballay *et al*., *supra*), and these transfected in 293 cells (a human cell line making adenovirus E1A protein, ATCC No. CRL 1573), for which the adenoviral vectors are defective, to yield pure stocks of Sindbis structural proteins and Sindbis vector carried separately in defective adenovirus vectors. Since the titres of such vectors are typically 10⁷-10¹¹/ml, these stocks can be used to infect tissue culture cells simultaneously at high multiplicity. The cells will then be programmed to produce Sindbis proteins and Sindbis vector genomes at high levels. Since the adenovirus vectors are defective, no large amounts of direct cell lysis will occur and Sindbis vectors can be harvested from the cell supernatants.

Other viral vectors such as those derived from vectors unrelated to Sindbis (e.g., RSV, MMTV or HIV) may also be used in the same manner to generate vectors from primary cells. In one embodiment, these adenoviral vectors are used in conjunction with primary cells, giving rise to Sindbis vector preparations.

Recently an alternative expression system has also been described in which chimeric HIV/poliovirus genomes, result in the generation of chimeric minireplicons (*J*. *Virol. 65*:2875, 1991), capable of expressing fusion proteins. These chimeric polio virus minireplicons were later demonstrated to be encapsidated and produce infectious particles by using a recombinant vaccinia virus (VV-P1) expressing the substituted polio virus capsid precursor P1 protein which is defective in the chimeric minireplicon (*J*. *Virol*. *67*:3712. 1993). In the study, HIV-1 *gag-pol* sequences were substituted for the VP2 and VP3 capsid genes of the P1 capsid of poliovirus. In a similar fashion, the Sindbis vector genome can be substituted for the P1 capsid sequences and used in this system as a means for providing polio pseudotyped Sindbis vectors after transfecting *in vitro* transcribed Sindbis RNA transcripts into the cell line. Conversely, Sindbis structural proteins can also be substituted for the VP2 and VP3 sequences, subsequently providing an alternative packaging cell line system for Sindbis based vectors.

In an alternative system (which is more truly extracellular), the following components are used:
1. Sindbis structural proteins made in the baculovirus system in a similar manner as described in Smith *et al*. (*supra*) (or in other protein production systems, such as yeast or *E*. *coli*);
2. viral vector RNA made in the known T7 or SP6 or other in vitro RNA-generating system (*see*, for example, Flamant and Sorge, *J. Virol. 62*:1827, 1988);
3. tRNA made as in (2) or purified from yeast or mammalian tissue culture cells;
4. liposomes (with embedded *env* protein); and
5. cell extract or purified necessary components (when identified) (typically from mouse cells) to provide RNA processing, and any or other necessary cell-derived functions.

Within this procedure (1), (2) and (3) are mixed, and then *env* associated Sindbis proteins, cell extract and pre-liposome mix (lipid in a suitable solvent) added. It may, however, be necessary to earlier embed the Sindbis *env* proteins in the liposomes prior to adding the resulting liposome-embedded *env* to the mixture of (1), (2), and (3). The mix is treated (*e.g.,* by sonication, temperature manipulation, or rotary dialysis) to allow encapsidation of the nascent viral particles with lipid plus embedded Sindbis *env* protein in a manner similar to that for liposome encapsidation of pharmaceuticals, as described in Gould-Fogerite *et al.,* (*Anal. Biochem. 148*:15, 1985). This procedure allows the production of high titres of replication incompetent Sindbis virus vectors with out the requirement of establishing intermediate packaging cell lines.

### E. Expression of Infected Cells with Sindbis Vectors

ELISA and immunoprecipitation/Western blot to determine expression of HBV core antigen are performed as described above in Example 2F.

### F. Direct Vector Administration in Mice

The mouse system may also be used to evaluate the induction of humoral and cell-mediated immune responses with direct administration of vector encoding HBV core or HIV antigen Briefly, six- to eight-week-old female Balb/C, C57B16 or C3H/He mice are injected I.M., intradermally (I.D.), or subcutaneously (S.C.) with 0.1 ml of unpurified liquid or reconstituted (with sterile deionized, distilled water) lyophilized HBV core Sindbis vector and/or HIV antigen expressing retroviral vector. The titers of vector preparation between 10⁵ and 10⁹ cfu/ml were injected individually or mixed and administered together. Two injections are given one week apart. Seven days after the second injection, the animals are sacrificed. ⁵¹Chromium release CTL assays are then performed essentially as described in Example 2Hi.

### G. Cytotoxicity Assays

Cytotoxicity assays to determine the presence of CTL directed against heterologous proteins expressed by vector induced cells are performed as described in Example 2H above.

### H. Detection of Humoral Immune Response

Humoral immune responses in mice specific for HBV core antigens are detected by ELISA as described in Example 2I above.

### I. T-Cell Proliferation

Antigen induced T-helper activity resulting from two or three injections of direct vector preparations expressing HBV core antigen is measured *in vitro* by T cell proliferation assay as described above in Example 2J.

### J. Human and Non-Human Primate Administration Protocol

Vector construct administration protocol for human and non-human primates (*e*.*g*., monkey and or chimpanzee) is performed as described in Example 2G.

### Example 4

### Administration of HBV Antigen Retroviral Vector or DNA Vector with HIV Antigen Retroviral Expression Vector

### A. Induction of CTL and Humoral Antibody Responses with Retroviral Vectors Expressing HBV Core antigen:

### 1. Murine CTL Response:

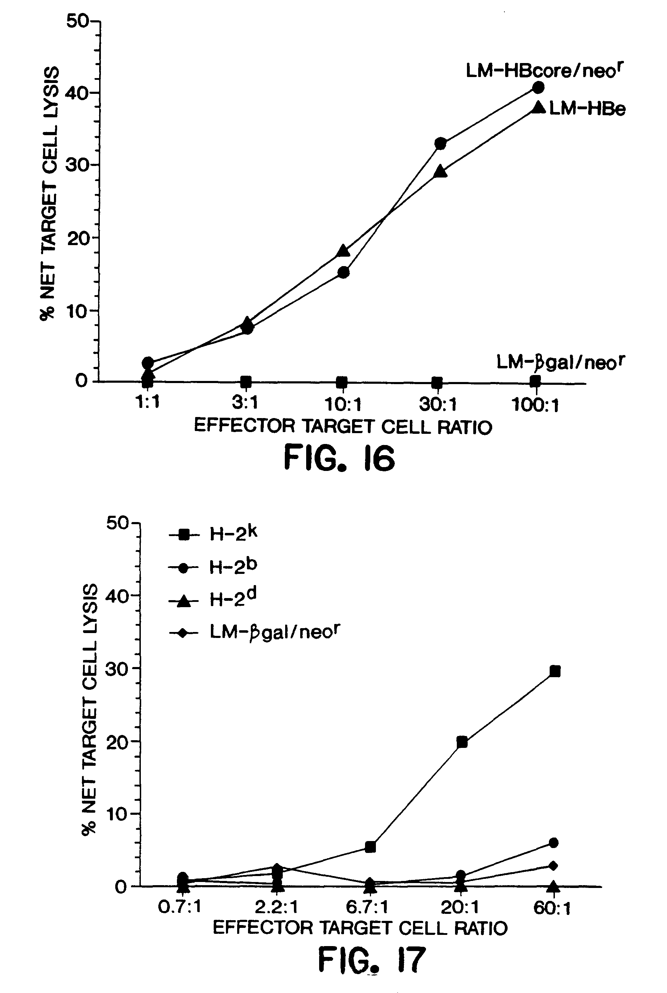

Effector cells obtained from C3H/He CR mice (H2^{k}) primed by i.m. administration of HBV core formulated vector were tested for their cytolytic activity using HB cAg retrovector-transduced LMTK-cells (H-2^{k}), HBcAg retrovector-transduced BL/6 cells (H-2^{b}), or HBcAg retrovector-transduced BC10ME cells (H-2^{d}) as targets in a chromium release assay. CTL responses against HBV core and HBV e antigen were induced in C3H/He CR mice (see Figure 16). The results in Figure 17 show that the effectors induced by immunization with HBcAg retrovector are H-2^{k} MCH class I restricted because the effectors kill targets that present HBcAg in the context of H-2^{k} but do not kill targets that present HBcAg in the context of H-2^{b} or H-2^{d}.

In order to determine whether the CTL response was mediated by CD4+ cells or CD8+ cells, the stimulated effector cells were depleted of either CD4 cells or CD8 cells by treatment with either anti-CD4 or anti-CD8 antibodies conjugated to magnetic beads. Stimulated effector cells depleted of CD4 cells were isolated by immunomagnetic separation using Dynabeads (Dynal Inc., Skoyen, NO) as follows: a) restimulated spenocytes were incubated 30 minutes at 4°C with monoclonal rat anti-L3T4 (Collaborative Biomedical Products, Becton Dickinson Labware, Bedford, ME), b) cells were washed twice with DMEM containing 10% FCS and resuspended to 1x10⁷ cells/ml in medium, c) approximately 75µl/1x10⁷ cells/ml of prewashed Dynabeads coated with sheep anti-rat IgG (Dynal Inc., cat #M-450) were added to the cells, and CD4⁺ cells were recovered magnetically. The remaining CD4-depleted cells
were then tested for their cytolytic activity using LM core/neo^{r} and B-gal/neo^{r} as targets in a chromium release assay.

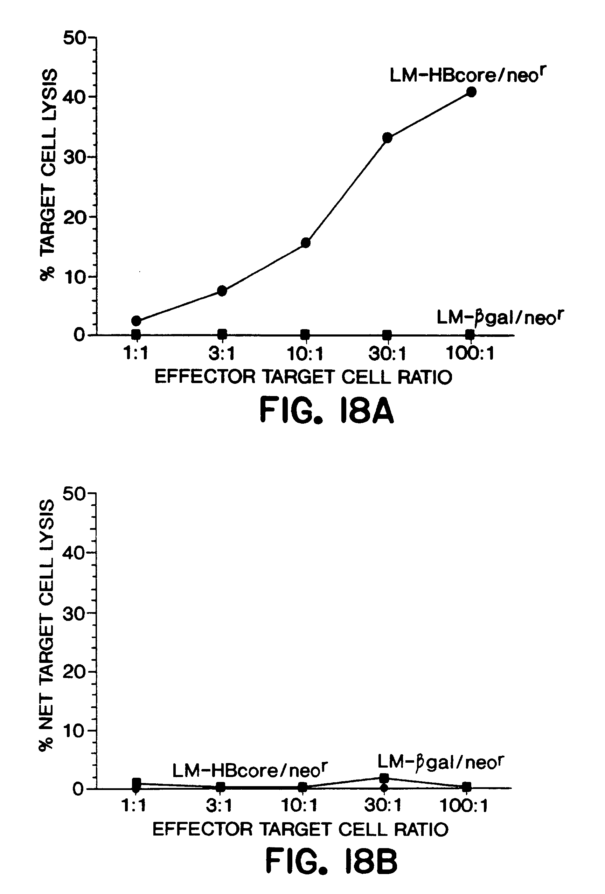

Stimulated effector cells depleted of CD8 cells are isolated by immunomagnetic separation using Dynabeads (Dynal Inc., Skoyen, NO) as follows: a) restimulated spenocytes are incubated 30 minutes at 4°C with monoclonal rat anti-Lyt-2 (Collaborative Biomedical Products, Becton Dickinson Labware, Bedford, ME), b) cells are washed twice with DMEM containing 10% FCS and resuspended to 1x10⁷ cells/ml in medium, c) approximately 75µl/1x10⁷ cells/ml of prewashed Dynabeads coated with sheep anti-rat IgG (Dynal Inc., cat #M-450) are added to the cells, and CD4⁺ cells are recovered magnetically. The remaining CD8-depleted cells are then tested for their cytolytic activity using i.m. core/neo^{r} and B-gal/neo^{r} as targets in a chromium release assay. The results showed that the CTL effectors are CD8+, CD4-. (See figure 18.)

### 2. Murine Humoral Immune Response:

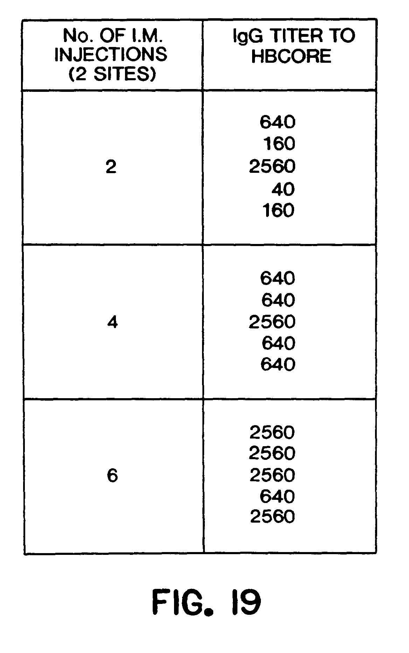

Humoral immune responses in mice specific for HBV core and e antigens were detected by ELISA. The ELISA protocol utilizes 100 µg/well of recombinant HBV core antigen (Biogen, Geneva, Switzerland) to coat 96-well plates. Sera from mice immunized with the HB Fcore/neoR vector expressing HBV core were serially diluted in the antigen-coated wells and incubated for 1 to 2 hours at room temperature. After incubation, a mixture of rabbit anti-mouse IgG1, IgG2a, IgG2b, and IgG3 with equivalent titers was added to the wells. Horseradish peroxidase ("HRP")-conjugated goat anti-rabbit anti-serum is added to each well and the samples were incubated for 1 to 2 hours at room temperature. After incubation, reactivity was visualized by adding the appropriate substrate. Color developed in wells that contain IgG antibodies specific for HBV core antigen. IgG antibody to HBV core antigen was induced in mice as shown in Figure 19. (The antibody titer is expressed as the reciprocal for the dilution required to yield 3 times the CD reading of pre-immunication sera.)

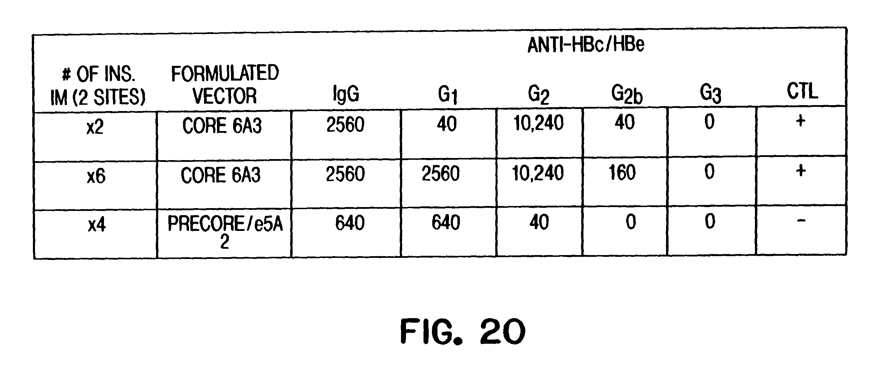

The isotype(s) of the humoral response in mice immunized with formulated HBV retrovectors were detected by an ELISA assay described above, with the following modification: sera from mice were serially diluted into the wells of a 96 well titer plate in which the wells have been coated with either recombinant core or e protein as described above. The specific isotype was determined by incubation with one of the following rabbit anti-mouse antisera: IgG1, IgG2a, IgG2b, or IgG3. The assay was developed as described above. Using this procedure, it was shown that both IgG2a and IgG1 antibodies were induced in C3H/He (CR) mice immunized with formulated HBV core vector (6A3) and with formulated HBVe vector (5A2) (see Figure 20).

### 3. Induction of a CTL response in transgenic HBeAg mice:

Transgenic HBe mice were successively backcrossed to C3H/He mice for three generations. Retroviral vectors encoding HBcAg were injected i.m. in the right and left gastronemus muscle of three mice. The mice were boosted on Day 7 and spleens were harvested on Day 14 for testing in the CTL assay. Results of the CTL assay showed that all three mice had CTL responses greater than 15% over the negative control an an effector:target ratio of 100:1.

### 4. CTL Immune Response in Macaques:

Male and female macaques of variable age (Primate Research Institute, White Sands, New Mexico) were injected intramuscularly (4 sites), or intradermally in the nape of the neck with 0.5 ml of formulated HB Fcore/neo^{R} vector, or HBV e retroviral vector. Four injections were given at 14 day intervals. Fourteen days after each injection, blood samples were collected for chromium release CTL assays.

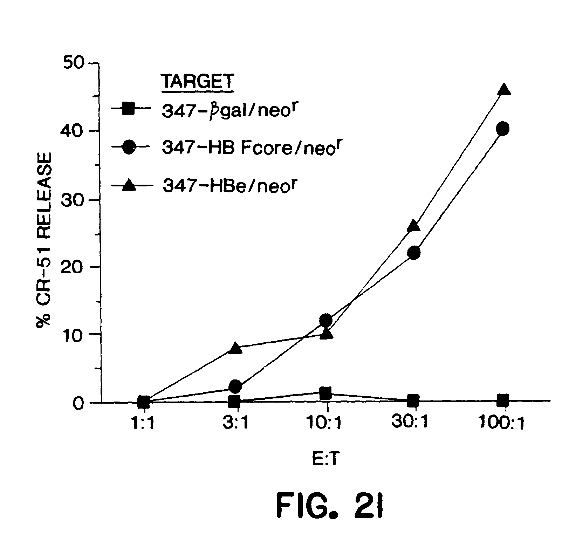

Blood samples were collected in heparinized tubes 14 days after each injection. The peripheral blood mononuclear cells (PBMCs) were then spun through a Ficoll-hypaque column at 2000 rpm for 30 minutes at room temperature. The PBMCs were stimulated *in vitro* with their autologous transduced LCL at a stimulator:effector ratio of 10:1 for 7-10 days. Culture medium was RPMI 1640 with 5% heat-inactivated fetal bovine serum (Hyclone, Logan, Utah), 1 mM sodium pyruvate, 10 mM HEPES, 2 mM L-glutamine, and 50 ugm/ml gentamycin. The resulting stimulated CTL effectors were tested for CTL activity against transduced and non-transduced autologous LCL in the standard chromium release assay. There was a positive CTL response to both the HBV core and e antigens. (See figure 21.)

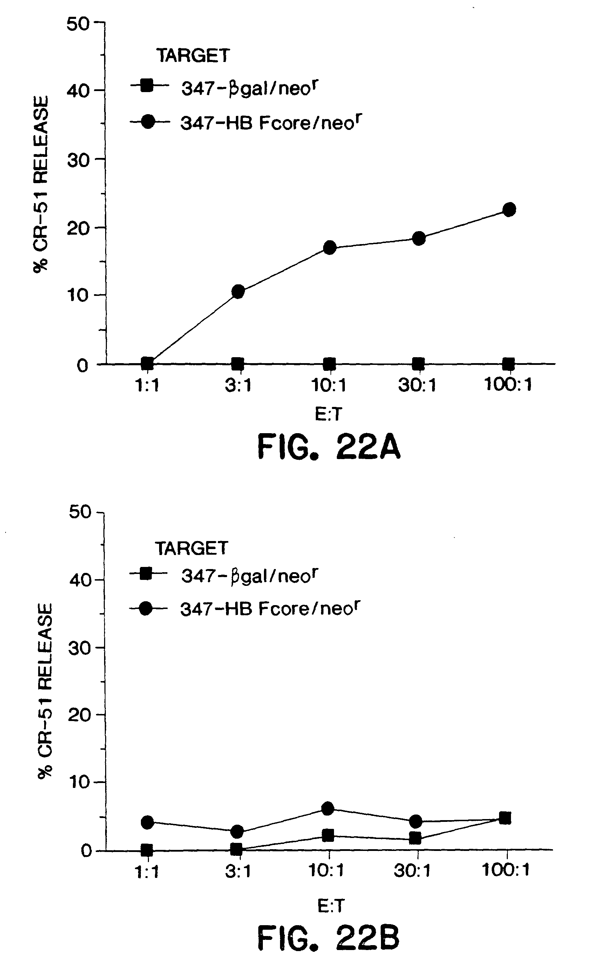

Stimulated macaque effector cells depleted of CD4 or CD8 cells were prepared using Dynabeads as described above for the murine effector cells. The CD8-depleted and the CD4-depleted cells were then tested for cytolytic activity using i.m. core/neoR and B-gal/neoR as targets in a chromium release assay. (See Figure 22).

### B. Induction of CTL Response with DNA Vectors expressing HBV core antigen:

### 1. Vector construction:

The eukaryotic expression vector pcDNA3 was obtained from Invitrogen (San Diego, California, USA). This vector includes the human cytomegolovirus (CMV) major inmediate early promoter/enhancer region and the expression of all inserted genes was driven using this promoter. Other relevant features of this vector include a bovine growth hormone (BGH) polyA+ signal and a neomycin resistance gene.

The Xho I to Cla I HBV core fragment from KT-HBc was ligated into the Xho I and Cla I sites of SK II+ (Stratagene, La Jolla, California, USA). This plasmid was designated SK II+ HBV core. From this plasmid, the Xho I-Xba fragment encoding HBV core antigen was excised and inserted into the Xho I-Xba I sites of the pcDNA3 vector. This plasmid was designated pcDNA3 HBV core.

### 2. CTL Induction:

Female C3H/HeN mice (Charles River Laboratories, Massachusetts, USA) were injected i.m. in the right and left Tibialis anterior muscle with 60-100 ug of plasmid DNA dissolved in 50 ul of PBS using a 3/10cc U-100 insulin syringe with a preattached 29G1/2 needle. All plasmid DNA was double cesium chloride purified using standard techniques. Mice were anesthetized with Avertin prior to pretreatment with 100 ul of 10uM cardiotoxin (Latoxin, Rosans, France) in PBS, 5 days prior to the injection of the plasmid DNA. Mice were injected on day 0 and boosted 4 weeks later, and their spleens were collected for testing in a CTL assay.

CTL assays were performed as described in Example 2, herein. Results of the CTL assays showed two of the three mice had CTL responses greater than 15% over the negative control at an effector: target ratio of 100: 1.

### C. Cytotoxicity Assays

### i. Cytotoxicity Assays to Determine the Presence of CTL

CTL directed against heterologous proteins expressed by vector induced cells of inbred mice are performed as described in Example 2Hi above.

### ii. HLA A2.1 Transgenic Mice

Six- to eight-week-old female HLA A2.1 transgenic mice are injected twice I.M., I.D., or S.C. at 1 week intervals with 10⁵ to 10⁷ cfu of HIV III*env* in combination with 10 to 100 ug of CMV KT-HBc expression vector DNA with or without additives such as bupivacaine, which enhances gene expression when injected I.M (Danko, I., *et al*., *Gene Therapy 1*:114-121, 1994). Animals are sacrificed 7 days later and the splenocytes (3 x 10⁶/ml) cultured in vitro with irradiated (10,000 rads) transduced Jurkat A2/K^{b} cells or with peptide coated Jurkat A2/K^{b} cells (6 x 10⁴/ml) in flasks. The remainder of the chromium 51 release assay is performed as described in Example 2Hi where the targets are transduced and non-transduced EL4 A2/K^{b} and Jurkat A2/K^{b} cells. Non-transduced cell lines are utilized as negative controls. The targets may also be peptide coated EL4 A2/K^{b} cells.

### D. Human and Non-Human Primate Administration Protocol

The data generated in the mouse system from Example 2Hi is used to determine the protocol of administration of vector in macaques or chimpanzees chronically infected with HBV. Based on the induction of HBV-specific CTL in mice, the subjects in monkeys or chimpanzee trials receive three doses of CMV HBV core antigen expression vector DNA and HIV IIIB*env* RRV at 28 day intervals given in two successively escalating dosage groups. Control subjects will receive a placebo comprised of HBV-IT (V) formulation media. The combined dosage is 10⁶, 10⁷, 10⁸, or 10⁹ HIV IIIB*env* RRV plus 1, 10, 100, 1000 µg of CMV HBV core antigen expression vector DNA given in four 0.5 ml injections I.M. on each injection day. The ratio of the two can be 100:1, 10:1, 1:1, 1:10, or 1:100. Blood samples are drawn on days 4, 12, 24, 36, 52, 70 and 84 and months 6, 12, 18, 24, 30, and 36 in order to measure serum ALT levels, the presence of HBV e antigen, the presence of antibodies directed against the HBV e antigen and to assess safety and tolerability of the treatment. The HBV e antigen and antibodies to HBV e antigen are detected by Abbott HBe rDNA EIA kit™ as described in Example 2Fi Efficacy of the induction of CTL against HBV core or can be determined as in Example 4E.

Based on the safety and efficacy results from the monkey and chimpanzee studies, the dosage and inoculation schedule will be determined for administration of the vector to subjects in human trials. These doses are in the range of 10⁶ to 10⁹ cfu of HIV-IT(V) and 1 to 1000 µg of CMV HBV core antigen expression vector. These subjects are monitored for serum ALT levels, presence of HBV e antigen and the presence of antibodies directed against the HBV e antigen, essentially as described above. Induction of human CTL against HBV core antigen is determined as in Example 4E.

### E. Human CTL Assays

Human CTL assays are performed as described in Example 2Hiv.

### F. Detection of Humoral Immune Response

Humoral immune responses in mice specific for HBV core antigens are detected by ELISA as described in Example 2I above.

### G. T-Cell Proliferation

Antigen induced T-helper activity resulting from two or three injections of direct vector preparations expressing HBV core antigen is measured *in vitro* by T-cell proliferation assay as described above in Example 2J.

### Example 5

### Administration of Recombinant Retroviral Vectors Expressing γ-IFN and HIV Antigen

### A. Cloning of mγ-IFN and Insertion into KT-3B

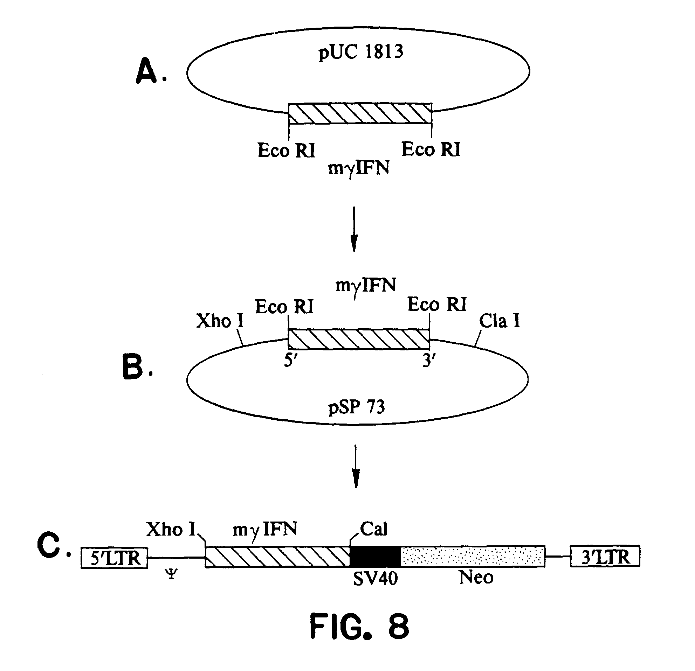

A mγ-IFN cDNA is cloned into the EcoR I site of pUC 1813 essentially as set forth below. Briefly, pUC1813 is prepared as essentially described by Kay *et. al., Nucleic Acids Research 15*:2778, 1987; and Gray *et. al., PNAS 80*:5842, 1983) (Figure 8A). The mγ-IFN cDNA is retrieved by Eco RI digestion and the isolated fragment is cloned into the EcoR I site of phosphatase-treated pSP73 (Promega, Madison, WI.) (Figure 8B). This construct is designated SP mγ-IFN. The orientation of the cDNA is verified by appropriate restriction enzyme digestion and DNA sequencing. In the sense orientation, the 5' end of the cDNA is adjacent to the Xho I site of the pSP73 polylinker and the 3' end adjacent to the Cla I site. The Xho I-Cla I fragment containing the mγ-IFN cDNA in either sense or antisense orientation is retrieved from SP mγ-IFN construct and cloned into the Xho I-Cla I site of the KT-3B retroviral. This construct is designated KT mγ-IFN (Figure 8C).

### B. Cloning of hγ-IFN and Insertion into KT-3B

### i. PHA Stimulation of Jurkat Cells

Jurkat cells (ATCC No. CRL 8163) are resuspended at a concentration of 1 x 10⁶ cells/ml in RPMI growth media with 5% FBS to a final volume of 158 ml. Phytohemoagglutinin (PHA) (Curtis Mathes Scientific, Houston, TX) is added to the suspension to a final concentration of 1%. The suspension is incubated at 37°C in 5% CO₂ overnight. The cells are harvested on the following day and aliquoted into three 50.0 ml centrifuge tubes. The three pellets are combined in 50.0 ml 1x PBS, 145 mM, pH 7.0 and centrifuged at 1,000 rpm for 5 minutes. The supernatant is decanted and the cells are washed with 50.0 ml PBS. The cells are collected for RNA isolation.

### ii. RNA Isolation

The PHA stimulated Jurkat cells are resuspended in 22.0 ml guanidinium solution (4 M guanidinium thiocyanate; 20 mM sodium acetate, pH 5.2; 0.1 M dithiothreitol, 0.5% sarcosyl). This cell-guanidinium suspension is then passed through a 20 gauge needle six times in order to disrupt cell membranes. A CsCl solution (5.7 M CsCI, 0.1 M EDTA) is then overlaid with 11.0 ml of the disrupted cell-guanidinium solution. The solution is centrifuged for 24 hours at 28,000 rpm in a SW28.1 rotor (Beckman, Fullerton, CA) at 20° C. After centrifugation the supernatant is carefully aspirated and the tubes blotted dry. The pellet is resuspended in a guanidinium-HCl solution (7.4 M guanidinium-HCl; 25 mM Tris-HCl, pH 7.5; 5 mM dithiothreitol) to a final volume of 500 µl. This solution is transferred to a microcentrifuge tube. 12.5 µl of concentrated glascial acetic acid and 250 µl of 100% EtOH are added to the microfuge tube. The solution is mixed and stored for several days at -20°C.

After storage, the solution is centrifuged for 20 minutes at 14,000 rpm, 4°C. The pellet is then resuspended in 75% EtOH and centrifuged for 10 minutes at 14,000 rpm, 4°C. The pellet is dried by centrifugation under vacuum, and resuspended in 300 µ| deionized (DI) H₂O. The concentration and purity of the RNA is determined by measuring optical densities at 260 and 280 nm.

### iii. Reverse Transcription Reaction

Immediately before use, 5 µl (3.4 mg/ml) of purified Jurkat RNA is heat treated for 5 minutes at 90°C, and then placed on ice. A solution of 10 µl of 10x PCR buffer (500 mM KCl; 200 mM Tris-HCl, pH 8.4; 25 mM MgCl₂; 1 mg/ml bovine serum albumin (BSA)); 10 µl of 10 mM dATP, 10 µl of 10 mM dGTP, 10 µl of 10 mM dCTP, 10 µl of 10 mM dTTP, 2.5 µl RNasin (40,000 U/ml, Promega, WI) and 33 µl DI H₂O, is added to the heat treated Jurkat cell RNA. To this solution 5 µl (108 nmol/ml) of (Sequence ID No. 1), and 5 µl (200,000 U/ml) MoMLV reverse transcriptase (EC 3.1.27.5, Bethesda Research Laboratories, MD) is mixed in a microfuge tube and incubated at room temperature for 10 minutes. Following the room temperature incubation, the reaction mixture is incubated for 1 hour at 37°C, and then incubated for 5 minutes at 95°C. The reverse transcription reaction mixture is then placed on ice in preparation for PCR.

### iv. PCR Amplification

The PCR reaction mixture contains 100 µl reverse transcription reaction; 356 µl DI H₂O; 40 µl 10x PCR buffer; 1 µl (137 nmol/ml) (Sequence ID No. 2); 0.5 µl (320 nmol/ml) (Sequence ID No. 3), and 2.5 µl, 5,000 U/ml, Taq polymerase (EC 2.7.7.7, Perkin-Elmer Cetus, CA). One hundred microliters of this mixture is aliquoted into each of 5 tubes.

This primer is complementary to a sequence of the mγ-IFN cDNA 30 base pairs downstream of the stop codon.

This primer is complementary to the 5' coding region of the mγ-IFN gene, beginning at the ATG start codon. The 5' end of the primer contains a Xho I restriction site.

This primer is complementary to the 3' coding region of the mγ-IFN gene, ending at the TAA stop codon. The 5' end of the primer contains a Cla I restriction site.

Each tube was overlaid with 100.0 µl mineral oil, and placed into a PCR machine (Ericomp Twin Block System, Ericomp, CA). The PCR program regulates the temperature of the reaction vessel first at 95°C for 1 minute, next at 67°C for 2 minutes and finally at 72°C for 2 minutes. This cycle is repeated 40 times. The last cycle regulates the temperature of the reaction vessel first at 95°C for 1 minute, next at 67°C for 2 minutes and finally at 72°C for 7 minutes. The completed PCR amplification reactions are stored at 4°C for 1 month in preparation for PCR DNA isolation.

### v. Isolation of PCR DNA

The aqueous phase from the PCR amplification reactions are transferred into a single microfuge tube. Fifty microliters of 3 M sodium acetate and 500 µl of chloroform:isoamyl alcohol (24:1) is added to the solution. The solution is vortexed and then centrifuged at 14,000 rpm at room temperature for 5 minutes. The upper aqueous phase is transferred to a fresh microfuge tube and 1.0 ml of 100% EtOH is added. This solution is incubated for 4.5 hours at -20°C and then centrifuged at 14,000 rpm for 20 minutes. The supernatant is decanted, and the pellet is rinsed with 500.0 µl of 70% EtOH. The pellet is dried by centrifugation under a vacuum. The isolated hγ-IFN PCR DNA is resuspended in 10 µl DI H₂O.

### vi. Creation and Isolation of Blunt-Ended hγ-IFN PCR DNA Fragments

The hγ-IFN PCR DNA is blunt ended using T4 DNA polymerase. Specifically, 10 µl of PCR amplified DNA; 2 µl, 10x, T4 DNA polymerase buffer (0.33 M Tris-acetate, pH 7.9, 0.66 M potassium acetate, 0.10 M magnesium acetate, 5 mM dithiothreitol, 1 mg/ml BSA); 1 µl, 2.5 mM dNTP (a mixture containing equal molar concentrations of dATP, dGTP, dTTP and dCTP); 7 µl DI H₂O; 1 µl, 5,000 U/ml, Klenow fragment (EC 2.7.7.7, New England Biolabs, MA); and 1 µl, 3,000 U/ml, T4 DNA polymerase (EC 2.7.7.7, New England Biolabs, MA) are mixed together and incubated at 37 °C for 15 minutes. The reaction mixture is then incubated at room temperature for 40 minutes and followed by an incubation at 68°C for 15 minutes.

The blunt ended hγ-IFN is isolated by agarose gel electrophoresis. Specifically, 2 µl of loading dye (0.25% bromophenol blue; 0.25% xylene cyanol; and 50% glycerol) is added to reaction mixture and 4 µl is loaded into each of 5 lanes of a 1% agarose/Tris-borate-EDTA (TBE) gel containing ethidium bromide. Electrophoresis of the gel is performed for 1 hour at 100 volts. The desired DNA band containing hγ-IFN, approximately 500 base pairs in length, is visualized under ultraviolet light.

This band is removed from the gel by electrophoretic transfer onto NA 45 paper (Schleicher and Schuell, Keene, NH). The paper is incubated at 68°C for 40 minutes in 400 µl of high salt NET buffer (1 M NaCl; 0.1 mM EDTA; and 20 mM Tris, pH 8.0) to elute the DNA. The NA 45 paper is removed from solution and 400 µl of phenol:chloroform:isoamyl alcohol (25:24:1) is added. The solution is vortexed and centrifuged at 14,000 for 5 minutes. The upper aqueous phase is transferred to a fresh tube and 400 µl of chloroform:isoamyl alcohol (24:1) is added. The mixture is vortexed and centrifuged for 5 minutes. The upper aqueous phase is transferred, a second time, to a fresh tube and 700 µl of 100% ethanol is added. The tube is incubated at -20°C for 3 days. Following incubation, the DNA is precipitated from the tube by centrifugation for 20 minutes at 14,000 rpm. The supernatant is decanted and the pellet is rinsed with 500 µl of 70% ethyl alcohol. The pellet, containing blunt ended hγ-IFN DNA, is dried by centrifugation under vacuum and resuspended in 50 µl of DI H₂O.

The isolated blunt ended hγ-IFN DNA is phosphorylated using polynucleotide kinase. Specifically, 25 µl of blunt-ended hγ-IFN DNA, 3 µl of 10x kinase buffer (0.5 M Tris-HCl, pH 7.6; 0.1 M MgCl₂; 50 mM dithiothreitol; 1 mM spermidine; 1 mM EDTA), 3 µl of 10 mM ATP, and 1 µl of T4 polynucleotide kinase (10,000 U/ml, EC 2.7.1.78, New England Biolabs, MD) is mixed and incubated at 37°C for 1 hour 45 minutes. The enzyme is then heat inactivated by incubating at 68°C for 30 minutes.

### vii. Ligation of hγ-IFN PCR DNA into the SK⁺ Vector

An SK⁺ plasmid is digested with Hinc II restriction endonuclease and purified by agarose gel electrophoresis as described above. Specifically, 5.9 µl (1.7 mg/ml) SK⁺ plasmid DNA, 4 µl 10x Universal buffer (Stratagene, San Diego, CA), 30.1 µl DI H₂O, and 4 µl Hinc II, 10,000 U/ml, are mixed in a tube and incubated for 7 hours at 37°C. Following incubation, 4 µl of loading dye is added to the reaction mixture and 4 µl of this solution is added to each of 5 lanes of a 1% agarose/TBE gel containing ethidium bromide. Electrophoresis of the gel is performed for 2 hours at 105 volts. The Hinc II cut SK⁺ plasmid, 2,958 base pairs in length, is visualized with ultraviolet light. The digested SK⁺ plasmid is isolated from the gel using the method described in Example 1B.

Dephosphorylation of the Hinc II cleavage site of the plasmid is performed using CIAP. Specifically, 50 µl digested SK⁺ plasmid; 5 µl 1 M Tris, pH 8.0; 2.0 µl 15 mM EDTA, pH 8.0; 43 µl H₂O and 2 µl, 1,000 U/ml, CIAP (Boehringer Mannheim, Indianapolis, IN) are mixed in a tube and incubated at 37°C for 15 minutes. Following incubation, 2 µl CIAP is added and the solution is incubated at 55°C for 90 minutes. Following this incubation, 2.5 µl 20% SDS, 1 µl 0.5 M EDTA, pH 8.0, and 0.5 µl, 20 mg/ml, proteinase K (EC 3.4.21.14, Boehringer Mannheim, Indianapolis, IN) are added, and the solution is incubated at 55°C for 2 hours. This solution is cooled to room temperature, and 110 µl phenol:chloroform:isoamyl alcohol (25:24:1) is added. The mixture is vortexed and centrifuged at 14,000 rpm for 5 minutes. The upper aqueous phase is transferred to a fresh tube and 200 µl of 100% EtOH is added. This mixture is incubated at 70°C for 15 minutes. The tube is centrifuged and the pellet is rinsed with 500 µl of 70% EtOH. The pellet was then dried by centrifugation under a vacuum. The dephosphorylated SK⁺ plasmid is resuspended in 40 µl DI H₂O.

The hγ-IFN PCR DNA is ligated into the SK⁺ plasmid using T4 DNA ligase. Specifically, 30 µl blunt ended, phosphorylated, hγ-IFN PCR DNA reaction mixture, 2 µl dephosphorylated SK⁺ plasmid and 1 µl T4 DNA ligase are combined in a tube and incubated overnight at 16°C. DNA was isolated using a miniprep procedure. More specifically, the bacterial *E coli* strain DH5α is transformed with 15 µl of ligation reaction mixture, plated on Luria-Bertani agar plates (LB plates) containing ampicillin and 5-bromo-4-chloro-3-indolyl-β-D-galactoside (X-gal, Gold Biotechnology, St. Louis, MO), and incubated overnight at 37°C. DNA is isolated from white bacterial colonies using the procedure described by Sambrook *et al. supra.* The presence of the hγ-IFN gene is determined by restriction endonuclease cleavage with Xho I, Cla I, Ava II, Dra I, and Ssp I. The expected endonuclease restriction cleavage fragment sizes for plasmids containing the hγ-IFN gene are presented in Table 1. The isolated DNA plasmid is designated SK hγ-IFN and used in constructing the retroviral vectors.

**Table 1**

| Enzyme | Fragments (bp) |
|---|---|
| Xho I and Cla I | 500, 2,958 |
| Ava II | 222, 1,307, 1,937 |
| Dra I | 700, 1,149, 1,500 |
| Ssp I | 750, 1,296, 2,600 |

### viii. Ligation of hγ-IFN Gene into Retroviral Vector

The interferon gene is removed from SK hγ-IFN vector by digestion with Xho I and Cla I restriction endonucleases. The resulting fragment containing the hγ-IFN gene is approximately 500 bp in length, and is isolated in a 1% agarose/TBE gel electrophoresis as described in Example 5Cii. The Xho I-Cla I hγ-IFN fragment is then ligated into the KT-3B retroviral. This construct is designated KT hγ-IFN. The expression of hγ-IFN is determined by transforming DH5α bacterial strain with the KT hγ-IFN construct. Specifically, the bacteria is transformed with 15 µl of ligation reaction mixture. The transformed bacterial cells are plated on LB plates containing ampicillin. The plates are incubated overnight at 37° C and bacterial colonies are selected. The DNA is digested with Xho I, Cla I, Dra I, Nde I, and Ssp I. The expected endonuclease restriction cleavage fragment sizes for plasmids containing the hγ-IFN gene are presented in Table 2.

**Table 1**

| Enzyme | Fragments (bp) |
|---|---|
| Xho I and Cla I | 500, 6,500 |
| Nde I | 1,900, 5,100 |
| Dra I | 692, 2,700, 3,600 |
| Ssp I | 541, 1,700, 4,700 |

### C. Transduction of Packaging Cell Lines DA and Murine Tumor Cell Lines (B16F10 and L33) with mγ-IFN Retroviral Vector

### i. Plasmid DNA Transfection

293 2-3 cells (a cell line derived from 293 cells ATCC No. CRL 1573, WO 92/05266) 5.0 x 10⁵ cells are seeded at approximately 50% confluence on a 6 cm tissue culture dish. The following day, the media is replaced with 4 ml fresh media 4 hours prior to transfection. A standard calcium phosphate-DNA coprecipitation is performed by mixing 10.0 µg of KT mγ-IFN plasmid and 10.0 µg MLP G plasmid with a 2M CaCl solution, adding a 1x HEPES buffered saline solution, pH 6.9, and incubating for 15 minutes at room temperature. The calcium phosphate-DNA coprecipitate is transferred to the 293 2-3 cells, which are then incubated overnight at 37°C, 5% CO₂. The following morning, the cells are rinsed 3 times in 1x PBS, pH 7.0. Fresh media is added to the cells, followed by overnight incubation at 37°C, 10% CO₂. The following day, the media is collected from the cells and passed through a 0.45 µ filter. This supernatant is used to transduce packaging and tumor cell lines.

DA cells are seeded at 5.0 x 10⁵ cells/10 cm dish. 0.5 ml of the 293 2-3 supernatant stored at -70°C is added to the DA cells. The following day, G418 is added to these cells and a drug resistant pool is generated over the period of a week. DA clones are selected for vector production.

### ii. L33 Cell Line Transduction

L33 cells are seeded at 1.0 x 10⁵ cells/6 cm dish. 1.0 ml of the 293 2-3 supernatant stored at -70°C is added to the L33 cells. The following day, G418 is added to these cells and a drug resistant pool is generated over a period of a week. This pool of cells is dilution cloned by adding one cell to each well of 96 well plates, at which time cell lysates are prepared for analysis of major histocompatability complex (MHC) expression. A clone, L33/mγ-IFN #15, which had significantly increased levels of MHC expression is used in subsequent mouse studies.

### iii. B16F10 Cell Line Transduction

B16F10 cells (Dennert, USC Comprehensive Cancer Center, Los Angeles, CA; Warner, et. al., *Nature 300:*113-121, 1982) are seeded at 2.0 x 10⁵ cell/10 cm dish with a 4 µg/ml polybrene. 0.1 ml of supernatant from the DA mγ-IFN pool is added to the cells and incubated for 6 hours at 37°C, 10% CO₂. G418 is added after incubation and a drug resistant pool is generated. This pool is dilution cloned by adding 1.0 cells to each well of 96 well plates. Twenty-four clones are expanded to 24 well plates, then to 6 well plates, at which time cell lysates are made for analysis of MHC expression. A clone, B16F10/mγ-IFN #4, having significantly increased levels of MHC expression is used in subsequent mouse studies.

### iv. CT 26 and Lewis Lung Tumor Cell Line Transduction

Colon tumor 26 (CT 26) (Brattain, Baylor College of Medicine, Houston TX) and Lewis lung tumor (LLT) (Waude, Southern Research Institute, Birmingham, AL, ATCC No. CRL 1642) cells are seeded 1.0 x 10⁵ cells/6 cm plate for each cell line in DMEM with 10% FBS and 4 µg/ml polybrene and incubated for 24 hours at 37°C, 10% CO₂. After incubation, 1.0 ml of KT mγ-IFN retroviral vector (9.0 x 10⁶ cfu/ml) is added to each respective cell line and incubated for 24 hours at 37°C, 10% CO₂. Following incubation, the medium is changed and replaced with DMEM with 10% FBS and 400 µg/ml G418. These cell lines are kept under G418 selection for approximately two weeks. Selected CT 26 and LLT resistant pools are dilution cloned by adding one cell to each well of 96 well plates. Two 96 well plates are seeded for each G418-selected pool. CT 26 and LLT mγ-IFN expressing clones are expanded into 24 well plates and then to 6 well plates. Lysates are prepared of each clone and analyzed for up-regulated MHC protein expression by Western blot analysis. A clone, CT 26/mγ-IFN, having up-regulated MHC protein expression is selected. All LLT studies are conducted using the non-clonal pool of the mγ-IFN expressing LLT cells.

### D. Transduction of Packaging Cell Line and Human Cell Lines with hγ-IFN Retroviral Vector

### i. Plasmid DNA Transfection

Approximately 5.0 x 10⁵ 293 2-3 cells are seeded at approximately 50% confluence on a 6 cm tissue culture dish. The following day, the media is replaced with 3 ml fresh media 4 hours prior to transfection. At the time of transfection, 5 µl of KT hγ-IFN plasmid is mixed with 2.0 µg MLP G plasmid in 0.1x Tris-EDTA, pH 7.4. A standard calcium phosphate-DNA coprecipitation is performed mixing the DNA with a CaCl solution, adding a 1x HEPES buffered saline solution, 2M, pH 6.9, and incubating for 15 minutes at room temperature. The calcium phosphate-DNA coprecipitate is transferred to the 293 2-3 cells, which are then incubated overnight at 37°C, 5% CO₂. The following morning, the cells are rinsed 3 times in 1x PBS, pH 7.0. Fresh media is added to the cells, followed by overnight incubation at 37°C in 10% CO₂. The following day, media is collected from the cells and passed through a 0.45 µ filter. The filtered supernatant is stored at -70°C for use in packaging cell transductions.

### ii. DA Transfection

Approximately 5.0 x 10⁵ HX cells are seeded at approximately 50% confluence on a 6 cm tissue culture dish. The following day, the media is replaced with 4 ml fresh media 4 hours prior to transfection. A standard calcium phosphate-DNA coprecipitation is performed by mixing 2 µl, 6.0 µg, of KT hγ-IFN plasmid with a 120 ml 2M CaCl solution, adding 240 ml of a 1x HEPES buffered saline solution, pH 6.9, and incubating for 15 minutes at room temperature. The calcium phosphate-DNA coprecipitate is transferred to the HX cells, which are then incubated overnight at 37°C, 5% CO₂. The following morning, the cells are rinsed 3 times with 1x PBS, pH 7.0. Fresh media is added to the cells and followed by overnight incubation at 37°C, 10% CO₂. The following day, the media is collected off the cells and passed through a 0.45 µ filter.

The previous day, DA cells are seeded at 1.0 x 10⁵ cells/6 cm dish. 1.0 ml of the freshly collected HX supernatant is added to the DA cells. The following day, G418 is added to these cells and a drug resistant pool is generated over a 2-week period. The pool of cells is dilution cloned by adding 1.0 cell to each well of 96 well plates. Twenty-four clones are expanded to 24 well plates, then to 6 well plates. The cell supernatants are collected for titering and clones with titers of at least 5.0 x 10⁵ cfu/ml are selected. A DA clone is selected and designated DA/hγ-IFN.

### iii. Human Cell Line Transductions

The following adherent human cell lines are seeded at 5 x 10⁵ cells/10 cm dish with 4 µg/ml polybrene: HT 1080 (ATCC No. CCL 121); Hela (ATCC No. CCL 2); 143B (ATCC No. CRL 8303); Caski (ATCC No. CRL 1550). The following suspension of human cell lines are seeded at 5 x 10⁵ cells/6 ml media with 4.0 µg/ml polybrene: HL 60 (ATCC No. CCL 240); U937 (ATCC No. CRL 1593); CEM (ATCC No. CCL 119); Hut 78 (ATCC No. TIB 161); Duadi (ATCC No. CCL 213); K562 (ATCC No. CCL 243). The following day, 1.0 ml of filtered supernatant from the DA hγ-IFN pool is added to each of the 10 cell cultures. The next day, 800 µg/ml, G418 is added to the media of all 10 cell cultures. The cultures are maintained until selection is complete and sufficient cell numbers are generated. Radioimmunoprecipitation assay (RIPA) lysates are made of selected cultures for analysis of hγ-IFN expression. Specifically, the RIPA lysates are electrophoresed on a 9% acrylamide gel and then transferred to Immobilon™ membrane (Millipore, Philadelphia, PA). The membrane is probed with an antibody against human MHC, W6/32 (Accurate Chemicals, Westbury, NY), and an autoradiogram is performed. Up-regulation of MHC is seen in Daudi, HL60; HeLa and Caski cell lines. Following analysis these cells are frozen -70 °C in preparation for of hγ-IFN expression.

### iv. Human Melanoma Transduction

Melanoma cells lines DM6, DM92, DM252, DM265, DM262 and DM259 (cell lines established from human tumor biopsies, Seigler, Duke University, NC) are established from human tumor biopsies. Each cell line is seeded at 10⁶ cells/10 cm dish with 4 µg/ml polybrene. The following day, 5-10 mls of filtered supernatant from the DA hγ-IFN pool is added to each of the cell cultures. This corresponds to a multiplicity of infection (MOI), of 5-10. The next day, the cells are selected with 800 µg/ml of G418. Samples of the supernatants of all transduced cell lines are collected. The supernatant is filtered through a 0.45 µ filter and stored at -70°C in preparation for analysis of hγ-IFN expression.

### E. MHC Class I Expression

### i. Determination of Mouse MHC Class I Expression by Western Blot Analysis

RIPA lysates are prepared from confluent plates of cells. Specifically, the media is first aspirated off the cells. Depending upon the size of the culture plate containing the cells, a volume of 100 to 500 µl ice cold RIPA lysis buffer (10 mM Tris, pH 7.4; 1% Nonidet P40 (Calbiochem, San Diego, CA); 0.1% SDS; 150 mM NaCI) is added to the cells. Cells are scrapped from plates using a micropipet and the mixture is transferred to a microfuge tube. The tube is centrifuged for 5 minutes to precipitate cellular debris and the lysate supernatant is transferred to another tube. The lysates are electrophoresed on a 10% SDS-polyacrylamide gel and the protein bands are transferred to an Immobilon™ membrane in CAPS buffer (10 mM CAPS, pH 11.0; 10% methoanol) at 10 to 60 volts for 2 to 18 hours. The membrane is transferred from the CAPS buffer to 5% Blotto (5% nonfat dry milk; 50 mM Tris, pH 7.4; 150 mM NaCI; 0.02% Na azide, and 0.05% Tween 20) and probed with a rat IgM antibody, 72.14S (Richard Dutton, UCSD, San Diego, CA). This antibody probe is directed against a conserved intracellular region of the mouse MHC class I molecule. Antibody binding to the membrane is detected by the use of ¹²⁵I-Protein A.

### ii. Analysis of MHC Expression in Murine Tumor Cell Lines With and Without mγ-IFN Retroviral Vector

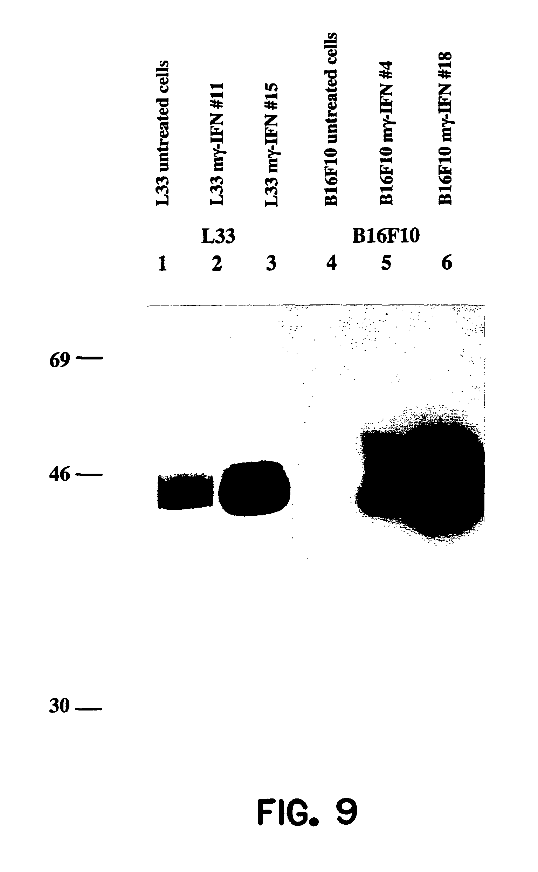

MHC expression is confirmed by Western blot and flow cytometry analysis. Specifically, L33, CT 26, and LLT parent cell lines express relatively normal levels of MHC class I protein and the B16F10 parent cell line has down-regulated levels of MHC class I protein. The mγ-IFN-transduced pools and clones of these cell lines express greater levels of MHC class I than their corresponding parent cell lines which is demonstrated by Western blot and flow cytometry analysis. Western blot of lysates of various CT 26 mγ-IFN, LLT mγ-IFN subclones, and the parent CT 26 and LLT cell lines show up-regulated MHC class I expression. A Western blot analysis of two L33 mγ-IFN subclones, two B16F10 mγ-IFN subclones, parent L33 cell line, and parent B16F10 cell line illustrates the up-regulated MHC class I expression of the mγ-IFN clones as compared to the parent cells, Figure 9. Flow cytometry analysis of L33 and two L33 mγ-IFN subclones illustrates that the subclones have considerable more MHC class I expressed on the surface as compared to the parent cells. Flow cytometry analysis is performed on harvested cells. Specifically, cells are incubated with an MHC class I specific antibody 34.4 anti-D^{d} antibody (Richard Dutton, UCSD, CA). This bound antibody is detected by incubating the 34.4 anti-D^{d} bound cells with fluoroscene conjugated rabbit anti-mouse IgG antibody (Capell, Durham, NC). Fluorescent emission from the cell bound antibody-fluorescence conjugate is detected and quantitated by flow cytometry analysis.

### F. HLA Class I and hγ-IFN Expression in Transduced Human Melanomas

### i. Determination of Human MHC (HLA) Class 1 Expression By Western Blot Analysis

HLA expression is determined essentially as described in Example 5E for murine MHC except that the HLA Class I specific antibody W6/32 is used.

### ii. Analysis and HLA Expression In Human Melanomas With and Without hγ-IFN Retroviral Vector

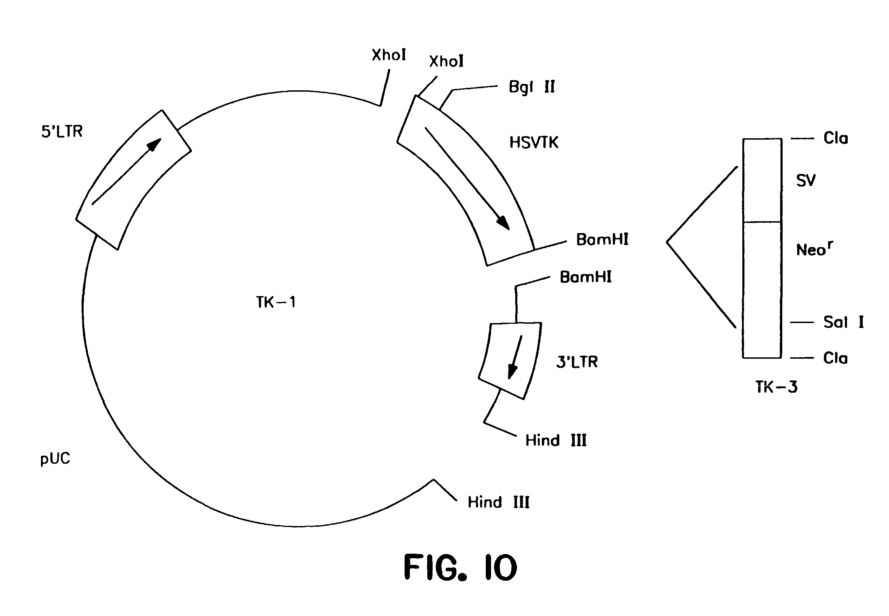

DM92, DM252, or DM265 are treated with hγ-IFN vector or hIL-2 as a control for vector transduction. The data in Figure 10 indicates that hγ-IFN vector increases the level of HLA compared with the non-transduced cells whereas, those transduced with IL-2 did not. Transduction of DM265 results in increased HLA even though there is little or no hγ-IFN secreted into the medium.

hγ-IFN is quantified by viral inhibition of encephalomyocarditis virus on a human cell line A549, ATCC CCL 185. The activity of γ-IFN is quantified by measuring the protective effect against cytocidal infection with encephalomyocarditis (EMC) virus. The filtered supernatants are added to the A549 cells at different concentrations and then the cells are challenged with the EMC virus. Hγ-IFN samples are co-assayed with the appropriate NIH reference reagents, and the results are normalized to NIH reference units (U/ml) (Brennan *et al*., *Biotechniques 1*:78, 1983).

For melanoma cell lines the activity is determined by comparison with authentic NIH reference reagents and normalized to NIH reference units (U/mL) (Brennan *et al., supra*). Human melanomas transduced with the hγ-IFN retroviral vector express readily detectable levels of biologically active hγ-IFN. This expression is often stable with time but sometimes decreases with time in culture. This time dependent decrease in hγ-IFN may indicate that expression of the gene is somewhat toxic, thus resulting in a selective advantage for cells expressing low levels of hγ-IFN.

### G. Determination of mγ-IFN Activity

The activity of mγ-IFN is quantified by measuring the protective effect against cytocidal infection with encephalomyocarditis (EMC) virus. The mouse cell line 3T3TK⁻, (NIAID Research Reference Reagent Note #28, November, 1983 edition.), is used to assay for mγ-IFN. 3T3TK⁻ cells are added to the wells of 96-well microtiter tissue culture plates. When the cells reach confluency, serial dilutions of cell culture supernatants (test samples) to be evaluated for the presence of interferon are applied to the cells. After an incubation period, the cells are challenged with the EMC virus. Mγ-IFN samples are co-assayed with the appropriate NIH reference reagents, and the results are normalized to NIH reference units (U/ml) (Brennan *et al*., *supra*) Activities recorded for cell types CT 26 (Brittain, *et al*., *Cancer 36*:2441, 1975), BC10ME, LLT, and B16F10 are presented in Tables 1 and 2.

**Table 1**

| γ-IFN PRODUCTION IN VARIOUS BALB/C CELL LINES | |
|---|---|
| Cell Type | U/ml |
| CT26 | 3.5 |
| CT26 IFN pool | 3400 |
| CT26 IFN clone #10 | 4500 |
| BC10ME | 22 |
| BC10ME IFN pool | 110 |
| L33 | < 0.3 |
| L33 -IFN | 7.7 |

**Table 2**

| γ-IFN PRODUCTION IN VARIOUS C57BL/6 CELL LINES | |
|---|---|
| Cell Type | U/ml |
| LLT | < 0.7 |
| LLT IFN pool | 82 |
| LLT IFN #21 | 40 |
| LLT IFN #28 | 2.1 |
| LLT IFN pool tumor | 21 |
| LLT IFN pool lg met | 11 |
| B16F10 | < 2.6 |
| B16F10 IFN #4 | 90 |

### H. Stimulation of an Immune Response in Mice by Direct Injection of Retroviral Vector

Experiments are performed to evaluate the ability of recombinant retroviral vectors to induce expression of HIV *env* proteins following direct injection in mice. Approximately 10⁴ to 10⁷ cfu of a combination of the recombinant retrovirus carrying the HIV III B *env* encoding vector and the recombinant retrovirus carrying the hγ-IFN encoding vector are injected twice at three week intervals either by the I.P. or I.M. route. A separate set of mice are injected with 10⁵ to 10⁸ cfu of the HIV III B *env* vector alone. Spleen cells are prepared for CTL approximately 7 to 14 days after the second injection of vector and CTL are restimulated *in vitro* using irradiated BC *env* stimulator cells as described above (Example 2J). The results showed that direct vector injection of the HIV III B *env* vector and of the combination stimulates the development of CTL which kill BC*env* target cells but not control BC cells. However, the combination vector stimulates the CTL response at lower doses of HIV III B *env* vector. Thus, the injection of 10⁴ to 10⁵ units of retrovirus (an amount of vector that does not usually stimulate an immune response) may induce expression of HIV envelope in conjunction with local mγ-IFN production in the host which thereby leads to the induction of a specific CTL immune response.

The same procedure can be used in humans to potentiate the response to HIV antigen by combination administration of 10⁶, 10⁷, 10⁸, 10⁹, 10¹⁰, or 10¹¹ cfu of HIV recombinant retroviral vector or 10⁶, 10⁷, 10⁸, 10⁹, 10¹⁰, or 10¹¹ cfu of hγ-IFN recombinant retroviral vector.

### I. Human and Non-Human Primate Administration Protocol

Vector construct administration protocol for human and non-human primates (e.g., monkey and chimpanzee) is performed as described in Example 2G.

### Example 6

### Administration of Retroviral Vectors Expressing GMCSF and TK

### A. GMCSF Retroviral Vector Construction

The vector p91023(B)(ATCC No. 39754) containing the cDNA for human GMCSF is used as a template for the PCR. Two oligonucleotides are synthesized by BioSynthesis Inc. (Lewisville, TX) The first oligonucleotide is the sense sequence corresponding to bases 29 to 54 of GMCSF with two Xho I sites at the 5' end. The second oligonucleotide is the antisense sequence corresponding to bases 520 to 493 of GMCSF with two Cla I sites at the 5' end.

Ten nanograms of template DNA is used in a PCR reaction with Vent polymerase (New England Biolabs, Beverly MA) and the two oligonucleotides Nos. 47 and 36 as primers. The PCR product is digested with Xho I and Cla I restriction enzymes and ligated into Xho I- Cla I digested KT3 vector. This construct is designated pKT3-GMCSF.

Protein production from this vector is verified by ELISA (Biosource International, Camarillo, CA ). Biological activity of this vector product is verified by the colony formation assay of Koeffler and Gould (*Science 200*:1153, 1978) using KG-1 cells (ATCC CCL 246). Ten µl samples are added to microtiter wells containing 400 KG-1 cells in 140 µl of Iscoves medium with 0.3% agar, 20% fetal calf serum, and 10⁻⁴ M α-thioglycerol. The assays are incubated at 37°C for 14 days, and the number of colonies over background is scored.

### B. Construction of TK-1 and TK-3 Retroviral Vectors and Production of Vector Particles

The vector TK-1 is constructed from the following fragments:
1. the 5 Kb Xho I/Hind III 5' LTR and plasmid sequences are isolated from p31N2R5(+),
2. HSVTK coding sequences lacking transcriptional termination sequences are isolated as a 1. 2 Kb Xho I/Bam HI fragment from pTKΔA, and
3. 3' LTR sequences are isolated as a 1.0 Kb Bam E/Hind III fragment from pN2R3(-).

These fragments were mixed, ligated, transformed into bacteria, and individual clones identified by restriction enzyme analysis. This vector construct is designated TK-1 (Figure 10).

TK-3 was constructed by linearizing TK-1 with Bam HI, filling in the 5' overhang and blunt-end ligating a 5'-filled Cla I fragment containing the bacterial lac UV5 promoter, SV40 early promoter, plus Tn5 Neo^{r} gene ( Figure 10). Kanamycin-resistant clones were isolated and individual clones were screened for the proper orientation by restriction enzyme analysis.

These constructs were used to generate infectious recombinant vector particles in conjunction with a packaging cell line, such as DA as described above.

### C. Tumor Growth Cytotoxicity Assays on CT26 and CT26TK, CT26GMCSF, and the Combination of CT26TK and CT26GMCSF

### i. In Vivo Tumor Growth

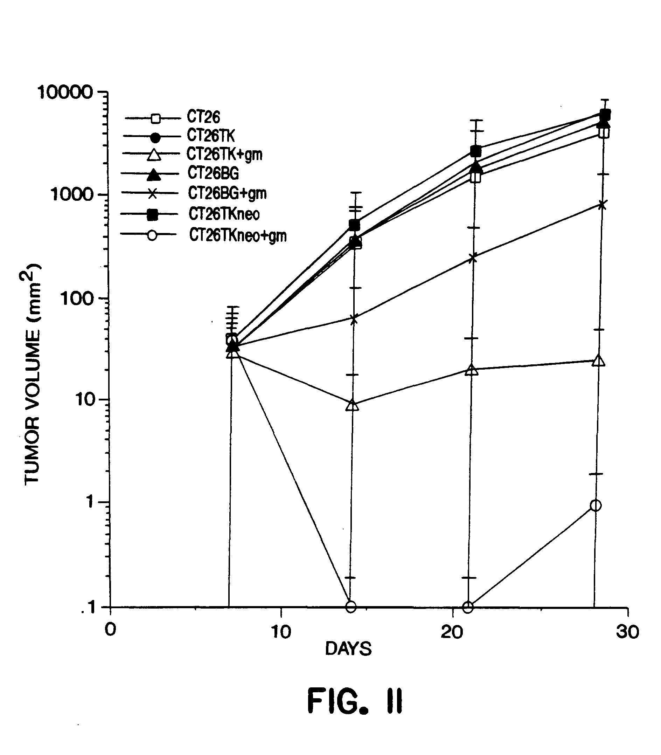

In order to determine whether ganciclovir has an effect on the growth of unmodified CT26 tumor cells (colon tumor 26, Brattain, Baylor College of Medicine, Houston, TX) *in vivo,* 2 groups of 7 mice are injected S.C. with 2.0 x 10⁵ unmodified CT26 cells and 2 groups of 7 mice are injected S.C. with 2.0 x 10⁵ CT26TK *neo* cells. Seven days after tumor implantation, one group of CT26 injected mice and one group of CT26TK *neo* injected mice are placed on a twice daily (AM and PM) regimen of I.P. ganciclovir at 62.5 mg/Kg. These mice are treated for 12 days or until the CT26TK *neo* injected animals have no detectable tumor burden. Tumor growth is monitored over a three week period. Mice injected with CT26 and treated with ganciclovir had tumors that were somewhat smaller than untreated mice injected with CT26, indicating a small HSVTK-independent inhibition of tumor growth (Figure 11). However, a dramatic decrease in tumor burden was observed if, and only if, CT26 TK *neo* containing mice were treated with ganciclovir (Figure 11). Similarly, the growth of CT26 cells carrying GMCSF and/or GMCSF and TK (introduced in combination) can be compared to the normal tumor.

### ii. In Vivo Transduction of CT26 Tumor Cells by TK-3 and GMCSF

This experiment is designed to demonstrate that TK-3 vector and the GMCSF vector can deliver the HSVTK and GMCSF genes to target cells *in vivo* and inhibit tumor growth in the presence of ganciclovir. Firstly, the effect TK-3 alone was examined. Six groups of 10 mice are injected S.C. with 1.0 x 10⁵ CT26 tumor cells. In addition, one group of 10 mice is injected S.C. with 1.0 x 10⁵ CT26TK *neo* cells as a control. The area of the S.C. injection is circled with a water-resistant marker. Twenty-four hours after tumor implantation, TK-3, GMCSF, or *β-gal* viral supernatants (0.2 ml total) formulated with polybrene (4 µg/ml) are injected within the area marked by the water-resistant marker. Vector administration is continued for four consecutive days with one dose of vector per day. Each vector dose contains 2.0 x 10⁵ cfu/ml. The results are shown in Figure 11. The data indicates that a substantial reduction of growth of CT26 occurred only when the animal was injected with both TK-3 and ganciclovir. The level of inhibition was not as substantial as that observed for CT26 TK *neo in vitro* transduced and selected presumably in due to less than 100% *in vivo* transduction. Surprisingly, there was also a decrease in tumor growth when treated with the control vector, CB-β-*gal* and ganciclovir. This may indicate some inhibition of tumor growth due to the vector cell free supernatant itself, added with the previously observed small decrease caused by ganciclovir alone (Figure 11). Regardless of that observation, the average tumor size is significantly smaller in the TK-3/ganciclovir treated animals than that of the CB-β-*gal*/ganciclovir treated animals (7 fold and 10 fold and 75-fold smaller, at the 14 and 21 day time points, respectively). Thus, it appears that *in vivo* transduction by direct injection of HSVTK expressing retroviral vectors can result in inhibition of tumor growth in combination with ganciclovir administration.

The same experiment is repeated, only in this case, the directly administered vector is a combination of GMCSF and TK vector in approximately equal proportions. In this case, the regression of tumors with TK alone, GMCSF alone, and other controls are included. The combination of the TK and GMCSF vectors, gives greater regression than any of the other control treatments. The proportion and titer of TK and GMCSF vector can be varied from 1:10 to 10:1 and be 10⁴, 10⁵, 10⁶, 10⁷, 10⁸, 10⁹, 10¹⁰, or 10¹¹ cfu/ml to provide more efficient tumor regression, depending on the timing of the treatment, the tumor burden, and the type of tumor model.

A preferred preparation of the GMCSF vector is 3:1. Alternatively, the TK and GMCSF vector can be administered on alternate days. Twenty-four hours after the last vector treatment, these mice are injected I.P. twice daily (AM and PM) with ganciclovir at 62.5 mg/Kg for 8 days. Finally, the mice receive a single daily dose of ganciclovir at 62.5 mg/Kg until the end of the experiment. Tumor growth is measured over a 4 week period (Figure 11). The experiment is summarized in Table 3 below, with the predicted outcome. The same procedure can be used in treating human tumors using combinations of a prodrug vector such as KT-3 and a cytokine vector such as the one encoding GMCSF, interferon, IL2, and others. Vector is administered in Example 2Gii.

**Table 3**

| Group | Innoculum | Retroviral Vector | Ganciclovir (65 mg/Kg) | Tumor Growth |
|---|---|---|---|---|
| 1 | CT26 | None | - | + |
| 2 | CT26 | None | + | + |
| 3 | CT26 | TK-3 | - | + |
| 4 | CT26 | TK-3 | + | reduced |
| 5 | CT26 | GMCSF | - | + |
| 6 | CT26 | GMCSF | + | + |
| 7 | CT26 | TK-3 + GMCSF | - | + |
| 8 | CT26 | TK-3 + GMCSF | + | greatly reduced |
| 9 | CT26 | *β-gal* | - | + |
| 10 | CT26 | *β-gal* | + | + |
| 11 | CT26 TK-3 | None | - | + |
| | | None | + | none |
| 12 | CT26 *β-gal* | None | - | + |
| | | None | + | + |
| 13 | CT26 GMCSF | None | - | + |
| | | None | + | + |

In addition to delivering a gene of interest *in vivo* using direct injection of vector, mice are treated by injecting the vector producer cell line from a PCL such as DA into, or around the tumor, or both. Varying numbers of irradiated of unirradiated vector producer cells are injected with and without a polycationic reagent to improve transduction efficiencies. Control mice can be injected with diluent D17 (ATCC No. CCL 183) transduced with TK-3 and a CB-β-*gal* vector producing cell lines (VCL). After sufficient time for *in vivo* transduction, approximately 2 weeks, ganciclovir injections commence and efficacy is determined by tumor measurements and/or overall survival.

### Example 7

### Administration of Recombinant Retroviral Vectors Expressing IL-2 and γ-IFN

### A. Cloning of hIL-2 into KT-3B

The method for cloning hIL-2 into KT-3B retroviral vector is essentially identical to the procedure for cloning hγ-IFN into KT-3B (Example 5B), with the exception that different primers are required for amplification of the hIL-2 DNA sequence. The following hIL-2 PCR primer sequences are used:

This primer is complimentary to a sequence of the hIL-2 cDNA downstream of the stop codon.

This primer is the sense sequence of the hIL-2 gene complimentary to the 5' coding region beginning at the ATG start codon. The 5' end of the primer contains a Xho I restriction site.

The primer is the anti-sense sequence of the hIL-2 gene complimentary to the 3' coding region ending at the TAA stop codon. The 5' end of the primer contains the Cla I restriction site.

### B. Transduction of Packaging Cell Lines DA and Murine Tumor Cell Line B16F10 with mγ-IFN and hIL-2 Retroviral Vector

Transductions of packaging cell line DA and murine tumor cell line B16F10 with mγ-IFN and hIL-2 retroviral vectors were performed as described in Example 5C.

### C. Tumorigenicity of B16F10 and B16F10/mγ-IFN #4 Cells

Parental B16F10, a selected clone B16F10/mγ-IFN#4, and B16F10/hIL-2 cells are harvested, counted, and resuspended to a concentration of 8.0 x 10⁵ cells/ml in Hanks buffered salt solution (HBSS, Irvine Scientific, CA). Two Black 6 mice are injected I.V. with 0.5 ml of the B16F10 cell suspension (3.0 x 10⁵ cells). Five Black 6 mice are injected I.V. with 0.5 ml of the B16F10/mγ-IFN#4 cell suspension and five black 6 are injected I.V. with 0.5 ml of B16F10/hIL-2 cell suspension. Fourteen days after injection, the lungs are removed from the mice, stained, and preserved in Bouin's Solution (Sigma, St. Louis, MO). The four lobes of the lungs are separated, examined under 10x magnification, and the number of black tumors present on each is determined.

The average number of tumors per lung for each group and the standard deviation is measured to show the effects of the γ-IFN vectors, IL-2 vector, and the concentration of vectors.

### D. Direct Administration of Vector into Tumor Bearing Animals

### i. Direct Administration of Vector into Mice

Mouse tumor systems may be utilized to show that cell mediated immune responses can be enhanced by direct administration of a vector construct which expresses at least one anti-tumor agent. For example, six to eight week old female Balb/C or C57B1/6 mice are injected subcutaneously with 1 x 10⁵ to 2 x 10⁵ tumor cells which are allowed to grow within the mice for one to two weeks. The resulting tumors may be of variable size (usually 1-4 mm³ in volume) as long as the graft is not compromised by either infection or ulceration. One-tenth to two-tenths of a milliliter of a vector construct which expresses an anti-tumor agent such as γ-IFN, vector construct that expresses IL-2, or a combination of both vectors (minimum titer 10⁶ cfu/ml) is then injected intratumorally (with or without polybrene or promatine sulfate to increase efficiency of transduction). Multiple injections of the vector are given to the tumor every two to three days.

Depending on the parameters of the particular experiment, the nature of the vector preparations may be variable as well. The vector may be from filtered or unfiltered supernatant from VCL, or may be processed further by filtration, concentration or dialysis and formulation. Other standard purification techniques, such as gel filtration and ion exchange chromatography, may also be utilized to purify the vector. For example, dialysis can be used to eliminate γ-IFN that has been produced by the VCL itself (and which, if administered, may affect tumor growth). Dialysis may also be used to remove possible inhibitors of transduction. Another option is to perform intratumor injections of the γ-IFN and IL-2 VCL, or a combination of VCL's in order to more extensively introduce the vector. Briefly, cells are injected after being spun down from culture fluid and resuspended in a pharmaceutically acceptable medium (*e.g.*, PBS containing 1 mg/ml human serum albumin (HSA)). As few as 10⁵ cells may be used within this aspect of the invention.

Efficacy of the vector construct may be determined by measuring the reduction in primary tumor growth, the reduction in tumor burden (as determined by decreased tumor volume), or by the induction of increased T-cell activity against tumor target cells (as measured in an *in vitro* assay system using lymphocytes isolated from the spleens of these tumor bearing cells). In a metastatic murine tumor model, efficacy may also be determined by first injecting tumor cells that are metastatic, and, when the tumor is 1-4 mm³ in volume, injecting vector several times into that tumor. The primary tumor graft may then be surgically removed after 2-3 weeks, and the reduction in metastases to the established target organ (lung, kidney, liver, etc.) counted. To measure the change in metastases in a target organ, the organ may be removed, weighed, and compared to a non-tumor bearing organ. In addition, the amount of metastases in the target organ may be measured by counting the number of visible metastatic nodules by using a low powered dissecting microscope.

### ii. Direct Administration of Vector into Humans

For humans, the preferred location for direct administration of a vector construct depends on the location of the tumor or tumors. The hγ-IFN (Example 5C), IL-2 gene, or other sequences which encode anti-tumor agents or combination of these agents may be introduced directly into solid tumors by vector administration. They may also be delivered to leukemias, lymphomas or ascites tumors. For skin lesions such as melanomas, the vector may be directly injected into or around the lesion. At least 10⁵ cfu/per vector of vector particles should be administered, with preferably more than 10⁶ cfu in a pharmaceutically acceptable formulation (*e*.*g*., 10 mg/ml lactose, 1 mg/ml HSA, 25 mM Tris pH 7.2 and 105 mM NaCl). For internal tumor lesions, the effected tumor may be localized by X-ray, CT scan, antibody imaging, or other methods known to those skilled in the art of tumor localization. Vector injection may be through the skin into internal lesions, or by adaptations of bronchoscopy (for lungs), sigmoidoscopy (for colorectal or esophageal tumors) or intra-arterial or intra-blood vessel catheter (for many types of vascularized solid tumors). The injection may be into or around the tumor lesion. The efficiency of induction of a biological response may be measured by CTL assay or by delayed type hypersensitivity (DTH) reactions to the tumor. Efficacy and clinical responses may be determined by measuring the tumor burden using X-ray, CT scan, antibody imaging, or other methods known to those skilled in the art of tumor localization.

### Example 8

### Administration of Retroviral Vectors Expressing Glucocerebrosidase and E3/19K

### A. Construction of KT-3B-GC

A glucocerebrosidase (GC) cDNA clone containing a Xho I restriction enzyme site 5' of the cDNA coding sequence and a Cla I restriction enzyme site 3' of the cDNA coding sequence is first generated. The clone is generated by digesting pMFG-GC (Ohashi *et al*., *PNAS 89*:11332, 1992, Nolta *et al*., *Blood, 75*:787, 1991) with Nco I (New England Biolabs, Beverly, MA), blunted with Vent DNA polymerase, then ligated with Xho I linkers. The plasmid is then digested with Bam HI, blunted with Vent DNA polymerase, then ligated to Cla I linkers. The fragment is then digested with Xho I and Cla I and ligated in a three part ligation in which the Xho I -Cla I GC fragment and the 1.0 Kb MoMLV 3' LTR Cla I-Hind III fragment are inserted into the Xho I-Hind III site of pUC31/N2R5gM plasmid (Example 1). This construct is designated KT-3B-GC.

### B. Cloning of E3/19K Gene into KT-3B

### i. Isolation and Purification of Adenovirus

The isolation and purification of adenovirus is described by Green *et al*. (*Methods in Enzymology 58*: 425, 1979). Specifically, five liters of Hela cells (3-6.0 x 10⁵ cells/ml) are infected with 100-500 plaque forming units (pfu) per ml of adenovirus type 2 (Ad2) virions (ATCC No. VR-846). After incubation at 37°C for 30-40 hours, the cells are placed on ice, harvested by centrifugation at 230 xg for 20 minutes at 4°C, and resuspended in Tris-HCl buffer (pH 8.1). The pellets are mechanically disrupted by sonication and homogenized in trichlorotrifluoroethane prior to centrifugation at 1,000 xg for 10 min. The upper aqueous layer is removed and layered over 10 mis of CsCI (1.43 g/cm³) and centrifuged in a SW27 rotor for 1 hour at 20,000 rpm. The opalescent viral band is removed and adjusted to 1.34 g/cm³ with CsCl and further centrifuged in a Ti 50 rotor for 16-20 hours at 30,000 rpm. The visible viral band in the middle of the gradient is removed and stored at 4°C until purification of adenoviral DNA.

### ii. Isolation and Purification of Adenovirus DNA

The adenovirus band is incubated with protease for 1 hour at 37°C to digest proteins. After centrifugation at 7,800 xg for 10 minutes at 4°C, the particles are solubilized in 5% SDS at room temperature for 30 minutes before being extracted with equal volumes of phenol. The upper aqueous phase is removed, re-extracted with phenol, extracted three times with ether, and dialyzed in Tris buffer for 24 hours. The viral Ad2 DNA is precipitated in ethanol, washed in ethanol, and resuspended in Tris-EDTA buffer (pH 8.1). Approximately 0.5 mg of viral Ad2 DNA is isolated from virus produced in 1.0 L of cells.

### iii. Isolation of E3/19K Gene

The viral Ad2 DNA is digested with EcoR I and separated by electrophoresis on a 1% agarose gel. The resulting 2.7 Kb Ad2 EcoR I D fragments, located in the Ad2 coordinate region 75.9 to 83.4, containing the E3/19K gene (Herisse *et al*., *Nucleic Acids Research 8*:2173, 1980, Cladaras *et al*., *Virology 140*:28, 1985) are eluted by electrophoresis, phenol extracted, ethanol precipitated, and dissolved in Tris-EDTA (pH 8.1).

### iv. Cloning of E3/19K Gene into KT-3B

The E3/19K gene is cloned into the EcoR I site of PUC1813. PUC1813 is prepared as essentially described by Kay *et al*. (*Nucleic Acids Research 15*:2778, 1987) and Gray *et al*. (*PNAS 80*:5842, 1983). The E3/19K is retrieved by EcoR I digestion and the isolated fragment is cloned into the EcoR I site of phosphatase-treated pSP73 plasmid. This construct is designated SP-E3/19K. The orientation of the SP-E3/19K cDNA is verified by using appropriate restriction enzyme digestion and DNA sequencing. In the sense orientation, the 5' end of the cDNA is adjacent to the Xho I site of the pSP73 polylinker and the 3' end adjacent to the Cla I site. The Xho I-Cla I fragment containing the E3/19K cDNA in either sense or antisense orientation is retrieved from the SP-E3/19K construct and cloned into the Xho I-Cla I site of the KT-3B retroviral. This construct is designated KT-3B/E3/19K.

### C. Cloning of PCR Amplified E3/19K Gene into KT-3B

### i. PCR Amplification of E3/19K Gene

The Ad2 DNA E3/19K gene, including the amino terminal signal sequence, followed by the intraluminal domain and carboxy terminal cytoplasmic tail which allow the E3/19K protein to embed itself in the endoplasmic reticulum (ER), is located between viral nucleotides 28,812 and 29,288. Isolation of the Ad2 E3/19K gene from the viral genomic DNA is accomplished by PCR amplification, with the primer pair shown below:
The forward primer corresponds to the Ad2 nucleotide sequences 28,812 to 28,835. The reverse primer corresponds to the Ad2 nucleotide sequences 29,241 to 29,213.

In addition to the Ad2 complementary sequences, both primers contain a five nucleotide "buffer sequence" at their 5' ends for efficient enzyme digestion of the PCT amplicon products. This sequence in the forward primer is followed by the Xho I recognition site and by the Cla I recognition site in the reverse primer. Thus, in the 5' to 3' direction, the E3/19K gene is flanked by Xho I and Cla I recognition sites. Amplification of the E3/19K gene from Ad2 DNA is accomplished with the following PCR cycle protocol:

| Temperature°C | Time (min) | No. Cycles |
|---|---|---|
| 94 | 2.0 | 1 |
| 94 | 0.5 | |
| | | |
| 55 | 0.17 | 5 |
| 72 | 3.5 | |
| | | |
| 94 | 0.5 | 30 |
| 70 | 3.5 | |
| | | |
| 72 | 10.0 | 10 |

### ii. Ligation of PCR Amplified E3/19K Gene into KT-3B

The E3/19K gene from the SP-E3/19K construct, approximately 780 bp in length, is removed and isolated by 1% agarose/TBE gel electrophoresis. The Xho I-Cla I E3/19K fragment is then ligated into the KT-3B retroviral backbone. This construct is designated KT-3B/E3/19K. It is amplified by transforming *E. coli ,* DH5 alpha bacterial strain (Bethesda Research Labs, Gaithersburg, MD) with the KT-3B/E3/19K construct. Specifically, the bacteria is transformed with 1-100 ng of ligation reaction mixture DNA. The transformed bacterial cells are plated on LB plates containing ampicillin. The plates are incubated overnight at 37°C, bacterial colonies are selected and DNA prepared from them. The DNA is digested with Xho I and Cla I. The expected endonuclease restriction cleavage fragment sizes for plasmids containing the E3/19K gene are 780 and 1,300 bp.

### D. Transduction of Packaging Cell Line DA with the Recombinant Retroviral Vector KT-3B/E3/19K

### i. Plasmid DNA Transfection

293 2-3 cells (a cell line derived from 293 cells ATCC No. CRL 1573, (WO 92/05266) 5.0 x 10⁵ cells are seeded at approximately 50% confluence on a 6 cm tissue culture dish. The following day, the media is replaced with 4 ml fresh media 4 hours prior to transfection. A standard calcium phosphate-DNA coprecipitation is performed by mixing 10.0 µg of KT-3B/E3/19K plasmid and 10.0 µg MLP G plasmid with a 2M CaCl₂ solution, adding a 1x HEPES buffered saline solution, pH 6.9, and incubating for 15 minutes at room temperature. The calcium phosphate-DNA coprecipitate is transferred to the 293 2-3 cells, which are then incubated overnight at 37°C, 5% CO₂. The following morning, the cells are rinsed three times in 1x PBS, pH 7.0. Fresh media is added to the cells, followed by overnight incubation at 37°C, 10% CO₂. The following day, the media is collected off the cells and passed through a 0.45 µ filter. This supernatant is used to transduce packaging and tumor cell lines. Transient vector supernatant for other vectors are generated in a similar fashion.

### ii. Packaging Cell Line Transduction

Packaging cell line transduction is performed as described in Example 2Bv.

### iii. Detection Of Replication Competent Retroviruses

Detection of replication competent retroviruses is performed as described in Example 2C

### E. Transduction of Cell Lines with the Recombinant Retroviral Vector KT-3B/E3/19K

The following adherent human and murine cell lines are seeded at 5 x 10⁵ cells/10 cm dish with 4 µg/ml polybrene: HT 1080, Hela, and BC10ME. The following day, 1.0 ml of filtered supernatant from the DA E3/19K pool is added to each of the cell culture plates. The following day, 800 µg/ml G418 is added to the media of all cell cultures. The cultures are maintained until selection is complete and sufficient cell numbers are generated to test for gene expression. The transduced cell lines are designated HT 1080-E3/19K, Hela-E3/19K and BC10ME-E3/19K, respectively.

EBV transformed cell lines (BLCL), and other suspension cell lines, are transduced by co-cultivation with irradiated producer cell line, such as DA-E3/19K. Specifically, irradiated (10,000 rads) producer line cells are plated at 5.0 x 10⁵ cells/6 cm dish in growth media containing 4 µg/ml polybrene. After the cells have been allowed to attach for 2-24 hours, 1.0 x 10⁶ suspension cells are added. After 2-3 days, the suspension cells are removed, pelleted by centrifugation, resuspended in growth media containing lmg/ml G418, and seeded in 10 wells of a round bottom 96 well plate. The cultures were expanded to 24 well plates, then to T-25 flasks.

### F. Expression of E3/19K in the Recombinant Retroviral Vector Construct KT3B-E3/19K

### i. Western Blot Analysis

RIPA lysates are made from selected confluent cell cultures for analysis of E3/19K expression. Specifically, the media is first aspirated off the cells. Depending upon the size of the culture plate containing the cells, a volume of 100 to 500 µl ice cold RIPA lysis buffer (10 mM Tris, pH 7.4; 1% Nonidet P40; 0.1% SDS; 150 mM NaCl) is added to the cells. Cells are removed from plates and the mixture is transferred to a microfuge tube. The tube is centrifuged for 5 minutes to precipitate cellular debris and the supernatant is transferred to another tube. The supernatants are electrophoresed on a 10% SDS-polyacrylamide gel and the protein bands are transferred to an Immobilon membrane in CAPS buffer (10 mM CAPS, pH 11.0; 10% methanol) at 10 to 60 volts for 2 to 18 hours. The membrane is transferred from the CAPS buffer to 5% Blotto (5% nonfat dry milk; 50 mM Tris, pH 7.4; 150 mM NaCI; 0.02% Na azide, and 0.05% Tween 20) and probed with a mouse monoclonal antibody to E3/19K (Severinsson *et al*., *J*. *Cell. Biol*. *101*:540-547, 1985). Antibody binding to the membrane is detected by the use of ¹²⁵I-Protein A.

### G. Expression of KT3B-GC And KT3B-E3/19K Recombinant Retroviral Vector Constructs in Animal Models

### i. Murine CTL Assay

KT3B-GC and KT3B-E3/19K vectors are injected individually or mixed together and compare the CTL responses. The E3/19K CTL response to GC is negligible while the mixed GC and E3/19K response is less than the administration of the GC vector alone.

Balb/c mice are injected with 1.0 x 10⁵ to 5.0 x 10⁷ cfu of single or combinations of vectors. After 7 days, the spleens are harvested, dispersed into single cell suspension and 3.0 x 10⁶ splenocytes/ml are cultured *in vitro* with 6.0 x 10⁴ cells/ml irradiated BC-GC or BC-GC-E3/19K cells for 7 days at 37°C in T-25 flasks. BC-GC cells are BC10ME cells transduced with the KT3B-GC vector. BC-GC-E3/19K are BC10ME mouse fibroblasts transduced with the KT3B-GC vector and the KT3B-E3/19K vector. Culture medium consists of RPMI 1640; 5% FBS; 1 mM pyruvate; 50 µg/ml gentamicin and 10⁻⁵ M β-2-mercaptoethanol. Effector cells are harvested 7 days later and tested using various effector:target cell ratios in 96 well microtiter plates in a standard 4-6 hour assay. The assay employs Na₂⁵¹CrO₄-labeled, 100 µCi, 1 hour at 37°C, with target cells BC, BC*env*, (Warner *et al*., *AIDS Res*. *and Human Retroviruses 7*:645, 1991), or BC*env* E3/19K at 1.0 x 10⁴ cells/well with the final total volume per well of 200 µl. Following incubation, 100 µl of culture medium is removed and analyzed in a WALLAC gamma spectrometer (Gaithersburg, MD). Spontaneous release (SR) is determined as counts per minute (CPM) CPM from targets plus medium and maximum release (MR) is determined as from targets plus 1M HCl. Percent target cell lysis is calculated as: [effector cell + target CPM) - (SR)]/[(MR) - (SR)] x 100. Spontaneous release values of targets are typically 10%-30% of the MR. Cells expressing GC or GC plus E3/19K are used as stimulator and/or target cells in this assay to demonstrate the reduction of GC-specific CTL induction and detection with E3/19K expressing cells as compared to the GC expressing line which is the positive control.

### H. Tumor Rejection of L33GC Cells by Balb/C Mice is Abrogated when Class I Molecule Surface Expression is Decreased by the E3/19K-Vector Transduction.

The L33GC cell is being employed as a model for gene therapy treated transformed cells. Gene therapy treated cells produce a foreign protein making them possible targets for clearance by CTL. It has been demonstrated that Balb/c mice injected with live L33 tumor cells will develop a solid tumor identifiable by caliper measurement within three weeks post-exposure. However, Balb/c mice injected with live L33GC transformed tumor cells (L33 cells transformed with the KT3B-GC vector and selected for the GC protein) recognize GC protein in the context of MHC class I and reject the tumor cells with no apparent tumor up to 15 weeks later. Transformation of L33GC cells with the E3/19K vector decreases cell surface expression of MHC class I molecules allowing these cells to evade immune surveillance and thereby establish a tumor. Development of an L33GC-E3/19K tumor indicates that cell surface expression of MHC class I molecules has been decreased by co-transducing cells with the E3/19K gene. This impedes optimal immune system clearance mechanisms.

Three tumor cell lines L33, L33GC, and L33GC-E3/19K are grown in DMEM containing 10% FBS. The tumor cells are gently rinsed with cold (4°C) PBS and treated with Versene to remove them from the plate. After aspirating cells from plates, single cell suspensions are added to sterile plastic tubes. Cell suspensions are washed two times in sterile PBS (4°C), counted and resuspended in PBS to 1.0 x 10⁷ cells/ml. Four to six week old Balb/c mice are injected subcutaneous with 1.0 x 10⁶ live tumor cells (0.1 ml) and assessed for tumor formation and tumor clearance. Different mice are injected with different tumor cell lines. Mice injected with L33 cells are positive control animals for tumor formation while those injected with L33GC are negative controls and should therefore reject the tumor cells because of the *env* specific CTL response. The group of mice injected with E3/19K-transformed, L33GC cells are monitored to show the effect that E3/19K expression in *L33env* cells has on the murine immune response to these tumor cells.

### I. Ex-Vivo Administration of a Glucocerebrosidase Retroviral Vector

Pluripotent hematopoetic stem cells, CD34⁺, are collected from the bone marrow of a patient by a syringe evacuation performed by known techniques. Alternatively, CD34⁺ cells may also be obtained from the cord blood of an infant. Generally, 20 bone-marrow aspirations are obtained by puncturing femoral shafts or from the posterior iliac crest under local or general anesthesia. Bone marrow aspirations are then pooled and suspended in HEPES-buffered Hanks' balanced salt solution containing heparin sulfate at 100 U/ml and deoxyribonuclease I at 100 µg/ml and then subjected to Ficoll gradient separation. The buffy coated marrow cells are then collected and washed according to CEPRATE LC (CD34) separation system (Cellpro, Bothell, WA). The washed buffy coated cells are then stained sequentially with anti-CD34 monoclonal antibody, washed, and stained with biotinylated secondary antibody supplied with the CEPRATE system. This cell mixture is loaded onto the CEPRATE avidin column. The biotin-labeled cells are adsorbed onto the column while unlabeled cells pass through. The column is rinsed according to the CEPRATE system directions and CD34⁺ cells eluted by agitation of the column by manually squeezing the gel bed. Once the CD34⁺ cells are purified, the stem cells are counted and plated at a concentration of 1.0 x 10⁵ cells/ml in Iscove's modified Dulbecco's medium, IMDM (Irvine Scientific, Santa Ana, CA) containing 20% pooled non-heat inactivated human AB serum (hAB).

After purification, several methods of transforming CD34⁺ cells may be performed. One approach involves transduction of the purified stem cell population with vector containing supernatant cultures derived from vector producing cells. A second approach involves co-cultivation of an irradiated monolayer of vector producing cells with the purified population of non-adherent CD34⁺ cells. A third and preferred approach involves co-cultivation with purified CD34⁺ cells which are pre-stimulated with various cytokines and cultured 48 hours prior to the co-cultivation with the irradiated vector-producing cells. Pre-stimulation prior to transduction increases effective gene transfer (Nolta *et al*., *Exp. Hematol. 20:*1065, 1992). The increased level of transduction is attributed to increased proliferation of the stem cells necessary for efficient retroviral transduction. Stimulation of these cultures to proliferate also provides increased cell populations for re-infusion into the patient.

Pre-stimulation of the CD34⁺ cells is performed by incubating the cells with a combination of cytokines and growth factors which include IL-1, IL-3, IL-6 and mast cell growth factor (MGF). Pre-stimulation is performed by culturing 1.0 x 10⁵ to 2.0 x 10⁵ CD34⁺ cells/ml of medium in T25 tissue culture flasks containing bone marrow stimulation medium for 48 hours. The bone marrow stimulation medium consists of IMDM containing 30% non-heat inactivated hAB serum, 2mM L-glutamine, 0.1 mM 2-mercaptoethanol, 1 µM hydrocortisone, and 1% deionized bovine serum albumin. All reagents used in the bone marrow cultures are screened for their ability to support maximal numbers of granulocyte erythrocyte macrophage megakaryocyte colony-forming units from normal marrow. Purified recombinant human cytokines and growth factors (Immunex Corp., Seattle, WA) for pre-stimulation should be used at the following concentrations: *E*. *coli*-derived IL-1α (100 U/ml), yeast-derived IL-3 (5 ng/ml), IL-6 (50 U/ml), and MGF (50 ng/ml) (Anderson *et al*., *Cell Growth Differ*. *2*:373, 1991).

After pre-stimulation of the CD34⁺ cells, they are transduced by co-cultivating on the combined irradiated DA-based producer cell lines, expressing; (1) the KT3B-GC therapeutic vector; (2) the KT3B-E3/19K vector, in the continued presence of the stimulation medium. The DA vector producing cell lines are first trypsinized, irradiated using 10,000 rads and replated at 1.0 x 10⁵ to 2.0 x 10⁵/ml of bone marrow stimulation medium. The following day, 1.0 x 10⁵ to 2.0 x 10⁵ prestimulated CD34⁺ cells/ml are added onto the DA vector producing cell line monolayer followed by polybrene to a final concentration of 4 µg/ml. Co-cultivation of the cells is performed for 48 hours. After co-cultivation, the CD34⁺ cells are collected from the adherent DA vector producing cell monolayer by vigorous flushing with medium and plated for 2 hours to allow adherence of any dislodged vector producing cells. The cells are then collected and expanded for an additional 72 hours. The cells are collected and frozen in liquid nitrogen using a cryo-protectant in aliquots of 1.0 x 10⁷ cells per vial. Once the transformed CD34⁺ cells have been tested for the presence of adventitious agents, frozen transformed CD34⁺ cells may be thawed, plated to a concentration of 1.0 x 10⁵ cells/ml and cultured for an additional 48 hours in bone marrow stimulation medium. Transformed cells are then collected, washed twice and resuspended in normal saline. The number of transformed cells used to infuse back into the patient per infusion is projected to be at a minimum of 1.0 x 10⁷ to 1.0 x 10⁸ cells per patient per injection. The site of infusion may be directly into the patients bone marrow or into the peripheral blood stream. Patients receiving autologous transduced bone marrow cells may be either partially or whole body irradiated, to deplete existing bone marrow populations. Assessment of treatment may be performed at various time points, post infusion, by monitoring glucocerebrosidase activity in differentiated cell types and for length of expression. At the point when expression decreases or is non-existent, transformed autologous cells may be re-injected into the patient.

### J. Allogeneic Marrow Grafts

### i. Remove Bone Marrow from C3H (H-2^{k}) and Balb/C (H-2^{d})

Mouse femurs are dissected and exposed. The bone marrow plugs are removed using a number 23 gauge needle and syringe. The marrow is collected and resuspended in Hank's balanced salt solution (Mauch *et al*., *PNAS 77*:2927, 1980)

### ii. Transduction of Marrow Cells with KT3B-E3/19K and KT3B-GC Retroviral Vector

Marrow cells are prepared by centrifugation and resuspension in 1.0 ml DMEM and 10% FBS containing KT3B-E3/19K and KT3B-GC vectors. The marrow cells and KT3B-E3/19K and KT3B-GC retroviral vector is incubated for 4 hours at 33°C in 9 mls of Fischer's medium supplemented with 25% donor horse serum and 0.1 mM hydrocortisone sodium succinate. After 24 hours, the marrow cells are washed and resuspended in HBSS at 2.0 x 10⁶ cells/ml for injection.

### iii. Injection of Marrow Cells into Mice

The C57BL/6 (B6, H-2^{b}) mice are irradiated with 700 rads of gamma irradiation just prior to injection. Two groups of B6 mice are injected intravenously with 0.5 ml of C₃H marrow cells. After 5 days, the mice are again irradiated with 700 rads and injected I.V. with 0.5 ml of either vector-transduced C₃H marrow cells or untreated C₃H marrow cells. Lethally irradiated naive B6 mice are injected I.V. with 0.5ml (1.0 x 10⁶) of C₃H bone marrow cells for the positive control and 0.5ml (1.0 x 10⁶) of Balb/c bone marrow cells for the negative control. Results from such experiments are shown below.

| RECIPIENT | 1° | 2° | RESULT (MARROW GROWTH) |
|---|---|---|---|
| B6 (H-2^{b}) | | Balb/c (H-2^{d}) | - |
| B6 (H-2^{b}) | | C₃H (H-2^{k}) | + |
| B6(H-2^{b}) | C₃H | C₃H | - |
| B6(H-2^{b}) | C₃H | C₃H-E3/19K | + |

### iv. Evaluation of Graft Rejections

The bone marrow graft rejections are evaluated 5 days following injection by either of the two methods:
a. After sacrificing the mice, the spleens are removed and placed into 10% formalin. Spleen colonies are counted and recorded.
b. Mice are injected with FUdR (Sigma, St. Louis, MO) and 30 minutes later with ¹²⁵I-IUdR (Amersham, Arlington Height, IL). After 18 hours the incorporation of ¹²⁵I-IUdR is determined in spleen colonies. The value of incorporated radioactivity determined in the syngeneic growth control is arbitrarily set at 100 U, and all values in the experimental groups are normalized relative to this control. Animals with 10 U show no visible spleen colonies, whereas animals with 50 to 100 U have greater than 200 spleen colonies. Animals that show less than 10 U are considered to express strong rejection. Those with 10 to 30 U are considered to express weak rejection, while those with greater than 30 U show no significant rejection.

### K. Injection of Marrow Cells Directly into Bone

Alternatively, the vector may be delivered directly to the marrow of mice using a device such as a Hamilton syringe. The doses would be equivalent to those used in Example 2Gii. In humans, bone marrow may be directly injected using commercially available devices such as the First-Med™ osseous autoinjector (Life Quest Medical, San Antonio, TX). Doses would be 0.1 to 5.0 ml of material at 10⁵ to 10⁹ cfu/ml in a suitable excipient (e.g.,. lactose (40 mg/ml), human serum albumin (1 mg/ml) and Tris (1 mM) pH 7.2 - 7.5).

### L. Evaluation of GC Gene Expression

The response of the mice against human GC can be evaluated by CTL assays as described above. Human GC expression itself can be monitored by the immunohistochemical procedure as described by Krall *et al*., (*Blood 83*: 2737, 1994) using cryosections and a human monoclonal antibody to GC.

### EXAMPLE 9

### Administration of Adenoviral Vector Expressing HBV core Antigen and a Retroviral Vector Expressing HIV

### A. Generation of Recombinant Adenoviral Vectors

Approximately 1.0 µg of pAdM1-HBe linearized with Cla I is mixed with 1.0 µg of Cla I cleaved Ad5delta e1 delta E3 viral DNA (Gluzman *et al*., in *Eucaryotic Viral Vectors,* pp. 187-192, Cold Spring Harbor, 1982). This DNA mixture is transfected onto 5-6 x 10⁵ 293 cells in 60 mm diameter dishes using 7 µl of lipofectamine (BRL, Gaithersburg, MD) in 0.8 ml of Opti-MEM™ I Reduced Serum Medium (BRL, Gaithersburg, MD). One milliliter of DMEM media with 20% FBS is added after 5 hours and DMEM media with 10% FBS is replenished the following day. After the appearance of cytopathic effects (CPE), at approximately 8-10 days, the culture is harvested and the viral lysates are subjected to two rounds of plaque purification. Individual viral plaques are chosen and amplified by infecting 293 cells in 6 well plates at a cell density of 1 x 10⁵ cells per well. Recombinants are identified by Southern analysis of viral DNA extracted from infected cells by the Hirt procedure (Hirt, B. *J*. *Mol Biol*., *26*:365-69, 1967). After positive identification, the recombinant virus is subjected to two additional rounds of plaque purification. Titers are determined by plaque assay as described in Graham *et al*. (*J*. *Gen*. *Virol*. *36*:59-72, 1977). Viral stocks are prepared by infecting 2 x 10⁶ confluent 293 cells in 60 mm diameter dishes at a multiplicity of 20 pfu/cell. All viral preparations are purified by CsCI density centrifugation (Graham and Van der Elb, *Virol*. *52*:456-457, 1973), dialyzed, and stored in 10 mM Tris-HCl (pH 7.4), 1 mM MgCl₂ at 4°C for immediate use, or stored with the addition of 10% glycerol at -70°C.

### B. Infection with Recombinant Adenoviral Vector

Subconfluent monolayers of L⁻M(TK⁻) cells (approximately 10⁶ cells) growing in 35 mm dishes are infected with recombinant HBV core adenovirus vectors from Example 9A above at a multiplicity of 100 pfu/cell. One hour after adsorption at 37°C, the virus inocula is removed and DMEM supplemented with 2% FBS is added. Thirty to forty hours after infection, when pronounced CPE is observed, cell extracts are harvested and assayed for expression.

### C. Administration into Mice, Humans, and Non-Human Primates

### i. Direct Vector Administration and Mouse CTL Assays

The mouse system may also be used to evaluate the induction of humoral and cell-mediated immune responses with direct administration of vector encoding HBV core or HIV antigen. Briefly, six- to eight-week-old female Balb/C, C57B16, or C3H mice are injected I.M., I.D., or S.C. with 0.1 ml of unpurified liquid or reconstituted 10⁴ - 10¹¹ pfu lyophilized HBV core Adenoviral vector and/or 10⁵ - 10⁹ cfu HIV antigen expressing retroviral vector (Example 3). The vectors are injected individually or mixed and administered together. Two injections are given one week apart. Seven days after the second injection, the animals are sacrificed. Chromium release CTL assays are then performed as described in Example 2Hi.

### ii. Measurement of Immune Response

Humoral and cellular responses in mice, humans, and non-human primates are measured as described in Example 2I.

### iii. Human and Non-Human Primate Administration Protocol

Vector construct administration protocol for human and non-human primates is performed essentially as described in Example 2G.

### iv. Human and Chimpanzee Administration Protocol

The data generated in the mouse system above is used to determine the protocol of administration of vector in humans or chimpanzees chronically infected with HBV and/or HIV-1. Based on the induction of HBV-specific CTL in mice, the subjects in human or chimpanzee trials receive three doses of vector encoding core antigen at 28 day intervals given in two successively escalating dosage groups. Control subjects receive a placebo comprised of HBV core adenoviral vector formulation media. The combined dosage is either 10⁵, 10⁶, 10⁷, 10⁸ ,10⁹, 10¹⁰, 10¹¹ cfu of the HBV core adenoviral vector and 10⁶, 10⁷, 10⁸ or 10⁹ cfu of the HIV vector given each in four 0.5 ml injections I.M. on each injection day. Injections are administered 2 to 4 weeks apart. Blood samples will be drawn on days 4, 12, 24, 36, 52, 70 and 84 and months 6, 12, 18, 24, 30, and 36 in order to measure serum ALT levels, the presence of HBV core antigen, the presence of antibodies directed against the HBV core antigen and to assess safety and tolerability of the treatment. Efficacy of the induction of CTL against HBV core can be determined as in Example 12A iii.

### EXAMPLE 10

### Preservation of A Recombinant Retrovirus

### A. Lactose Formulation of a Recombinant Retrovirus

Crude recombinant retrovirus is obtained from a Celligan bioreactor (New Brunswick, New Brunswick, NJ) containing DA cells transformed with the recombinant retrovirus bound to the beads of the bioreactor matrix. The cells release the recombinant retrovirus into the growth media that is passed over the cells in a continuous flow process. The media exiting the bioreactor is collected and passed initially through a 0.8 micron filter then through a 0.65 micron filter to clarify the crude recombinant retrovirus. The filtrate is concentrated utilizing a cross flow concentrating system (Filtron, Boston, MA). Approximately 50 Units of DNase (Intergen, New York, NY) per ml of concentrate is added to digest exogenous DNA. The digest is diafiltrated using the same cross flow system to 150 mM NaCl, 25 mM tromethamine, pH 7.2. The diafiltrate is loaded onto a Sephadex S-500 gel column (Pharmacia, Piscataway, NJ), equilibrated in 50 mM NaCI, 25 mM tromethamine, pH 7.4. The purified recombinant retrovirus is eluted from the Sephadex S-500 gel column in 50 mM NaCI, 25 mM tromethamine, pH 7.4.

The formulation buffer containing lactose was prepared at a 2X concentrated stock solution. The formulation buffer contains 25 mM tromethamine, 70 mM NaCI, 2 mg/ml arginine, 10 mg/ml HSA, and 100 mg/ml lactose in a final volume of 100 mls at a pH 7.4.

The purified recombinant retrovirus is formulated by adding one part 2X lactose formulation buffer to one part S-500 purified recombinant retrovirus. The formulated recombinant retrovirus can be stored at -70°C to -80°C or dried.

The formulated retrovirus is lyophilized in an Edwards Refrigerated Chamber (3 Shelf RC3S unit) attached to a Supermodulyo 12K freeze dryer (Edwards High Vacuum, Tonawanda, NY). When the freeze drying cycle is completed, the vials are stoppered under a vacuum following a slight nitrogen gas bleeding. Upon removal, vials are crimped with aluminum seals.

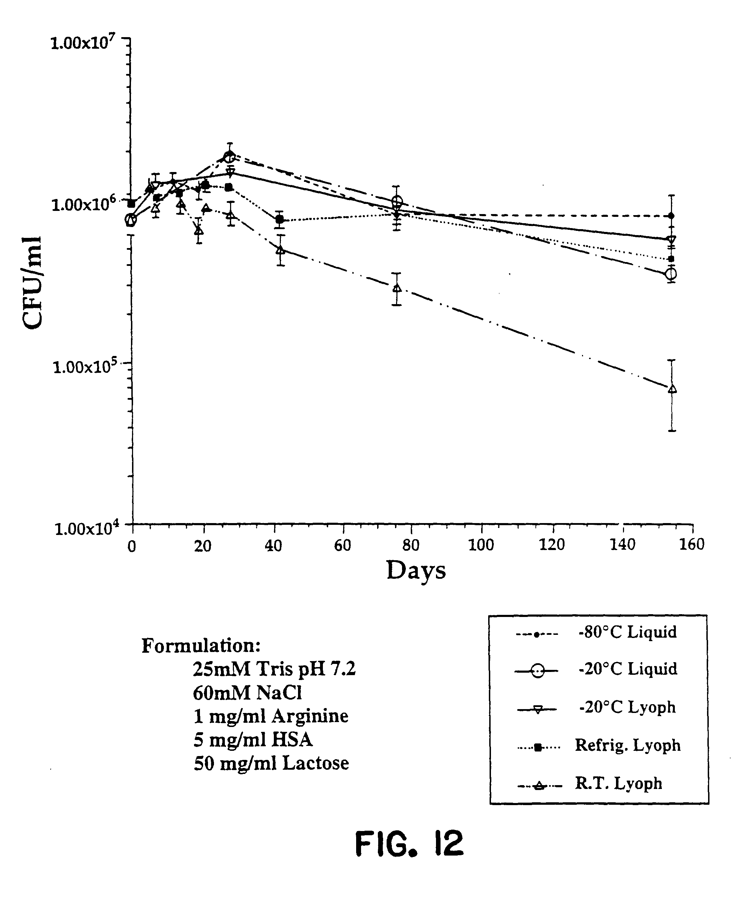

In the given lactose study, formulated liquid product was stored at both -80° C and at -20°C cycling freezer. In Figure 12 viral infectivity of these samples were compared to the viral infectivity of lyophilized samples. The lyophilized samples were stored at -20°C, refrigerator temperature and room temperature. Activity of the samples upon reconstitution are determined by titer assay.

The lyophilized recombinant retrovirus is reconstituted with 1.0 ml water. The infectivity of the reconstituted recombinant retrovirus is determined by a titer activity assay. The assay is conducted on HT 1080 fibroblasts or 3T3 mouse fibroblast cell line (ATCC No. CCL 163). Specifically, 1 x 10⁵ cells are plated onto 6 cm plates and incubated overnight at 37°C, 10% CO₂. Ten microliters of a dilution series of reconstituted recombinant retroviruses are added to the cells in the presence of 4 µg/mL polybrene (Sigma, St. Louis, MO) and incubated overnight at 37°C, 10% CO₂. Following incubation, cells are selected for neomycin resistance in G418 containing media and incubated for 5 days at 37°C, 10% CO₂. Following initial selection, the cells are re-fed with fresh media containing G418 and incubated for 5-6 days. After final selection, the cells are stained with Commassie blue for colony detection. The titer of the sample is determined from the number of colonies, the dilution, and the volume used.

Figure 12 demonstrates that storage in lyophilized form at -20°C to refrigerator temperatures retains similar viral activity as a recombinant retrovirus stored in liquid at -80 to -20°C permitting less stringent temperature control during storage.

### B. Mannitol Formulation of a Recombinant Retrovirus

The recombinant retrovirus utilized in this example was purified as described in Example 10A.

The formulation buffer containing mannitol was prepared as a 2X concentrated stock solution. The formulation buffer contains 25 mM tromethamine, 35 mM NaCl, 2 mg/ml arginine, 10 mg/ml HSA and 80 mg/ml mannitol at a final volume of 100 mls at a pH 7.4.

The purified recombinant retrovirus is formulated by adding one part mannitol formulation buffer to one part S-500 purified recombinant retrovirus. The formulated recombinant retrovirus can be stored at this stage at -70°C to -80°C or dried.

The formulated retrovirus is dried in an Edwards Refrigerated Chamber (3 Shelf RC3S unit) attached to a Supermodulyo 12K freeze dryer. When the freeze drying cycle is completed, the vials are stoppered under a vacuum following nitrogen gas bleeding to 700 mbar. Upon removal, vials are crimped with aluminum seals.

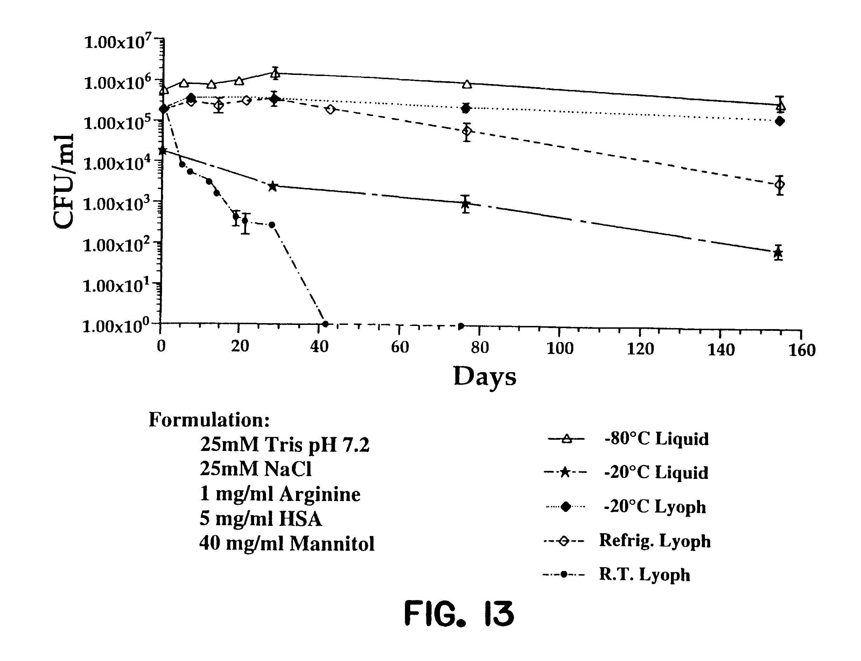

In the given mannitol study, formulated liquid product was stored at both -80 °C and at -20°C in cycling freezers. The viral infectivity of these samples were compared to the viral infectivity of lyophilized samples, Figure 13 The lyophilized samples were stored at -20°C, refrigerator temperature and room temperature. Activity of the samples upon reconstitution are determined using the titer assay described in Example 10A.

Figure 13 demonstrates that storage in lyophilized form at -20°C to refrigerator temperature retains significant viral activity as compared to recombinant retrovirus stored in liquid at -80°C or -20°C, permitting less stringent temperature control during storage.

### C. Trehalose Formulation of a Recombinant Retrovirus

The recombinant retrovirus utilized in this example was purified as described in Example 10A.

The formulation buffer containing trehalose was prepared as a 2X concentrated stock solution. The formulation buffer contains 25 mM tromethamine, 70 mM NaCI, 2.0 mg/ml arginine, 10.0 mg/ml HSA and 100 mg/ml trehalose at a final volume of 100 mis at a pH 7.2.

The purified recombinant retrovirus is formulated by adding one part trehalose formulation buffer to one part S-500 purified recombinant retrovirus. The formulated recombinant retrovirus can be stored at this stage at -70°C to -80°C or dried.

The formulated retrovirus is dried in an Edwards Refrigerated Chamber (3 Shelf RC3S unit) attached to a Supermodulyo 12K freeze dryer. When the freeze drying cycle is completed, the vials are stoppered under a vacuum following nitrogen gas bleeding to 700 mbar. Upon removal, vials are crimped with aluminum seals.

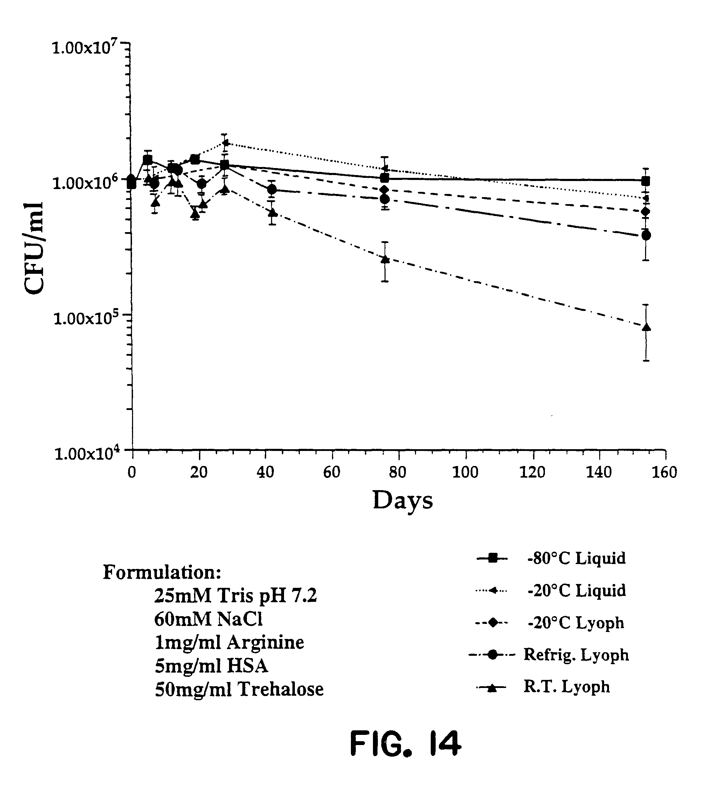

In the given trehalose study, formulated liquid product was stored at both -80 °C and at -20°C in cycling freezers. The viral infectivity of these samples was compared to the viral infectivity of lyophilized samples, Figure 14. The lyophilized samples were stored at -20°C, refrigerator temperature and room temperature. Activity of the samples upon reconstitution are determined using the titer assay as described in Example 10A.

Figure 14 demonstrates that storage in lyophilized form at -20°C to refrigerator temperature retains similar viral activity as compared to recombinant retrovirus stored in liquid at -80°C to -20°C permitting less stringent temperature control during storage.

Viral infectivity of liquid formulated recombinant retrovirus samples stored at -80°C was compared to viral infectivity of lyophilized formulated recombinant retrovirus stored at -20°C. Initially, a bulk of recombinant retrovirus was received and formulated in four different ways as shown below. The formulated recombinant retrovirus was then frozen in bulk for 1.5 months subsequent to being quick thawed and freeze dried. Positive controls were stored at -80°C for comparison with lyophilized samples which were stored at -20°C after freeze-drying. The formulations are listed below:

| Formulation | Sugar Concentration (mg/ml) | Buffer Concentration (mM tromehamine) | Salt Concentration (mM NaCl) | Arginine Concentration (mg/ml) | Human Serum Albumin Concentration (mg/ml) |
|---|---|---|---|---|---|
| Mannitol | 40 | 25 | 25 | 1 | 5 |
| Lactose | 40 | 25 | 75 | 1 | 5 |
| Sucrose | 50 | 25 | 60 | 1 | 5 |
| Trehalose | 50 | 25 | 60 | 1 | 5 |

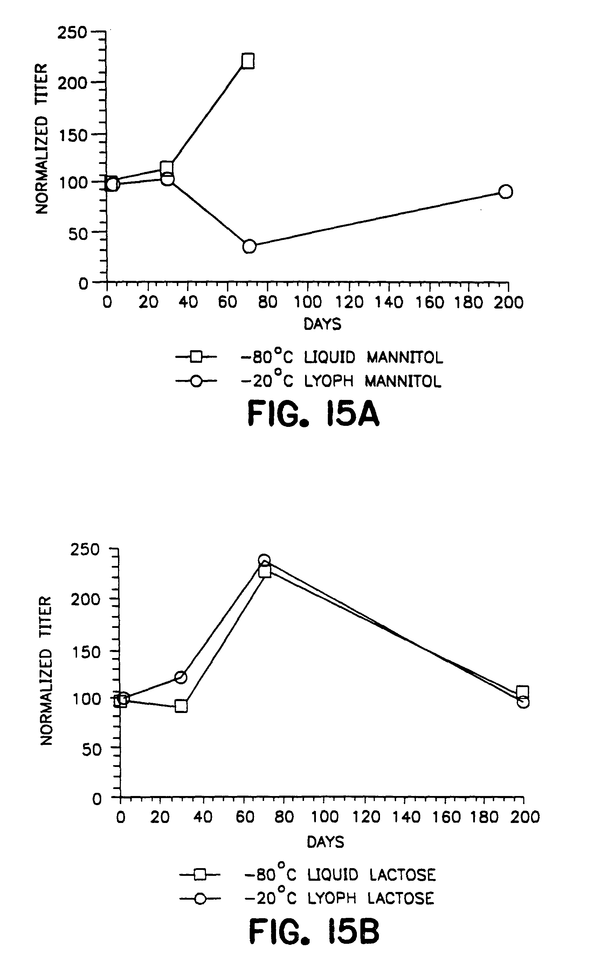

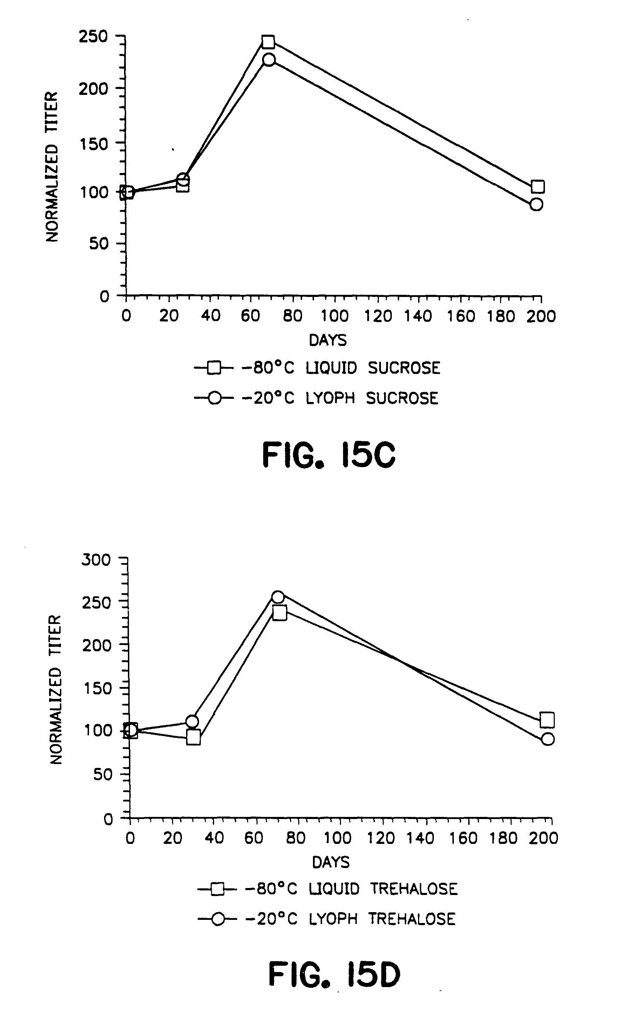

In the graphs of Figure 15, the y-axis on each of the 4 graphs (A, B, C, D) represent the normalized titer. At an initial time point after lyophilization, t = 0, a titer value was established for both the -80°C liquid sample and the -20°C lyophilized sample. At each time point of the stability study, the titer obtained was divided by the zero time point titer value and the % of original entered onto the graph.

The data demonstrates that post-lyphilization activity is maintained in the lyophilized sample (stored at -20°C) relative to the liquid sample (stored at -80°C). The formulated lyophilized recombinant retrovirus was stored in a -20°C freezer (a frost-free cycling freezer). Comparison to the formulated liquid recombinant retrovirus stored at -80° C indicates the lyophilized form permits less stringent control of storage conditions.

## Claims

1. A composition comprising two or more different viral vector gene delivery vehicles, each of said gene delivery vehicles containing a nucleic acid molecule not naturally contained within its corresponding gene delivery vehicle, in combination with a pharmaceutically acceptable carrier or diluent for use in introducing nucleic acid molecules to an animal

2. A product containing two or more different viral vector gene delivery vehicles, each of said gene delivery vehicles containing a nucleic acid molecule not naturally contained within its corresponding gene delivery vehicle, for use in introducing nucleic acid molecules to an animal at the same time and same site via a single administration device.

3. A composition as claimed in claim 1 or a product as claimed in claim 2 wherein each of said gene delivery vehicles directs the expression of at least one substance in host cells containing said gene delivery vehicle, the substance not naturally expressed by its corresponding gene delivery vehicle, said gene delivery vehicles a) collectively directing the expression of at least two different substances, or b) directing the expression of at least one substance wherein said gene delivery vehicles differ in one or more biological functions.

4. A composition as claimed in claim 1 or a product as claimed in claim 2 wherein each of said gene delivery vehicles contains at least one biologically active nucleic acid molecule wherein such biological activity is not naturally present in its respective gene delivery vehicle, said gene delivery vehicles a) collectively containing at least two different biologically active nucleic acid sequences, or b) containing at least one biologically active nucleic acid sequence wherein said gene delivery vehicles differ in one or more biological functions.

5. A composition as claimed in claim 1 or a product as claimed in claim 2 wherein at least one of said gene delivery vehicles directs the expression of at least one substance not naturally expressed by its corresponding gene delivery vehicle, and at least one of said gene delivery vehicles contains at least one biologically active nucleic acid sequence not naturally contained within its corresponding gene delivery vehicle.

6. A composition as claimed in any one of claims 1 and 3 to 5 wherein said pharmaceutically acceptable carrier or diluent enhances the administration of said gene delivery vehicles.

7. A composition or product as claimed in any one of claims 1 to 5 wherein said substance or said biological activity is not exhibited in said animal prior to said administration.

8. A composition or product as claimed in claim 4 or claim 5 wherein said biological activity is not exhibited in said host cells prior to administration.

9. A composition or product as claimed in claim 4 or claim 5 wherein said biological activity complements a biological activity present in said host cells prior to administration.

10. A composition or product as claimed in claim 4 or claim 5 wherein said biological activity activates a biological activity that had not been exhibited in said host cells prior to administration.

11. A composition or product as claimed in claim 4 or claim 5 wherein said biological activity replaces a biological activity exhibited in said host cells prior to administration.

12. A composition or product as claimed in claim 4 or claim 5 wherein said biological activity suppresses a biological activity exhibited in said host cells prior to administration.

13. A composition or product as claimed in claim 3 wherein a first gene delivery vehicle is capable of directing the expression of an antigen stimulator and a second gene delivery vehicle is capable of directing the expression of a cytokine or an immune activating protein.

14. A composition or product as claimed in claim 3 wherein a first gene delivery vehicle is capable of directing the expression of an enzyme capable of activating a conditionally lethal gene product and a second gene delivery vehicle is capable of directing the expression of a cytokine.

15. A composition or product as claimed in claim 3 wherein said gene delivery vehicles are capable of directing the expression of two or more antigens.

16. A composition or product as claimed in claim 3 wherein at least one of said gene delivery vehicles is capable of directing the expression of a systemically distributed gene product.

17. A composition or product as claimed in claim 16 wherein said systemically distributed gene product is a protein.

18. A composition or product as claimed in claim 3 wherein at least one of said gene delivery vehicles directs the expression of a locally distributed gene product.

19. A composition or product as claimed in claim 18 wherein said locally distributed gene product is a protein.

20. A composition or product as claimed in claim 3 where at leat one of said gene delivery vehicles is capable of directing the expression of an immune suppressing protein.

21. A composition as claimed in claim 1 or a product as claimed in claim 2 wherein said viral vector gene delivery vehicle is a recombinant retroviral vector.

22. A composition as claimed in claim 1 or a product as claimed in claim 2 wherein said viral vector gene delivery vehicle is a recombinant adenovirus vector.

## Patentansprüche

1. Zusammensetzung, umfassend zwei oder mehr unterschiedliche virale Vektor-Genabgabevehikel, wobei jedes der Genabgabevehikel ein Nucleinsäuremolekül enthält, das natürlicherweise nicht in seinem. entsprechenden Genabgabevehikel enthalten ist, in Kombination mit einem pharmazeutisch annehmbaren Träger oder Verdünnungsmittel, zur Verwendung bei der Einführung von Nucleinsäuremolekülen in ein Tier.

2. Produkt, das zwei oder mehr unterschiedliche virale Vektor-Genabgabevehikel, wobei jedes der Genabgabevehikel ein Nucleinsäuremolekül enthält, das natürlicherweise nicht in seinem entsprechenden Genabgabevehikel enthalten ist, enthält, zur Verwendung bei der Einführung von Nucleinsäuremolekülen zur gleichen Zeit und an dieselbe Stelle mit einer einzigen Verabreichung in ein Tier.

3. Zusammensetzung nach Anspruch 1 oder ein Produkt nach Anspruch 2, wobei jedes der Genabgabevehikel die Expression mindestens einer Substanz in Wirtszellen, die das Genabgabevehikel enthalten, steuert, die Substanz durch sein entsprechendes Genabgabevehikel natürlicherweise nicht exprimiert wird, die Genabgabevehikel (a) kollektiv die Expression von mindestens zwei unterschiedlichen Substanzen steuern oder (b) die Expression mindestens einer Substanz steuern und wobei sich die Genabgabevehikel in einer biologischen Funktion oder in mehreren biologischen Funktionen unterscheiden.

4. Zusammensetzung nach Anspruch 1 oder Produkt nach Anspruch 2, wobei jedes der Genabgabevehikel mindestens ein biologisch aktives Nucleinsäuremolekül enthält und wobei eine solche biologische Aktivität natürlicherweise nicht in seinem entsprechenden Genabgabevehikel vorhanden ist, die Genabgabevehikel (a) kollektiv mindestens zwei unterschiedliche biologisch aktive Nucleinsäuresequenzen enthalten oder (b) mindestens eine biologisch aktive Nucleinsäuresequenz enthalten und wobei die Genabgabevehikel sich in einer biologischen Funktion oder in mehreren biologischen Funktionen unterscheiden.

5. Zusammensetzung nach Anspruch 1 oder Produkt nach Anspruch 2, wobei mindestens eins der Genabgabevehikel die Expression mindestens einer Substanz, die natürlicherweise nicht durch sein entsprechendes Genabgabevehikel exprimiert wird, steuert und mindestens eins der Genabgabevehikel mindestens eine biologisch aktive Nucleinsäuresequenz enthält, die natürlicherweise nicht in seinem entsprechenden Genabgabevehikel enthalten ist.

6. Zusammensetzung nach einem der Ansprüche und 1 und 3 bis 5, wobei der pharmazeutisch annehmbare Träger oder das pharmazeutisch annehmbare Verdünnungsmittel die Verabreichung der Genabgabevehikel verstärkt.

7. Zusammensetzung oder Produkt nach einem der Ansprüche 1 bis 5, wobei die Substanz oder die biologische Aktivität vor einer Verabreichung in dem Tier nicht auftritt.

8. Zusammensetzung oder Produkt nach Anspruch 4 oder 5, wobei die biologische Aktivität vor einer Verabreichung in diesen Wirtszellen nicht auftritt.

9. Zusammensetzung oder Produkt nach Anspruch 4 oder Anspruch 5, wobei die biologische Aktivität eine biologische Aktivität ergänzt, die vor einer Verabreichung in den Wirtszellen vorhanden ist.

10. Zusammensetzung oder Produkt nach Anspruch 4 oder Anspruch 5, wobei die biologische Aktivität eine biologische Aktivität aktiviert, die vor einer Verabreichung in den Wirtszellen nicht auftrat.

11. Zusammensetzung oder Produkt nach Anspruch 4 oder Anspruch 5, wobei die biologische Aktivität eine biologische Aktivität ersetzt, die vor einer Verabreichung in den Wirtszellen auftrat.

12. Zusammensetzung oder Produkt nach Anspruch 4 oder Anspruch 5, wobei die biologische Aktivität eine biologische Aktivität unterdrückt, die in diesen Wirtszellen vor einer Verabreichung auftrat.

13. Zusammensetzung oder Produkt nach Anspruch 3, wobei ein erstes Genabgabevehikel fähig ist, die Expression eines Antigenstimulators zu steuern, und ein zweites Genabgabevehikel fähig ist, die Expression eines Cytokins oder eines immunaktivierenden Proteins zu steuern.

14. Zusammensetzung oder Produkt nach Anspruch 3, wobei ein erstes Genabgabevehikel fähig ist, die Expression eines Enzyms zu steuern, welches zur Aktivierung eines konditionell lethalen Genprodukts fähig ist, und ein zweites Genabgabevehikel fähig ist, die Expression eines Cytokins zu steuern.

15. Zusammensetzung oder Produkt nach Anspruch 3, wobei die Genabgabevehikel fähig sind, die Expression von zwei oder mehr Antigenen zu steuern.

16. Zusammensetzung oder Produkt nach Anspruch 3, wobei mindestens eins der Genabgabevehikel fähig ist, die Expression eines systemisch verteilten Genprodukts zu steuern.

17. Zusammensetzung oder Produkt nach Anspruch 16, wobei das systemisch verteilte Genprodukt ein Protein ist.

18. Zusammensetzung oder Produkt nach Anspruch 3, wobei mindestens eins der Genabgabevehikel die Expression eines lokal verteilten Genprodukts steuert.

19. Zusammensetzung oder Produkt nach Anspruch 18, wobei das lokal verteilte Genprodukt ein Protein ist.

20. Zusammensetzung oder Produkt nach Anspruch 3, wobei mindestens eins der Genabgabevehikel fähig ist, die Expression eines immunsuppremierenden Proteins zu steuern.

21. Zusammensetzung nach Anspruch 1 oder ein Produkt nach Anspruch 2, wobei das virale Vektor-Genabgabevehikel ein rekombinanter retroviraler Vektor ist.

22. Zusammensetzung nach Anspruch 1 oder Produkt nach Anspruch 2, wobei das virale Vektor-Genabgabevehikel ein rekombinanter Adenovirus-Vektor ist.

## Revendications

1. Composition comprenant deux véhicules différents d'acheminement de gènes ou davantage, de type vecteurs viraux, chacun desdits véhicules d'acheminement de gènes contenant une molécule d'acide nucléique qui n'est pas naturellement contenue dans son véhicule d'acheminement de gènes correspondant, en combinaison avec un support ou un diluant pharmaceutiquement acceptable, destinée à être utilisée pour introduire des molécules d'acide nucléique dans un animal.

2. Produit contenant deux véhicules différents d'acheminement de gènes ou davantage, de type vecteurs viraux, chacun desdits véhicules d'acheminement de gènes contenant une molécule d'acide nucléique qui n'est pas naturellement contenue dans son véhicule d'acheminement de gènes correspondant, destiné à être utilisé pour introduire des molécules d'acide nucléique dans un animal en même temps et au même site par un seul dispositif d'administration.

3. Composition selon la revendication 1 ou produit selon la revendication 2, dans lequel chacun desdits véhicules d'acheminement de gènes dirige l'expression d'au moins une substance dans des cellules hôtes contenant ledit véhicule d'acheminement de gènes, la substance n'étant pas naturellement exprimée par son véhicule d'acheminement de gènes correspondant, lesdits véhicules d'acheminement de gènes (a) dirigeant collectivement l'expression d'au moins deux substances différentes, ou (b) dirigeant l'expression d'au moins une substance, lesdits véhicules d'acheminement de gènes différant par une ou plusieurs fonctions biologiques.

4. Composition selon la revendication 1 ou produit selon la revendication 2, dans lequel chacun desdits véhicules d'acheminement de gènes contient au moins une molécule d'acide nucléique biologiquement active, cette activité biologique n'étant pas naturellement présente dans son véhicule respectif d'acheminement de gènes, lesdits véhicules d'acheminement de gènes (a) contenant collectivement au moins deux séquences d'acide nucléique biologiquement actives différentes, ou b) contenant au moins une séquence d'acide nucléique biologiquement active, lesdits véhicules d'acheminement de gènes différant par une ou plusieurs fonctions biologiques.

5. Composition selon la revendication 1 ou produit selon la revendication 2, dans lequel au moins l'un desdits véhicules d'acheminement de gènes dirige l'expression d'au moins une substance qui n'est pas naturellement exprimée par son véhicule d'acheminement de gènes correspondant, et au moins l'un desdits véhicules d'acheminement de gènes contient au moins une séquence d'acide nucléique biologiquement active qui n'est pas naturellement contenue dans son véhicule d'acheminement de gènes correspondant.

6. Composition selon l'une quelconque des revendications 1 et 3 à 5, dans laquelle ledit support ou diluant pharmaceutiquement acceptable améliore l'administration desdits véhicules d'acheminement de gènes.

7. Composition ou produit selon l'une quelconque des revendications 1 à 5, dans lequel ladite substance ou ladite activité biologique n'est pas présentée dans ledit animal avant ladite administration.

8. Composition ou produit selon la revendication 4 ou la revendication 5, dans lequel ladite activité biologique n'est pas présentée dans lesdites cellules hôtes avant l'administration.

9. Composition ou produit selon la revendication 4 ou la revendication 5, dans lequel ladite activité biologique complémente une activité biologique présente dans lesdites cellules hôtes avant l'administration.

10. Composition ou produit selon la revendication 4 ou la revendication 5, dans lequel ladite activité biologique active une activité biologique qui n'était pas présentée dans lesdites cellules hôtes avant l'administration.

11. Composition ou produit selon la revendication 4 ou la revendication 5, dans lequel ladite activité biologique remplace une activité biologique présentée dans lesdites cellules hôtes avant l'administration.

12. Composition ou produit selon la revendication 4 ou la revendication 5, dans lequel ladite activité biologique a un effet de suppression vis à vis d'une activité biologique présentée dans lesdites cellules hôtes avant l'administration.

13. Composition ou produit selon la revendication 3, dans lequel un premier véhicule d'acheminement de gènes est capable de diriger l'expression d'un stimulateur d'antigènes et un second véhicule d'acheminement de gènes est capable de diriger l'expression d'une cytokine ou d'une protéine d'activation immunitaire.

14. Composition ou produit selon la revendication 3, dans lequel un premier véhicule d'acheminement de gènes est capable de diriger l'expression d'une enzyme capable d'activer un produit de gène létal dans certaines conditions et un second véhicule d'acheminement de gènes est capable de diriger l'expression d'une cytokine.

15. Composition ou produit selon la revendication 3, dans lequel lesdits véhicules d'acheminement de gènes sont capables de diriger l'expression de deux antigènes ou davantage.

16. Composition ou produit selon la revendication 3, dans lequel au moins l'un desdits véhicules d'acheminement de gènes est capable de diriger l'expression d'un produit de gène distribué systémiquement.

17. Composition ou produit selon la revendication 16, dans lequel le produit de gène distribué systémiquement est une protéine.

18. Composition ou produit selon la revendication 3, dans lequel au moins l'un desdits véhicules d'acheminement de gènes dirige l'expression d'un produit de gène distribué localement.

19. Composition ou produit selon la revendication 18, dans lequel ledit produit de gène distribué localement est une protéine.

20. Composition ou produit selon la revendication 3, dans lequel au moins l'un desdits véhicules d'acheminement de gènes est capable de diriger l'expression d'une protéine de suppression immunitaire.

21. Composition selon la revendication 1 ou produit selon la revendication 2, dans . lequel ledit véhicule d'acheminement de gènes de type vecteur viral est un vecteur rétroviral recombiné.

22. Composition selon la revendication 1 ou produit selon la revendication 2, dans lequel ledit véhicule d'acheminement de gènes de type vecteur viral est un vecteur adénovirus recombiné.
